# EUROPEAN PATENT SPECIFICATION

(11) **EP 2 461 390 B1**
(45) Date of publication and mention of the grant of the patent: **23.05.2018**
(21) Application number: 10804576.6
(22) Date of filing: 30.07.2010
(51) Int. Cl.: H01L 51/54, C07D 401/04, C07D 401/10, C07D 403/10, C07F 15/00, C09K 11/06, G09F 9/30, H01L 27/32, C07D 401/14, C07D 403/14, C07D 471/04, C07D 209/86, C07D 209/88, H05B 33/14, H01L 51/52

(54) **ORGANIC ELECTROLUMINESCENT ELEMENT**
ORGANISCHES ELEKTROLUMINESZENZELEMENT
ÉLÉMENT ÉLECTROLUMINESCENT ORGANIQUE

(30) Priority: 31.07.2009 JP 2009180222; 31.08.2009 JP 2009201157
(43) Date of publication of application: 06.06.2012
(73) Proprietor: UDC Ireland Limited, Ballycoolin, Dublin 15 (IE)
(72) Inventor: KITAMURA, Tetsu, Ashigarakami-gun, Kanagawa (JP); WATANABE, Toru, Ashigarakami-gun, Kanagawa (JP); HAYASHI, Masayuki, Ashigarakami-gun, Kanagawa (JP); ISE, Toshihiro, Ashigarakami-gun, Kanagawa (JP)
(74) Representative: Maiwald Patentanwalts GmbH
(86) International application number: PCT/JP2010/062961
(87) International publication number: WO 2011/013830

(56) References cited:
- EP-A2- 1 017 118
- WO-A1-2005/112519
- WO-A1-2008/117889
- WO-A1-2008/123178
- JP-A- 2000 196 140
- JP-A- 2004 227 814
- JP-A- 2009 016 693
- JP-A- 2009 016 693
- JP-A- 2009 170 815
- JP-A- 2009 170 815

## Description

### Technical Field

The present invention relates to an organic electroluminescence device that converts electric energy into light to emit the light.

### Background Art

Since organic electroluminescence devices (hereinafter, referred to as "devices" or "organic EL devices") are capable of obtaining a light emission with high luminance intensity by driving low voltage, the devices have been actively researched and developed. Organic electroluminescence devices have an organic layer interposed between a pair of electrodes, and utilize, for light emission, energy of the exciton generated as a result of recombination of electrons injected from a cathode and holes injected from an anode in the organic layer.

Improvement in the efficiency of devices has been recently made by using a phosphorescence emitting material. As a phosphorescence emitting material, iridium complexes, platinum complexes and the like are known.

In addition to light emitting materials, various materials for improvement of device properties have been proposed. For example, devices which aim to achieve the improvement of chromaticity have been proposed, by using a compound containing a nitrogen-containing heterocyclic group and a carbazole structure as a host material in a light emitting layer (for example, see Patent Documents 1 to 3). Further, for the purpose of improvement of luminous efficiency, devices, which use the compound containing a nitrogen-containing heterocyclic group and a carbazole structure as an electron transporting material in an electron-transporting layer, have been proposed (see Patent Document 4).

Further examples of devices with platinum complexes as phosphorescent emitters and compounds containing nitrogen-containing heterocyclic group and a carbazole structure are found in Patent Documents 5-7.

### Related Art

### Patent Document

Patent Document 1: International Publication No. WO03/080760
Patent Document 2: International Publication No. WO03/078541
Patent Document 3: International Publication No. WO05/085387
Patent Document 4: Japanese Patent Application Laid-Open No. 2007-220721
Patent Document 5: Japanese Patent Application Laid-Open No. 2009-16693
Patent Document 6: Japanese Patent Application Laid-Open No. 2009-170815
Patent Document 7: International Publication No. WO08/117889

### Disclosure of Invention

### Problems to Be Solved by the Invention

As an evaluation of device properties of organic electroluminescence devices, a change in chromaticity according to driving has been conventionally used as an evaluation item along with increase in driving voltage or degradation in efficiency. Further, the evaluation of the environmental temperature from room temperature (mainly as a meaning of an acceleration test) to a high temperature has also been conducted. However, it has been unnoticed that variation in chromaticity increases when driving at a high temperature as compared with when driving at a low temperature. Recently, when it is considered that the use of organic electroluminescence devices, such as the use of displays or panels, and illumination, has been expanding, and the use of a in-vehicle panel which may be maintained at a high temperature of 80°C or more, it may be expected that the change in chromaticity when driving at a high temperature becomes a serious problem.

Although indium complexes are used as light emitting materials in the devices disclosed in Patent Documents 1 to 4, the present inventors have found out that there are large changes in chromaticity in devices disclosed in these documents when driving at a high temperature.

An object of the present invention is to provide an organic electroluminescence device which may be driven at low voltage and have high efficiency and excellent durability, and has a small change in chromaticity when driving at a high temperature. Moreover, another object of the present invention is to provide a light emission apparatus, a display apparatus and an illumination apparatus, including the organic electroluminescence device.

### Means for Solving the Problems

The present inventors have studied to solve the above-mentioned problem, and as a result, found out that the above-mentioned problem may be solved by using a compound having a specific structure containing a nitrogen-containing heterocyclic group and a carbazole structure, and a platinum complex having a specific structure as a light emitting material. That is, the present invention may be accomplished by the following means.
[1] An organic electroluminescence device, having on a substrate: a pair of electrodes; and at least one organic layer including a light emitting layer disposed between the electrodes, wherein a compound represented by the following Formula (1) is contained in a layer directly adjacent to the light-emitting layer,
   and wherein a phosphorescent emitting material represented by the following
   Formula (C-1) is contained in the light emitting layer

   (Cz)p-L-(A)q (1)

   In Formula (1), Cz is a substituted or unsubstituted arylcarbazolyl group or a carbazolylaryl group, L is a single bond or a substituted or unsubstituted arylene group, a substituted or unsubstituted cycloalkylene group, or a substituted or unsubstituted aromatic heterocyclic ring, A is a substituted or unsubstituted nitrogen-containing six-membered aromatic heterocyclic ring, and each of p and q is independently an integer of 1 to 6. 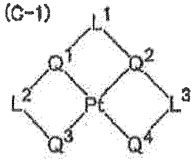
   In Formula (C-1), each of Q¹, Q², Q³ and Q⁴ independently represents a ligand which is coordinated to Pt, and each of L¹, L² and L³ independently represents a single bond or a divalent linking group and whereby Q³ and Q⁴ are not linked.
[2] The organic electroluminescence device of [1], wherein the compound represented by Formula (1) is a compound represented by the following Formula (2). 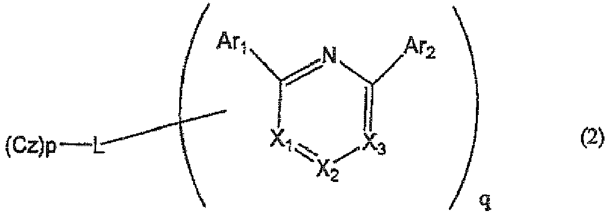
   In Formula (2), Cz represents a substituted or unsubstituted arylcarbazolyl group or carbazolylaryl group, L represents a single bond, a substituted or unsubstituted arylene group, a substituted or unsubstituted cycloalkylene group, or a substituted or unsubstituted aromatic heterocyclic ring, and is linked to a carbon atom of Ar₁, Ar₂, X₁, X₂ or X₃, each of Ar₁ and Ar₂ independently represents a substituted or unsubstituted aryl group, or a substituted or unsubstituted aromatic heterocyclic group, and each of X₁, X₂ and X₃ independently represents a nitrogen atom or a hydrogen atom, or a carbon atom to which a substituent is bound, and each of p and q independently represents an integer of 1 to 6.
[3] The organic electroluminescence device of [2], wherein in Formula (2), at least one of X₁ and X₃ is a nitrogen atom.
[4] The organic electroluminescence device of [2] or [3], wherein in Formula (2), each of Ar₁ and Ar₂ is independently a phenyl group or a biphenyl group.
[5] The organic electroluminescence device of any one of [1] to [4], wherein an aryl group in the arylcarbazolyl group or carbazolylaryl group is substituted at the 9-position on a carbazole ring.
[6] The organic electroluminescence device of any one of [1] to [5], wherein in Formula (1), Cz is a carbazolylaryl group.
[7] The organic electroluminescence device of any one of [1] to [6], wherein the compound represented by Formula (1) is a compound represented by the following Formula (3). 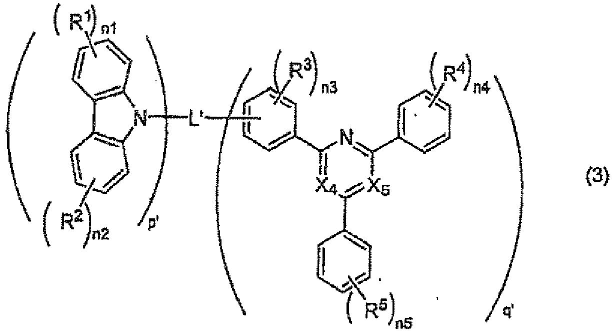
   In Formula (3), each of X₄ and X₅ independently represents a nitrogen atom or a hydrogen atom, or a carbon atom to which a substituent is bound, and one of X₄ and X₅ is a nitrogen atom and the other is a hydrogen atom or a carbon atom to which a substituent is bound, L' represents a single bond, a substituted or unsubstituted arylene group, a substituted or unsubstituted cycloalkylene group, or a substituted or unsubstituted aromatic heterocyclic ring, each of R¹ to R⁵ independently represents a substituent, each of n1 to n5 independently represents an integer of 0 to 5. Each of p' and q' independently represents an integer of 1 to 4.
[8] The organic electroluminescence device of any one of [1] to [7], wherein the compound represented by Formula (1) is composed only of a carbon atom, a hydrogen atom and a nitrogen atom.
[9] The organic electroluminescence device of any one of [1] to [8], wherein the compound represented by Formula (1) has a molecular weight of 450 to 800.
[10] The organic electroluminescence device of any one of [1] to [9], wherein the compound represented by Formula (1) has a lowest triplet excited state (T₁) energy of 2.61 eV to 3.51 eV in a film state.
[11] The organic electroluminescence device of any one of [1] to [10], wherein the compound represented by Formula (1) has a glass transition temperature Tg of 80°C to 400°C.
[12] The organic electroluminescence device of [1], wherein the phosphorescence emitting material represented by Formula (C-1) is represented by Formula (C-2). 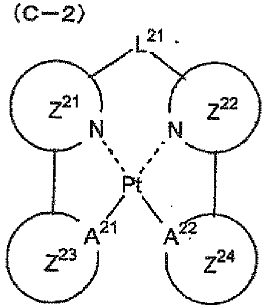
   In Formula (C-2), L²¹ represents a single bond or a divalent linking group, each of A²¹ and A²² independently represents a carbon atom or a nitrogen atom, each of Z²¹ and Z²² independently represents a nitrogen-containing aromatic heterocyclic ring, and each of Z²³ and Z²⁴ independently represents a benzene ring or an aromatic heterocyclic ring.
[13] The organic electroluminescence device of any one of [1] to [12], wherein the compound represented by Formula (1) is contained in a layer adjacent to the light emitting layer.
[14] The organic electroluminescence device of any one of [1] to [13], wherein a maximum light emission wavelength is 400 nm to 465 nm.
[15] The organic electroluminescence device of any one of [1] to [14], wherein the light emitting material is contained in the light emitting layer in an amount of 1 mass% to 30 mass%.
[16] The organic electroluminescence device of any one of [1] to [15], having a hole injection layer or a hole transporting layer containing an electron-accepting dopant as an organic layer.
[17] A composition, having: the compound represented by Formula (1) and the phosphorescence emitting material represented by Formula (C-1), as defined in [1].
[18] The composition of [17], further having a compound represented by the following Formula (PQ-1). 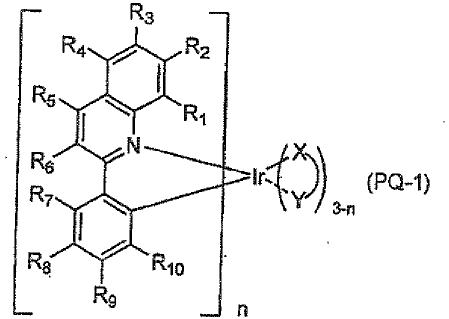
   In Formula PQ-1, each of R₁ to R₁₀ represents a hydrogen atom or a substituent, the substituent may be bonded to each other to form a ring if possible, X-Y represents a mono-anionic bidentate ligand, and n represents an integer of 1 to 3.
[19] A light emitting layer, having: the compound represented by Formula (1) and the phosphorescence emitting material represented by Formula (C-1), as defined in [1].
[20] The light emitting layer of [19], further having a compound represented by the following Formula (PQ-1). 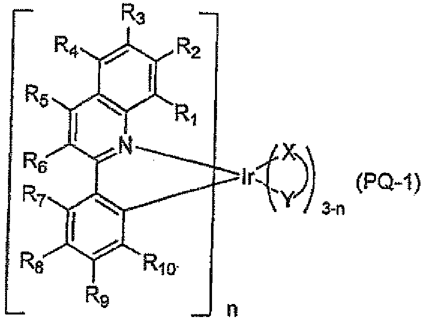
   In Formula, each of R₁ to R₁₀ independently represents a hydrogen atom or a substituent, the substituents may be bonded to each other to form a ring if possible, X-Y represents a mono-anionic bidentate ligand, and n represents an integer of 1 to 3.
[21] A light emission apparatus using the organic electroluminescence device of any one of [1] to [16].
[22] A display apparatus using the organic electroluminescence device of any one of [1] to [16].
[23] An illumination apparatus using the organic electroluminescence device of any one of [1] to [16].

### Effects of the Invention

The organic electroluminescence device of the present invention may reduce electric power consumption by driving at low voltage, and has high luminous efficiency and excellent durability. Further, the organic electroluminescence device of the present invention has a small change in chromaticity when driving at a high temperature, and thus, is also appropriate for the use of which the driving durability is required in a high temperature environment, such as an in-vehicle use.

### Brief Description of the Drawings

FIG. 1 is a schematic view illustrating an example of the configuration of an organic electroluminescence device according to the present invention.
FIG. 2 is a schematic view illustrating an example of a light emission apparatus according to the present invention.
FIG. 3 is a schematic view illustrating an example of an illumination apparatus according to the present invention.

### Embodiments for Carrying Out the Invention

The organic electroluminescence device of the present invention includes a pair of electrodes and at least one organic layer including a light emitting layer between the electrodes, on a substrate as defined in claim 1. An organic electroluminescence device having excellent driving voltage, light emission efficiency, durability and the like, and a small change in chromaticity when driving at a high temperature, may be obtained by using a compound represented by Formula (1) and a phosphorescence emitting material represented by Formula (C-1). Although the reason why the change in chromaticity is suppressed when driving at a high temperature is not clearly proved, the following may be contemplated. That is, since a chemical reaction is promoted when driving at a high temperature, it may be contemplated that a light emitting material and a host material undergoes an unexpected chemical reaction in the light emitting layer. Likewise, it may be considered that a certain chemical reaction between the light emitting material and an adjacent layer material is also undergone at the interface of the light emitting layer and the adjacent layer. As a result, it is thought that the change in chromaticity is caused for the reasons that the light emission position is changed, or side light emitting components are produced. However, since it is difficult for a chemical reaction to take place between the compound represented by Formula (1) and the phosphorescence emitting material represented by Formula (C-1) even when driving at a high temperature, it is thought that it is difficult for the change in chromaticity to take place.

Further, a hydrogen atom in the descriptions of the following Formulas (1) to (3), Formulas (PQ-1) and (PQ-2), Formulas (C-1) to (C-6), and Formula (IV) also includes isotopes (a deuterium atom and the like), and furthermore, an atom constituting a substituent also includes isotopes thereof.

Hereinafter, the compound represented by Formula (1) will be described.

(Cz)p-L-(A)q (1)

In Formula (1), Cz represents a substituted or unsubstituted arylcarbazolyl group or a carbazolylaryl group. L represents a single bond, a substituted or unsubstituted arylene group, a substituted or unsubstituted cycloalkylene group or a substituted or unsubstituted aromatic heterocyclic ring. A is a substituted or unsubstituted nitrogen-containing six-membered heteroaromatic ring, and each of p and q independently represents an integer of 1 to 6.

Formula (1) will be described.

Cz is a substituted or unsubstituted arylcarbazolyl group or a substituted or unsubstituted carbazolylaryl group.

The aryl group in the arylcarbazolyl group and carbazolylaryl group has preferably 6 to 30 carbon atoms, and may be, for example, a phenyl group, a naphthyl group, an anthryl group, a phenanthryl group, a naphthacenyl group, a pyrenyl group, a fluorenyl group, a biphenyl group, a terphenyl group, and among them, preferably phenyl group, naphthyl group, biphenyl group and terphenyl group, and more preferably a phenyl group and a biphenyl group.

The substitution position of the aryl group on the carbazole ring (carbazolyl group) in the arylcarbazolyl group and carbazolylaryl group is not particularly limited, but from the viewpoint of chemical stability or carrier transportability, it is preferable that the aryl group is substituted at the 2-, 3-, 6-, 7- or 9-position of the carbazole ring, it is more preferable that the aryl group is substituted at the 3-, 6- or 9-position of the carbazole ring, and it is most preferable that the aryl group is substituted at the 9-position (N-position) of the carbazole ring.

When Cz is the arylcarbazolyl group, the position is not particularly limited, but from the viewpoint of chemical stability or carrier transportability, it is preferable that the group is linked to L at the 2-, 3-, 6-, 7- or 9-position (N-position) of the carbazole ring of the arylcarbazolyl group, it is more preferable that the group is linked to L at the 3-, 6- or 9-position (N-position) of the carbazole ring, and it is most preferable that the group is linked to L at the 9-position (N-position) of the carbazole ring.

Further, it is preferable that Cz is a carbazolylaryl group which may be substituted with an alkyl group, a silyl group, an aryl group, a cyano group or a carbazolyl group, and it is more preferable that Cz is a carbazolylaryl group which may be substituted with an ethyl group, a t-butyl group, a triphenylsilyl group, a phenyl group, a cyano group or a carbazolyl group.

A is a substituted or unsubstituted nitrogen-containing aromatic heterocyclic six-membered ring, and preferably a nitrogen-containing aromatic heterocyclic six-membered ring having 2 to 40 carbon atoms. A may have a plurality of substituents, and the substituents may be linked to each other to form a ring.

The nitrogen-containing aromatic heterocyclic six-membered ring or nitrogen-containing aromatic heterocyclic ring containing the nitrogen-containing aromatic heterocyclic six-membered ring may be pyridine, pyrimidine, pyrazine, pyridazine, triazine, azaindolizine, indolizine, purine, pteridine, β-carboline, naphthyridine, quinoxaline, terpyridine, bipyridine, acridine, phenanthroline, phenazine and imidazopyridine, and among them, more preferably pyridine, pyrimidine, pyrazine and triazine, and most preferably pyrimidine.

L is a single bond, a substituted or unsubstituted arylene group, a substituted or unsubstituted cycloalkylene group and a substituted or unsubstituted aromatic heterocyclic ring.

Further, in Formula (1), when p + q is 3 or more, L represents a p+q valent group obtained by subtracting any p+q-2 hydrogen atoms from the arylene group, a p+q valent group obtained by subtracting any p+q-2 hydrogen atoms from the cycloalkylene group, or a p+q valent aromatic heterocyclic group.

As a substituent of L, those exemplified above for the group A of substituents may be applied, and the substituent may be preferably a methyl group, an ethyl group, a propyl group, a butyl group, a cyclohexyl group, a cyclopentyl group, a phenyl group, a tolyl group, a xylyl group, a pyridyl group, a pyrimidyl group, a thienyl group, a fluorine atom, a cyano group, a trifluoromethyl group, a pentafluorophenyl group, a triphenylsilyl group and a trimethylsilyl group, more preferably a methyl group, an ethyl group, a butyl group, a phenyl group, a pyridyl group, a pyrimidyl group, a fluorine atom, a cyano group and a trifluoromethyl group, and even more preferably a methyl group, a phenyl group and a fluorine atom.

The arylene group is preferably an arylene group having 6 to 30 carbon atoms, and may include, for example, a phenylene group, a biphenylene group, a terphenylene group, a naphthylene group, an anthracenediyl group, a phenanthrene group, a vinylene group, a chrysenylene group, a fluoranthenylene group, and a perfluoroarylene group, and among them, preferably a phenylene group, a biphenylene group, a terphenylene group and a perfluoroarylene group, more preferably a phenylene group, a biphenylene group and a terphenylene group, and even more preferably a phenylene group and a biphenylene group.

The cycloalkylene group is preferably an cycloalkylene group having 5 to 30 carbon atoms, and may include, for example, a cyclopentylene group, a cyclohexylene group and a cycloheptylene group, and among them, preferably a cyclopentylene group and cyclohexylene group, and moer preferably a cyclohexylene group.

The aromatic heterocyclic is preferably an aromatic heterocyclic having 2 to 30 carbon atoms and may include a 1-pyrrolyl group, a 2-pyrrolyl group, a 3-pyrrolyl group, a pyrazynyl group, a 2-pyridinyl group, a 3-pyridinyl group, a 4-pyridinyl group, a 1-indolyl group, a 2-indolyl group, a 3-indolyl group, a 4-indolyl group, a 5-indolyl group, a 6-indolyl group, a 7-indolyl group, a 1-isoindolyl group, a 2-isoindolyl group, a 3-isoindolyl group, a 4-isoindolyl group, a 5-isoindolyl group, a 6-isoindolyl group, a 7-isoindolyl group, a 2-furyl group, a 3-furyl group, a 2-benzofuranyl group, a 3-benzofuranyl group, a 4-benzofuranyl group, a 5-benzofuranyl group, a 6-benzofuranyl group, a 7-benzofuranyl group, a 1-isobenzofuranyl group, a 3-isobenzofuranyl group, a 4-isobenzofuranyl group, a 5-isobenzofuranyl group, a 6-isobenzofuranyl group, a 7-isobenzofuranyl group, a 2-quinolyl group, a 3-quinolyl group, a 4-quinolyl group, a 5-quinolyl group, a 6-quinolyl group, a 7-quinolyl group, a 8-quinolyl group, a 1-isoquinolyl group, a 3-isoquinolyl group, a 4-isoquinolyl group, a 5-isoquinolyl group, a 6-isoquinolyl group, a 7-isoquinolyl group, a 8-isoquinolyl group, a 2-quinoxalinyl group, a 5-quinoxalinyl group, a 6-quinoxalinyl group, a 1-carbazolyl group, a 2-carbazolyl group, a 3-carbazolyl group, a 4-carbazolyl group, a 9-carbazolyl group, a 1-phenanthridinyl group, a 2-phenanthridinyl group, a 3-phenanthridinyl group, a 4-phenanthridinyl group, a 6-phenanthridinyl group, a 7-phenanthridinyl group, a 8-phenanthridinyl group, a 9-phenanthridinyl group, a 10-phenanthridinyl group, a 1-acrydinyl group, a 2-acrydinyl group, a 3-acrydinyl group, a 4-acrydinyl group, a 9-acrydinyl group, a 1,7-phenanthroline-2-yl group, a 1,7-phenanthroline-3-yl group, a 1,7-phenanthroline-4-yl group, a 1,7-phenanthroline-5-yl group, a 1,7-phenanthroline-6-yl group, a 1,7-phenanthroline-8-yl group, a 1,7-phenanthroline-9-yl group, a 1,7-phenanthroline-10-yl group, a 1,8-phenanthroline-2-yl group, a 1,8-phenanthroline-3-yl group, a 1,8-phenanthroline-4-yl group, a 1,8-phenanthroline-5-yl group, a 1,8-phenanthroline-6-yl group, a 1,8-phenanthroline-7-yl group, a 1,8-phenanthroline-9-yl group, a 1,8-phenanthroline-10-yl group, a 1,9-phenanthroline-2-yl group, a 1,9-phenanthroline-3-yl group, a 1,9-phenanthroline-4-yl group, a 1,9-phenanthroline-5-yl group, a 1,9-phenanthroline-6-yl group, a 1,9-phenanthroline-7-yl group, a 1,9-phenanthroline-8-yl group, a 1,9-phenanthroline-10-yl group, a 1,10-phenanthroline-2-yl group, a 1,10-phenanthroline-3-yl group, a 1,10-phenanthroline-4-yl group, a 1,10-phenanthroline-5-yl group, a 2,9-phenanthroline-1-yl group, a 2,9-phenanthroline-3-yl group, a 2,9-phenanthroline-4-yl group, a 2,9-phenanthroline-5-yl group, a 2,9-phenanthroline-6-yl group, a 2,9-phenanthroline-7-yl group, a 2,9-phenanthroline-8-yl group, a 2,9-phenanthroline-10-yl group, a 2,8-phenanthroline-1-yl group, a 2,8-phenanthroline-3-yl group, a 2,8-phenanthroline-4-yl group, a 2,8-phenanthroline-5-yl group, a 2,8-phenanthroline-6-yl group, a 2,8-phenanthroline-7-yl group, a 2,8-phenanthroline-9-yl group, a 2,8-phenanthroline-10-yl group, a 2,7-phenanthroline-1-yl group, a 2,7-phenanthroline-3-yl group, a 2,7-phenanthroline-4-yl group, a 2,7-phenanthroline-5-yl group, a 2,7-phenanthroline-6-yl group, a 2,7-phenanthroline-8-yl group, a 2,7-phenanthroline-9-yl group, a 2,7-phenanthroline-10-yl group, a 1-phenazinyl group, a 2-phenazinyl group, a 1-phenothiazinyl group, a 2-phenothiazinyl group, a 3-phenothiazinyl group, a 4-phenothiazinyl group, a 10-phenothiazinyl group, a 1-phenoxazinyl group, a 2-phenoxazinyl group, a 3-phenoxazinyl group, a 4-phenoxazinyl group, a 10-phenoxazinyl group, a 2-oxazolyl group, a 4-oxazolyl group, a 5-oxazolyl group, a 2-oxadiazolyl group, a 5-oxadiazolyl group, a 3-furazanyl group, a 2-thienyl group, a 3-thienyl group, a 2-methylpyrrole-1-yl group, a 2-methylpyrrole-3-yl group, a 2-methylpyrrole-4-yl group, a 2-methylpyrrole-5-yl group, a 3-methylpyrrole-1-yl group, a 3-methylpyrrole-2-yl group, a 3-methylpyrrole-4-yl group, a 3-methylpyrrole-5-yl group, a 2-t-butylpyrrole-4-yl group, a 3-(2-phenylpropyl)pyrrole-1-yl group, a 2-methyl-1-indolyl group, a 4-methyl-1-indolyl group, a 2-methyl-3-indolyl group, a 4-methyl-3-indolyl group, a 2-t-butyl-1-indolyl group, a 4-t-butyl-1-indolyl group, a 2-t-butyl-3-indolyl group and a 4-t-butyl-3-indolyl group, and among them, preferably a pyridinyl group, a quinolyl group, an indolyl group and a carbazolyl group, and more preferably a pyridinyl group and a carbazolyl group.

L is preferably a single bond, a phenylene group, a biphenylene group, a cyclopentylene group, a cyclohexylene group, a pyridinyl group and a carbazolyl group, more preferably a single bond, a phenylene group and a biphenylene group, and even more preferably a single bond and a phenylene group.

Further, the substituents of Cz, A and L in Formula (1) may include a halogen atom such as fluorine, chlorine, bromine and iodine, a carbazolyl group, a hydroxyl group, a substituted or unsubstituted amino group, a nitro group, a cyano group, a silyl group, a trifluoromethyl group, a carbonyl group, a carboxyl group, a substituted or unsubstituted alkyl group, a substituted or unsubstituted alkenyl group, a substituted or unsubstituted arylalkyl group, a substituted or unsubstituted aromatic group, a substituted or unsubstituted aromatic heterocyclic group, a substituted or unsubstituted aralkyl group, a substituted or unsubstituted aryloxy group and a substituted or unsubstituted alkyloxy group. Among them, a fluorine atom, a methyl group, a perfluorophenylene group, a phenyl group, a naphthyl group, a pyridyl group, a pyrazyl group, a pyrimidyl group, an adamantyl group, a benzyl group, a nitro group, a cyano group, a silyl group, a trifluoromethyl group, a carbazolyl group and a group formed in combinations thereof are preferable, a fluorine atom, a methyl group, a phenyl group, a pyridyl group, a pyrimidyl group, a cyano group, a silyl group, a carbazolyl group and a group formed in combinations thereof are more preferable, a phenyl group, a pyridyl group, a pyrimidyl group, a carbazolyl group and a group formed in combinations thereof are even more preferable, and a phenyl group is most preferable.

Each of p and q independently represents an integer of 1 to 6, preferably 1 to 4, more preferably 1 to 3, and even more preferably 1 or 2.

It is more preferable that the compound represented by Formula (1) is a compound represented by the following Formula (2).

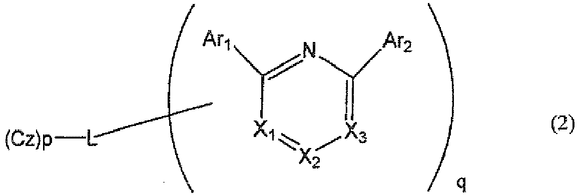

In Formula (2), Cz represents a substituted or unsubstituted arylcarbazolyl group or a carbazolylaryl group. L represents a single bond, a substituted or unsubstituted arylene group, a substituted or unsubstituted cycloalkylene group or a substituted or unsubstituted aromatic heterocyclic ring, and is linked to carbon atoms of Ar₁, Ar₂, X₁, X₂ or X₃. Each of Ar₁ and Ar₂ independently represents a substituted or unsubstituted aryl group or a substituted or unsubstituted aromatic heterocyclic group, each of X₁, X₂ and X₃ independently represents a nitrogen atom or a carbon atom to which a hydrogen atom or a substituent is bonded. Each of p and q independently represents an integer of 1 to 6.

Formula (2) will be described.

In Formula (2), definitions of Cz, L, p and q are the same as those of Cz, L, p and q in Formula (1), and preferable groups are the same, too.

Each of Ar₁ and Ar₂ independently represents a substituted or unsubstituted aryl group, a substituted or unsubstituted arylene group or a substituted or unsubstituted aromatic heterocyclic group.

The aryl group preferably has 6 to 30 substituted or unsubstituted carbon atoms and may include, for example, a phenyl group, a biphenyl group, a terphenyl group, a naphthyl group, an anthryl group, a phenanthryl group, a pyrenyl group, a chrysenyl group, a fluoranthenyl group and a perfluoroaryl group, and among them, preferably a phenyl group, a biphenyl group, a terphenyl group and a perfluoroaryl group, more preferably a phenyl group, a biphenyl group and a terphenyl group, and even more preferably a phenyl group and a biphenyl group.

The arylene group preferably has 6 to 30 substituted or unsubstituted carbon atoms and particular examples or preferable groups are the same as those in the description of L in the aforementioned Formula (1).

The aromatic heterocyclic group preferably has 2 to 30 substituted or unsubstituted carbon atoms, and particular examples or preferable groups are the same as those in the description of L in the aforementioned Formula (1). In the case where a substituent is bonded thereto, particular examples or preferable groups of the substituent are the same as the substituents of Cz, A and L in the aforementioned Formula (1).

Each of Ar₁ and Ar₂ preferably independently represents a phenyl group which may be substituted with a carbazolyl group or an unsubstituted terphenyl group, and more preferably a phenyl group which may be substituted with a carbazolyl group.

Each of X₁, X₂ and X₃ independently represents a nitrogen atom or a carbon atom to which a hydrogen atom or a substituent is bonded. The case where 0 to 2 of X₁, X₂ and X₃ are a nitrogen atom is preferable, the case where 0 to 1 of X₁, X₂ and X₃ is a nitrogen atom is more preferable, and the case where 1 of X₁, X₂ and X₃ is a nitrogen atom is most preferable. In the case where a nitrogen atom is contained in any one of X₁, X₂ and X₃, it is preferable that either one of X₁ and X₃ is a nitrogen atom. Particular examples or preferable groups of the substituent bonded to a carbon atom are the same as the substituents of Cz, A and L in the aforementioned Formula (1). Further, the connection position of L in Formula (2) is not particularly limited, but from the viewpoint of chemical stability or carrier transportability, it is preferable that L is linked to a carbon atom of Ar₁.

The compound represented by Formula (1) is more preferably a compound represented by the following Formula (3).

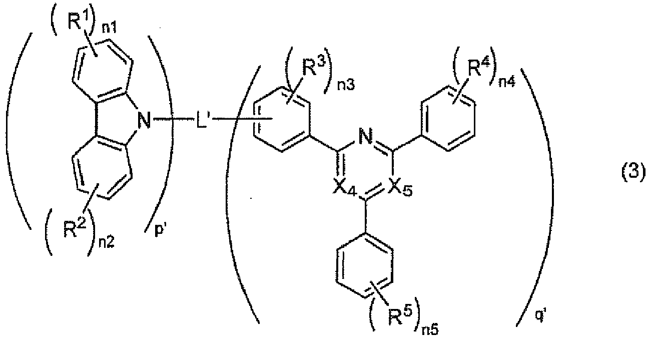

In Formula (3), each of X₄ and X₅ independently represents a nitrogen atom or a carbon atom to which a hydrogen atom or a substituent is bonded, and either one of X₄ and X₅ is a nitrogen atom, the other is a carbon atom to which a hydrogen atom or a substituent is bonded. L' represents a single bond, a substituted or unsubstituted arylene group, a substituted or unsubstituted cycloalkylene group or a substituted or unsubstituted aromatic heterocyclic ring. Each of R¹ to R⁵ independently represents a substituent. Each of n1 to n5 independently represents an integer of 0 to 5. Each of p' and q' independently represents an integer of 1 to 4.

Formula (3) will be described.

Each of X₄ and X₅ independently represents a nitrogen atom or a carbon atom to which a hydrogen atom or a substituent is bonded. It is preferable that either one of X₄ and X₅ is a nitrogen atom, and the other is a carbon atom to which a hydrogen atom or a substituent is bonded. In Formula (3), it is preferable that the ring containing X₄ and X₅ represents pyridine or pyrimidine, and it is more preferable that the ring containing X₄ and X₅ represents pyrimidine. Particular examples or preferable groups of the substituent bonded to the carbon atom are the same as the substituents of Cz, A and L in the aforementioned Formula (1).

The definition of L' is the same as that of L in Formula (1), and the preferable group is the same as L. L' is linked to the benzene ring connected to R³ in the nitrogen-containing aromatic heterocyclic structure of Formula (3).

Each of R¹ to R⁵ independently represents a substituent. The particular examples of the substituent are the same as examples of the substituents of Cz and A in Formula (1). R¹ to R⁵ is preferably a fluorine atom, a methyl group, a t-butyl group, a phenyl group, a pyridyl group, a pyrazyl group, a pyrimidyl group, an adamantyl group, a cyano group, a trimethylsilyl group, a triphenylsilyl group, a trifluoromethyl group and a carbazolyl group, more preferably a fluorine atom, a methyl group, a t-butyl group, a phenyl group, a pyridyl group, a cyano group, a trimethylsilyl group, a triphenylsilyl group, a trifluoromethyl group and a carbazolyl group, even more preferably a fluorine atom, a methyl group, a t-butyl group, a phenyl group, a cyano group, a silyl group, a triphenylsilyl group, a trifluoromethyl group and a carbazolyl group, and still even more preferably a fluorine atom, a t-butyl group, a phenyl group, a cyano group, a triphenylsilyl group and a carbazolyl group. When there are a plurality of R¹ to R⁵, each of R¹ to R⁵ may be the same as or different from every other R¹ to R⁵.

Each of n1 to n5 independently represents an integer of 0 to 5. The integer is preferably 0 to 2, more preferably 0 to 1, and even more preferably 0.

Each of p' and q' independently represents an integer of 1 to 4. The integer is preferably 1 to 3, and more preferably 1 or 2.

Preferably, in Formula (3), each of X₄ and X₅ independently represents a nitrogen atom or a carbon atom to which a hydrogen atom is bonded, the ring containing X₄ and X₅ is pyridine or pyrimidine, L' represents a single bond or a phenylene group, each of R¹ to R⁵ independently represents a methyl group, a phenyl group, a cyano group, a pyridyl group, a pyrimidyl group, a silyl group, a carbazolyl group or a tert-butyl group, each of n1 to n5 independently represents 0 or 1, and each of p' and q' independently represents 1 or 2.

Further, in Formula (3), in the case where p'+q' is 3 or more, L' represents a p'+q' valent group obtained by subtracting any p'+q'-2 hydrogen atoms from the phenylene group.

The case where the compound represented by Formula (1) is formed of only carbon atoms, hydrogen atoms and nitrogen atoms is most preferable.

A molecular weight of the compound represented by Formula (1) is preferably 400 or more and 1,000 or less, more preferably 450 or more and 800 or less, and even more preferably 500 or more and 700 or less.

The lowermost triplet excited state (T₁) energy of the compound represented by Formula (1) in a film state is preferably 2.61 eV (62 kcal/mol) or more and 3.51 eV (80 kcal/mol) or less, more preferably 2.69 eV (63.5 kcal/mol) or more and 3.51 eV (80 kcal/mol) or less, and even more preferably 2.76 eV (65 kcal/mol) or more and 3.51 eV (80 kcal/mol).

The T₁ energy may be determined from a short wavelength limit which is obtained by measuring a phosphorescence emission spectrum of a thin film of the material. For example, the material is formed into a film having a thickness of about 50 nm on a cleaned quartz glass substrate by vacuum deposition, and the phosphorescence emission spectrum of the thin film is determined by using a F-7000 Hitachi fluorescence spectrophotometer (Hitachi High-Technologies Corporation) under a liquid nitrogen temperature. The T₁ energy may be determined by converting a rising wavelength on the short wavelength side of the obtained emission spectrum into an energy unit.

A glass transition temperature (Tg) of the compound represented by Formula (1) is preferably 80° C or more and 400° C or less, more preferably 100° C or more and 400° C or less, and even more preferably 120° C or more and 400° C or less.

Hereinafter, particular examples of the compound represented by Formula (1) are exemplified, but the present invention is not limited thereto. Further, Ph in the following particular examples represents a phenyl group. However, the compounds 32, 34, 35, 36, 38, 39, 41, 47, 49, 51, 52, 65 and 74 do not form part of the invention.

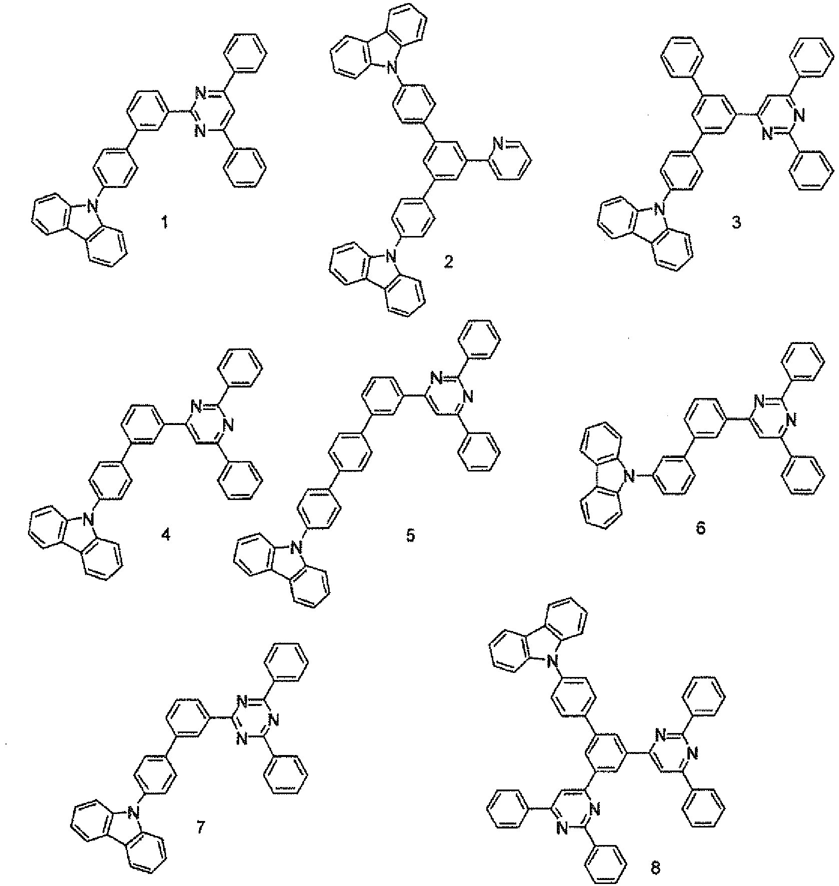

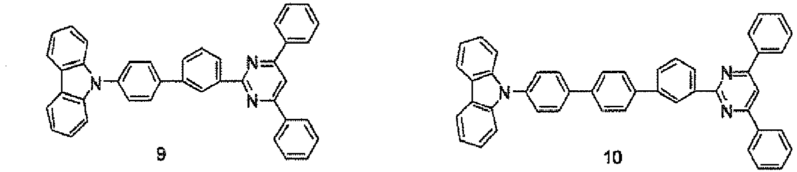

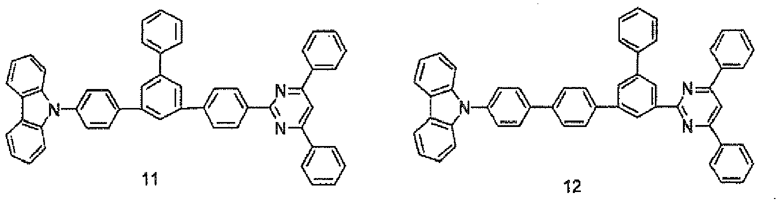

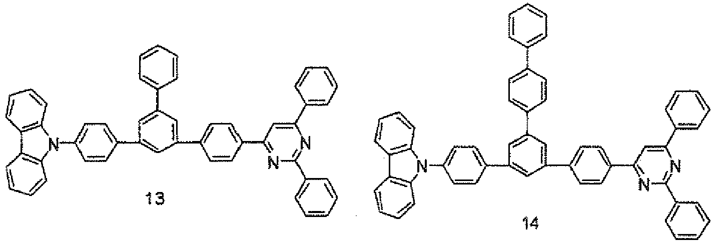

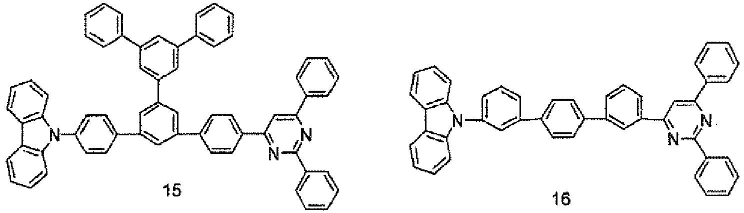

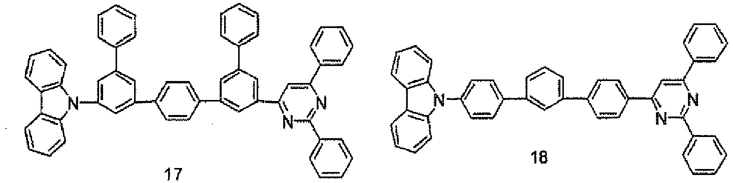

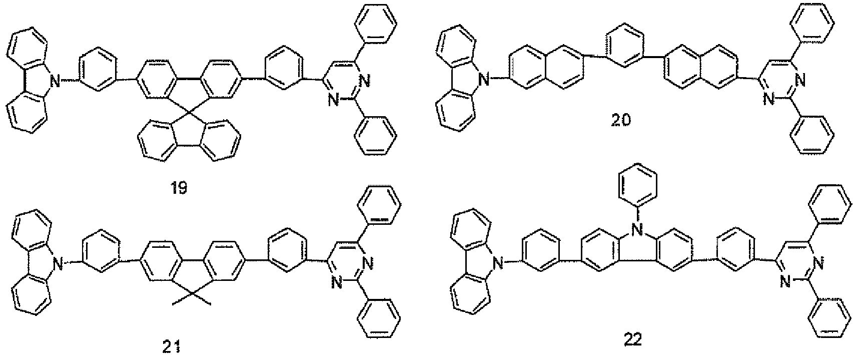

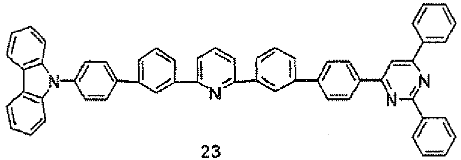

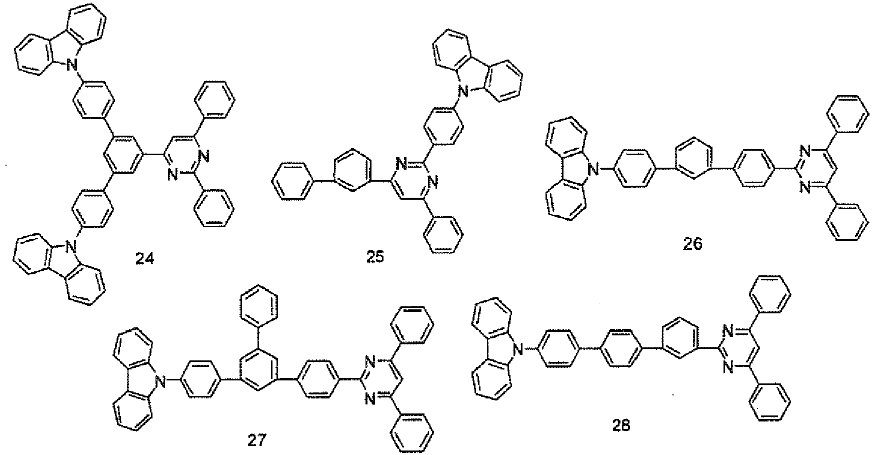

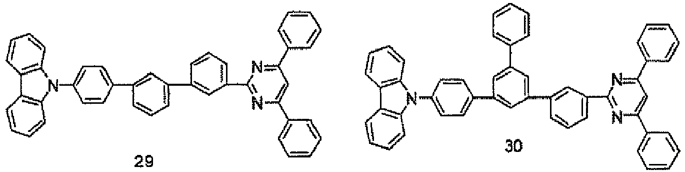

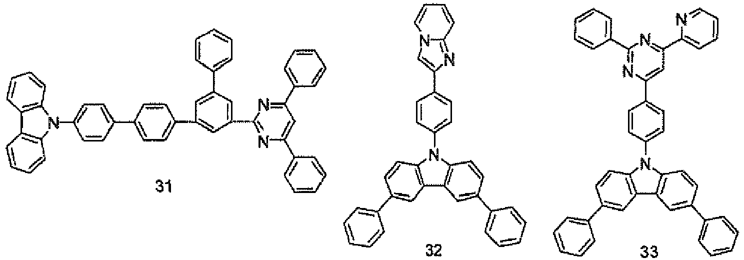

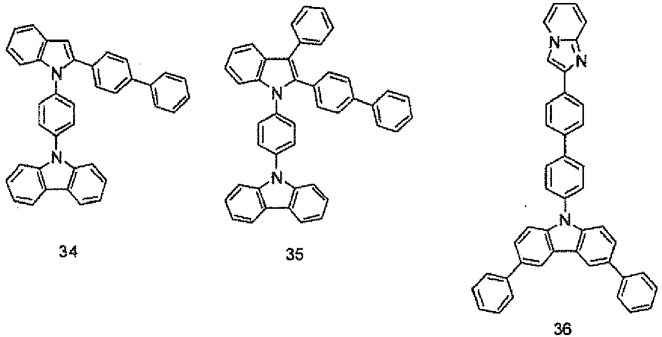

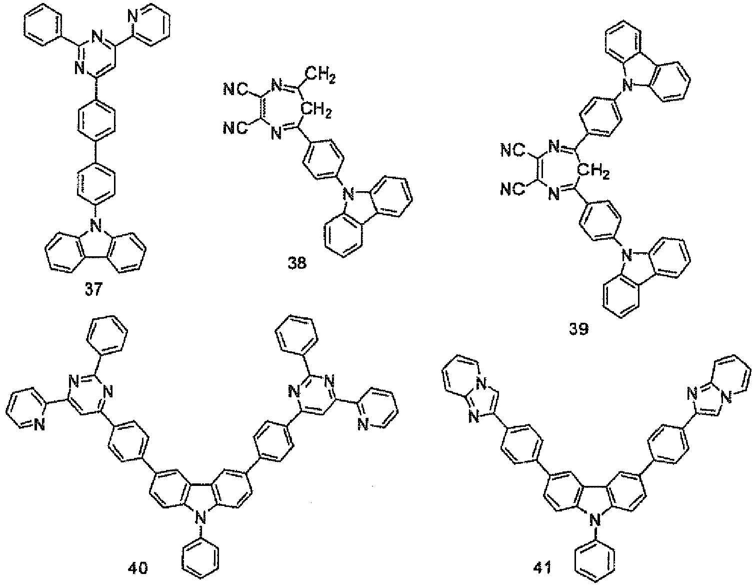

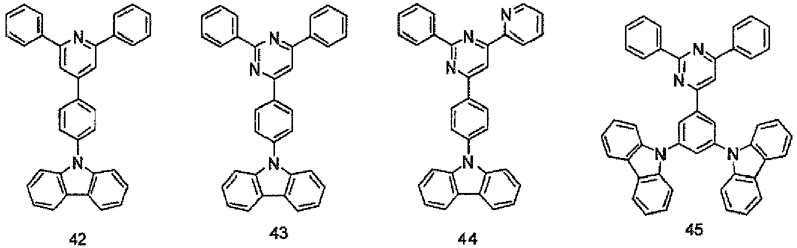

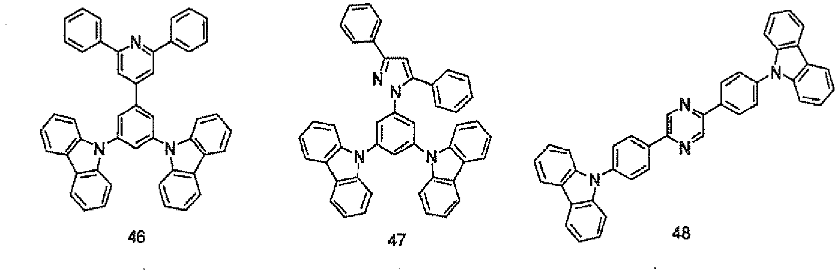

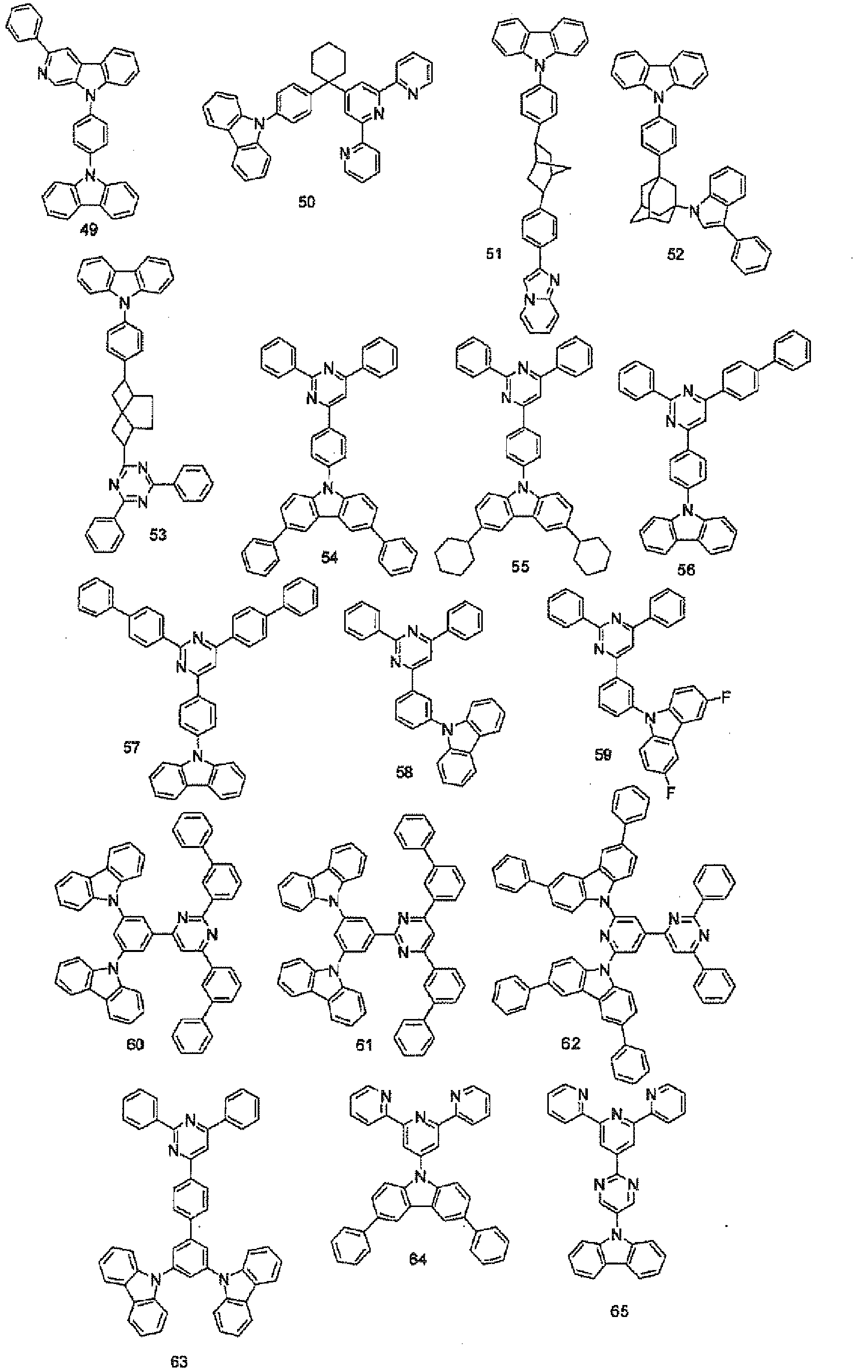

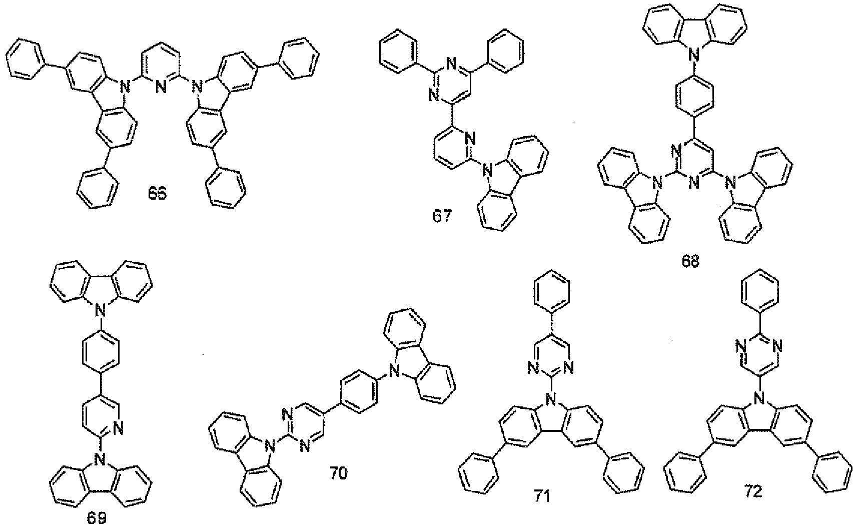

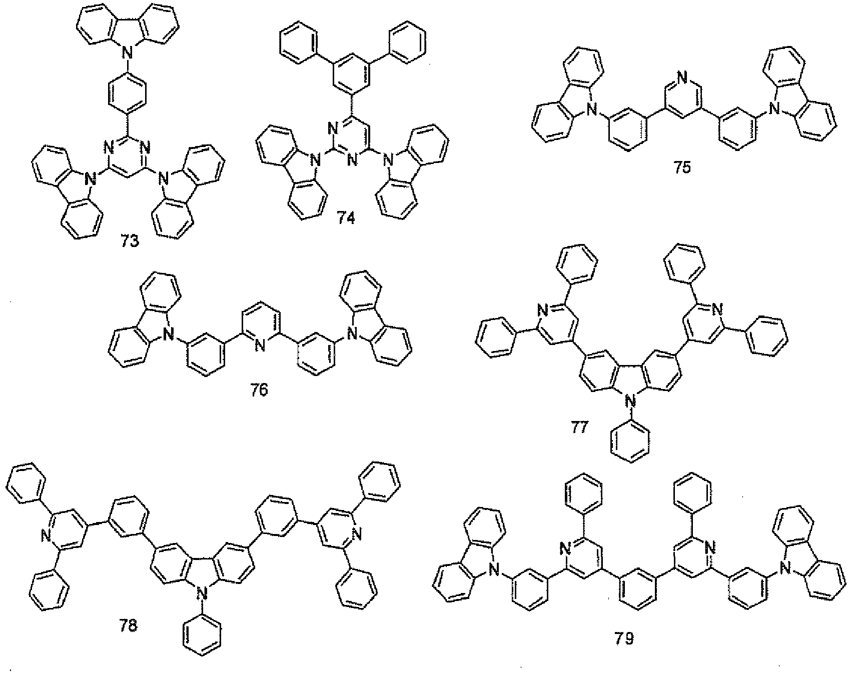

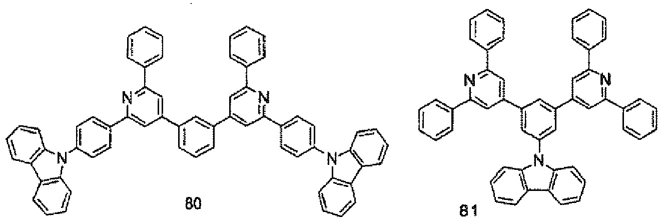

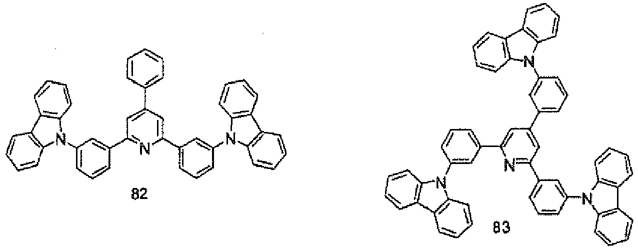

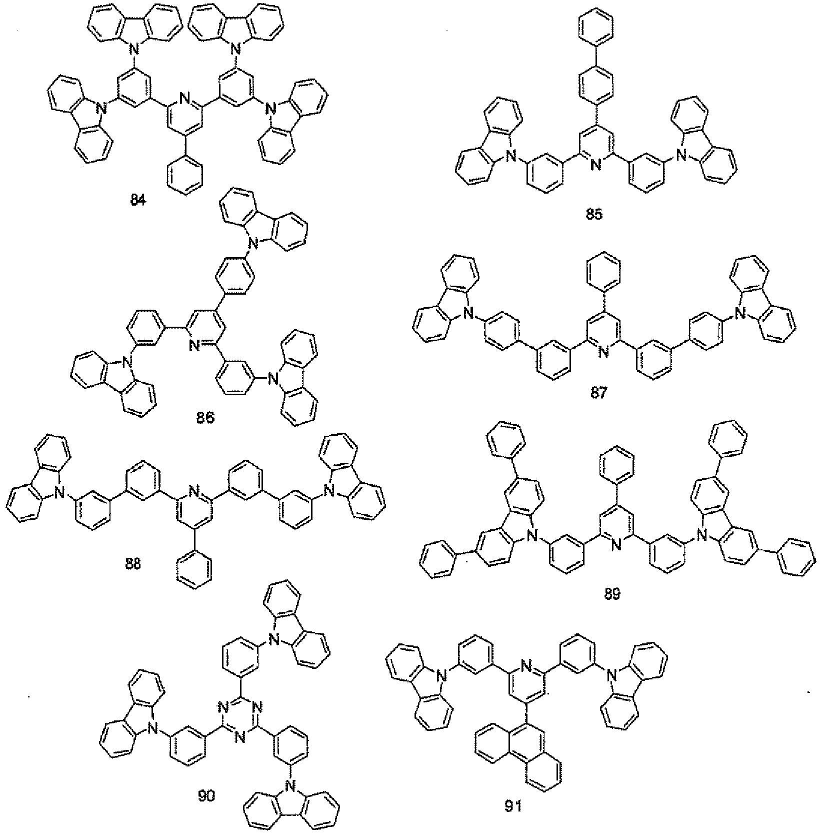

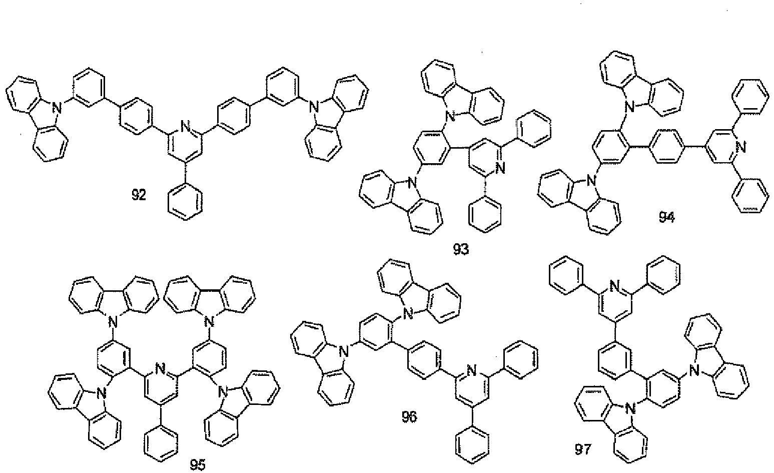

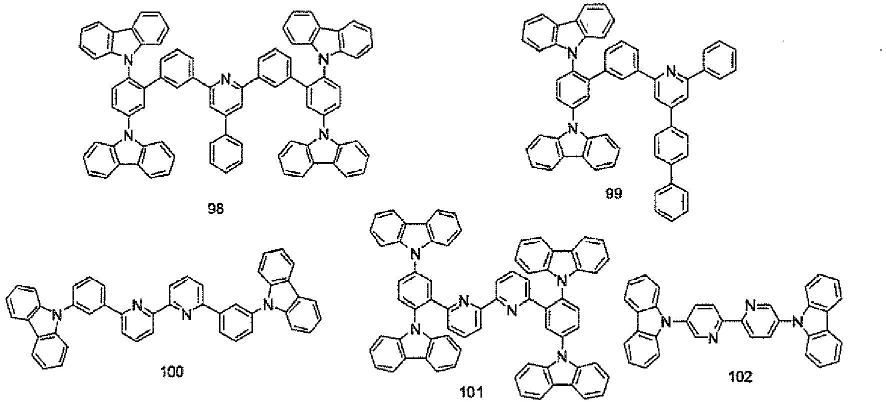

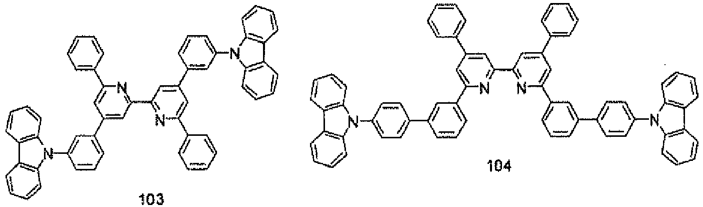

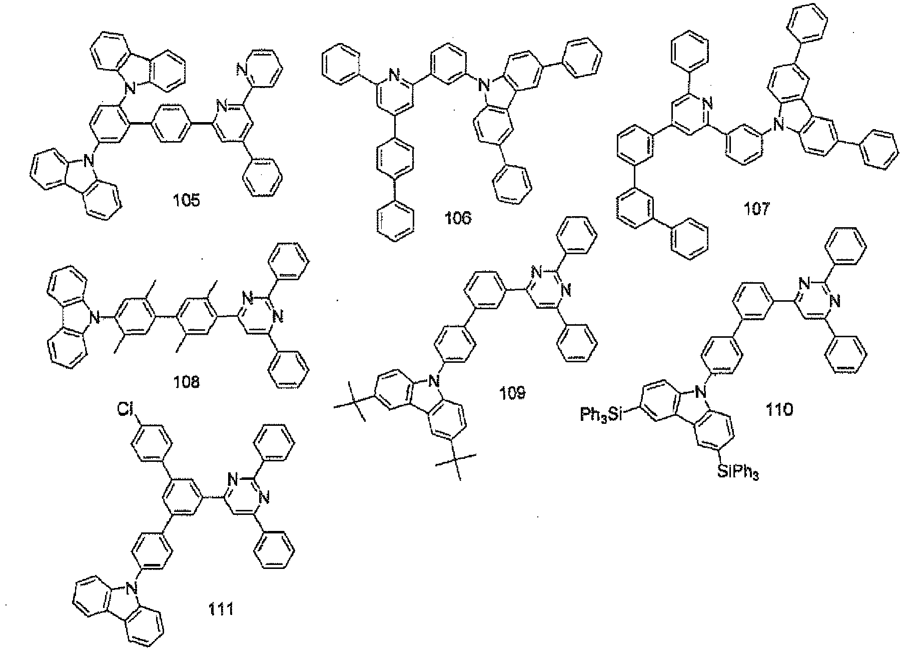

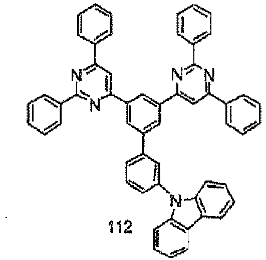

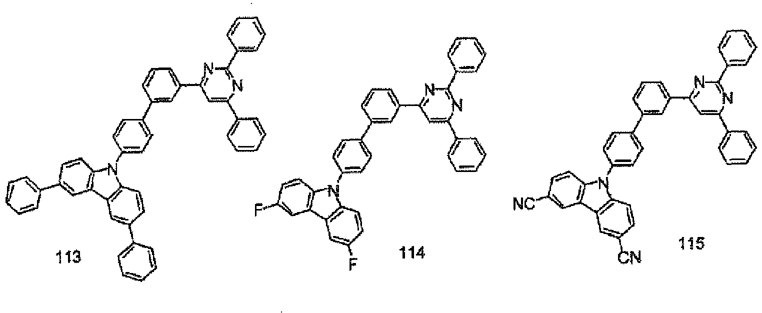

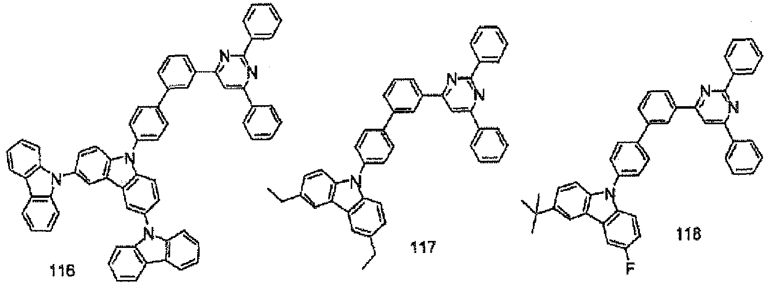

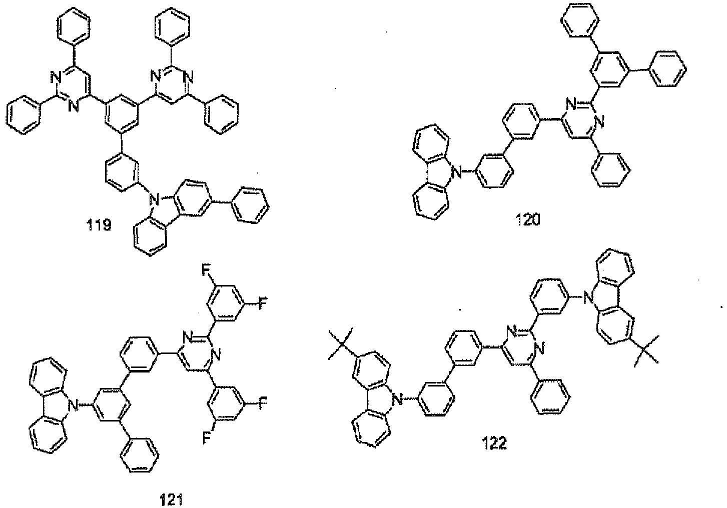

The compound exemplified as the compound represented by Formula (1) may be synthesized by various methods such as a method described in a pamphlet of International Publication No. WO03/080760, a method described in a pamphlet of International Publication No. WO03/078541, and a method described in a pamphlet of International Publication No. WO05/085387.

For example, the compound No. 4 may be synthesized by using m-bromobenzoaldehyde as a starting raw material by a method described in [0074] to [0075] of a pamphlet of International Publication No. WO05/085387 (page 45, line 11 to page 46, line 18). The compound No. 45 may be synthesized by using 3,5-dibromobenzoaldehyde as a starting raw material by a method described on page 46, line 9 to page 46, line 12 of a pamphlet of International Publication No. WO03/080760. The compound No. 77 may be synthesized by using N-phenylcarbazole as a starting raw material by a method described in on page 137, line 10 to page 139, line 9 of a pamphlet of International Publication No. WO05/022962.

In the present invention, the purpose of the compound represented by Formula (1) is not limited, and the compound may be contained in any layer of the organic layers. As a layer introducing the compound represented by Formula (1), in addition to the light emitting layer, it is preferable that the compound is contained in either any one or a plurality of a hole injection layer, a hole transporting layer, an electron transporting layer, an electron injection layer, an exciton blocking layer and a charge blocking layer.

In the invention, in order to further suppress a change in chromaticity when driving at a high temperature, the compound represented by Formula (1) is contained in the layer directly adjacent to the light emitting layer. Further, the compound represented by Formula (1) may be contained in both layers of the light emitting layer and the layer adjacent to the light emitting layer.

In the case where the compound represented by Formula (1) is contained in the light emitting layer, the compound represented by Formula (1) of the present invention is contained in an amount of preferably 0.1 to 99 mass%, more preferably 1 to 95 mass%, and more preferably 10 to 95 mass% on the basis of the total mass of the light emitting layer.

Further, in the case where the compound represented by Formula (1) is contained in a layer directly adjacent to the light emitting layer, the compound is contained in an amount of preferably 10 to 100 mass%, more preferably 30 to 100 mass%, and more preferably 50 to 100 mass% on the basis of the total mass of the layer other than the light emitting layer.

In the present invention, a host material may be used in combination with the compound represented by Formula (1). The host material in combination may be a hole transporting host material or an electron transporting host material, and the hole transporting host material can be used.

In the present invention, it is preferable that the light emitting layer contains the compound represented by Formula (1) and the host material. The host material is preferably a compound represented by the following Formula (4-1) or (4-2).

In the present invention, it is more preferable that the light emitting layer contains the compound represented by Formula (1) and further at least one of the compound represented by Formula (4-1) or (4-2).

In the present invention, when a compound represented by Formula (4-1) or (4-2) is contained in the light emitting layer, the compound represented by Formula (4-1) or (4-2) is contained in an amount of preferably 30 to 90 mass%, preferably 40 to 85 mass%, and particularly preferably 50 to 80 mass%, in the light emitting layer. In addition, when the compound represented by Formula (4-1) or (4-2) is used in a plurality of organic layers, it is preferable that the compound is contained in each layer in the above-mentioned range.

One kind of the compound represented by Formula (4-1) or (4-2) may be contained in any one organic layer, or a plurality of the compounds represented by Formula (4-1) or (4-2) may be contained in combination at any ratio.

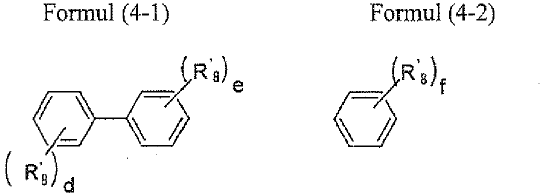

(In Formulas (4-1) and (4-2), each of d and e independently represents an integer of 0 to 3, and at least either one is 1 or more. f represents an integer of 1 to 4. R'₈ represents a substituent, and when d, e and f are 2 or more, R'₈ may be the same as or different from every other R'₈. Further, at least one of R'₈ represents a carbazole group represented by the following Formulas (5))

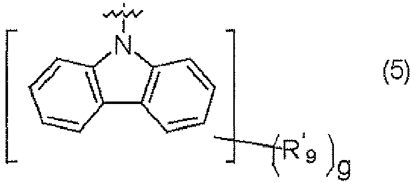

(In Formula (5), each of R'₉ independently represents a substituent. g represents an integer of 0 to 8)

Each of R'₈ independently represents a substituent, and specifically, is a halogen atom, an alkoxy group, a cyano group, a nitro group, an alkyl group, an aryl group, a heterocyclic group or a substituent represented by Formula (5). In the case where R'₈ does not represent Formula (5), R'₈ is preferably an alkyl group having 10 or less carbon atoms or a substituted or unsubstituted aryl group having 10 or less carbon atoms, and more preferably an alkyl group having 6 or less carbon atoms.

Each of R'₉ independently represents a substituent, and specifically, is a halogen atom, an alkoxy group, a cyano group, a nitro group, an alkyl group, an aryl group or a heterocyclic group, preferably an alkyl group having 10 or less carbon atoms and a substituted or unsubstituted aryl group having 10 or less carbon atoms, and more preferably an alkyl group having 6 or less carbon atoms.

g represents an integer of 0 to 8, and is preferably 0 to 4 from the viewpoint of not excessively shielding the carbazole structure transporting an electric charge. Further, from the viewpoint of easiness of synthesis, in the case where carbazole has a substituent, it is preferable that the carbazole has a substituent so as to be symmetrical with respect to the nitrogen atom.

In Formula (4-1), from the viewpoint of maintaining charge transportability, it is preferable that the sum of d and e is 2 or more. Further, it is preferable that R'₈ is meta-substituted with respect to the other benzene ring. The reason is deemed that, since a steric hindrance of the adjacent substituents is large in ortho-substitution, the bond is easily cleaved, and durability is decreased. In addition, in the para-substitution, since a molecular shape is close to substantially hard rod shape and crystallization is apt to occurs, deterioration of the device easily occurs under a high temperature condition. Specifically, the compound represented by the following structure is preferable. Moreover, R'₉ and g of the following compound are the same as R'₉ and g in Formula (5).

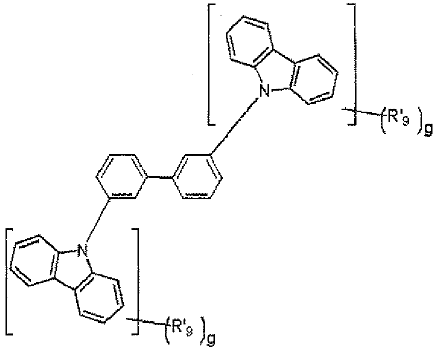

In Formula (4-2), it is preferable that f is 2 or more from the viewpoint of maintaining charge transportability. When f is 2 or 3, it is preferable that R'₈ is meta-substituted from the same viewpoint. In detail, the compound represented by the following structure is preferable. Further, R'₉ and g of the following compound are the same as R'₉ and g in Formula (5).

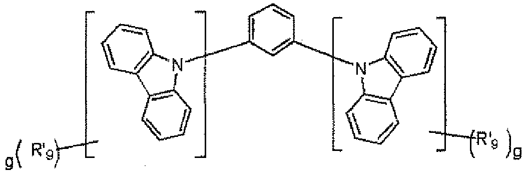

When Formulas (4-1) and (4-2) have a hydrogen atom, isotopes of hydrogen (deuterium atom etc.) are also included. In this case, all hydrogen atoms of the compound may be substituted with an isotope of hydrogen, and may be a mixture in which a portion of the compound contains an isotope of hydrogen. Preferably, R'₉ in Formula (5) is substituted with deuterium, and particularly preferably may have the following structure.

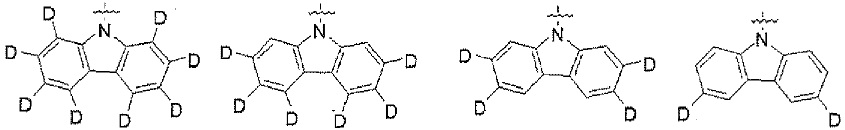

The atom further constituting a substituent includes the isotope thereof.

The compounds represented by Formula (4-1) and (4-2) may be synthesized by combining various known synthesis methods. Most generally, with respects to the carbazole compound, there may be a synthesis by dehydrogenation aromatization after Aza-Cope rearrangement reaction of arylhydrazine and a condensate with cyclohexane derivative (written by L. F. Tieze, and Th. Eicher, translated by Dakano, Ogasawara, Fine Organic Synthesis, p. 339 (published by Nankodo Co., Ltd.)). Further, with respects to the coupling reaction of the obtained carbazole compound and halogenated aryl compound using the palladium catalyst, there may be a method described in Tetrahedron Letters Vol. 39, p. 617 (1998), Vol. 39, p. 2367 (1998) and Vol. 40, p. 6393 (1999). The reaction temperature and reaction time are not particularly limited, and the condition described in the above document may be applied. In addition, several commercially available compounds such as mCP may be appropriately used.

In the present invention, it is preferable that the compounds represented by Formulas (4-1) and (4-2) form a thin layer by a vacuum deposition process, but a wet process such as solution coating may be appropriately used. The molecular weight of the compound is preferably 2,000 or less, more preferably 1,200 or less, and particularly preferably 800 or less from the viewpoint of deposition application or solubility. Further, from the viewpoint of deposition application, if the molecular weight is too small, since a vapor pressure is decreased and a change from a gas phase to a solid phase does not occur, it is difficult to form the organic layer, and therefore, the molecular weight is preferably 250 or more, and particularly preferably 300 or more.

It is preferable that Formulas {4-1} and (4-2) is the following structure or a compound in which the hydrogen atom is substituted by one or more deuterium atoms. Further, among the following compounds, R'₈ and R'₉ are the same meaning as R'₈ and R'₉ in Formulas (4-1), (4-2) and Formula (5).

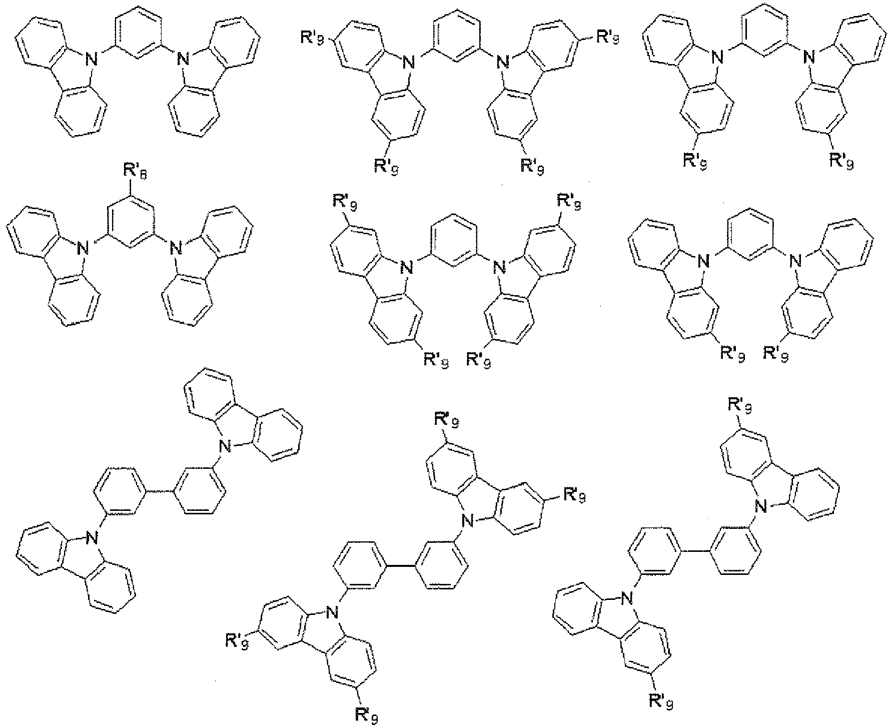

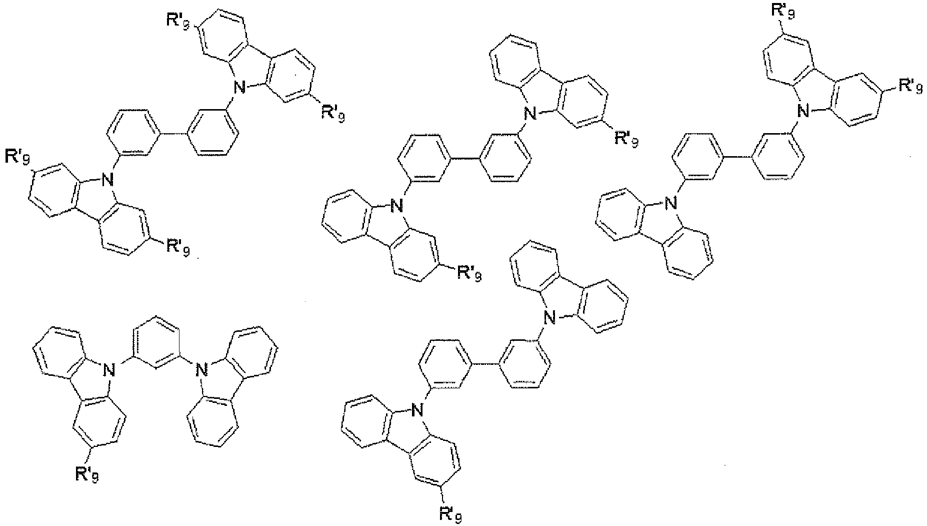

Hereinafter, particular examples of the compounds represented by Formula (4-1) and (4-2) in the present invention are described, but the present invention is not limited thereto.

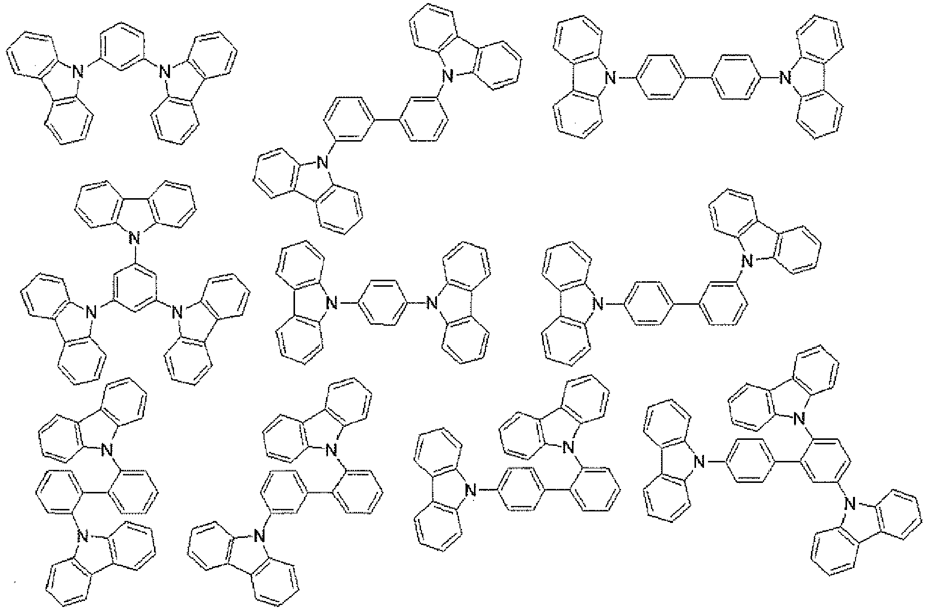

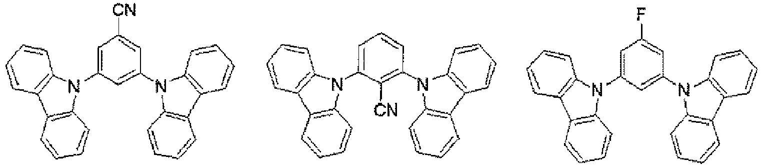

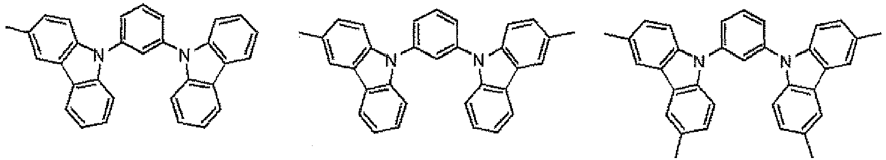

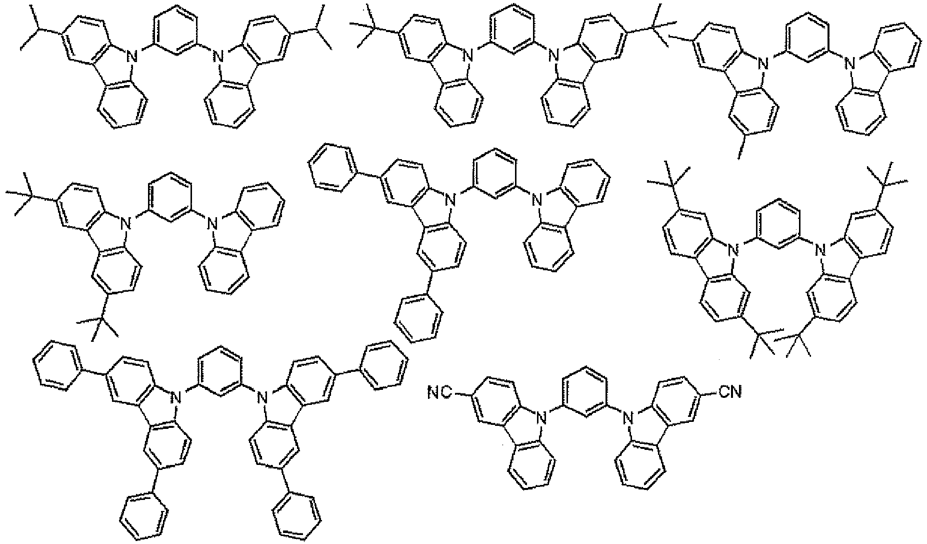

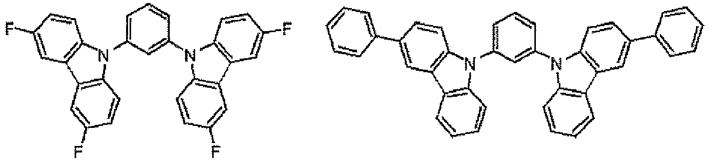

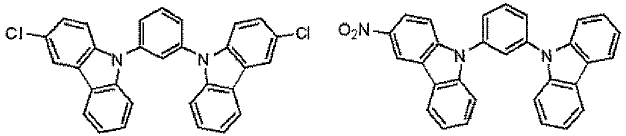

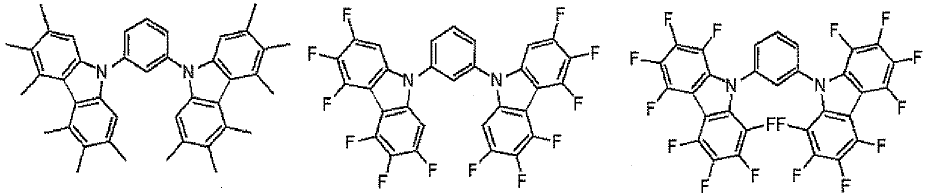

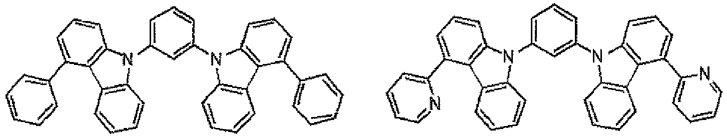

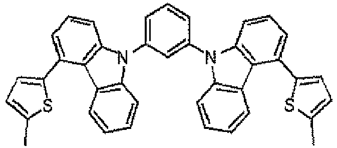

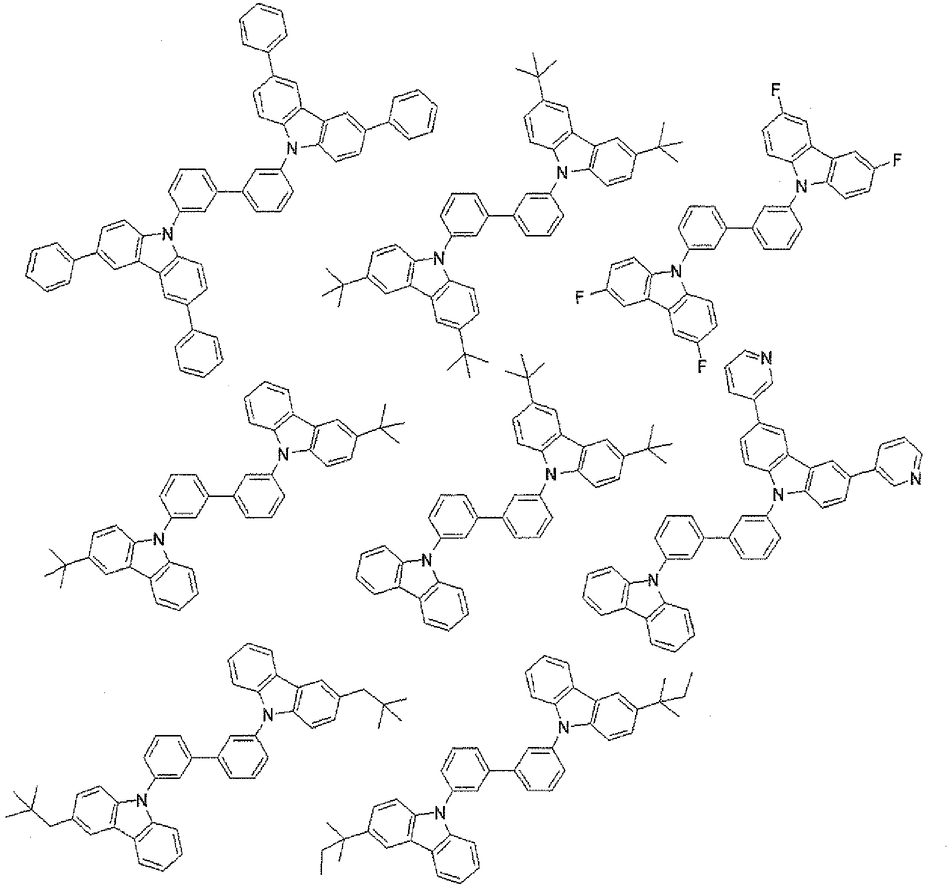

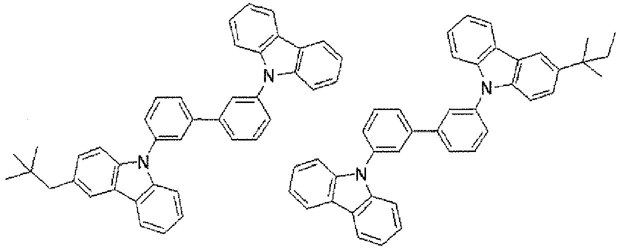

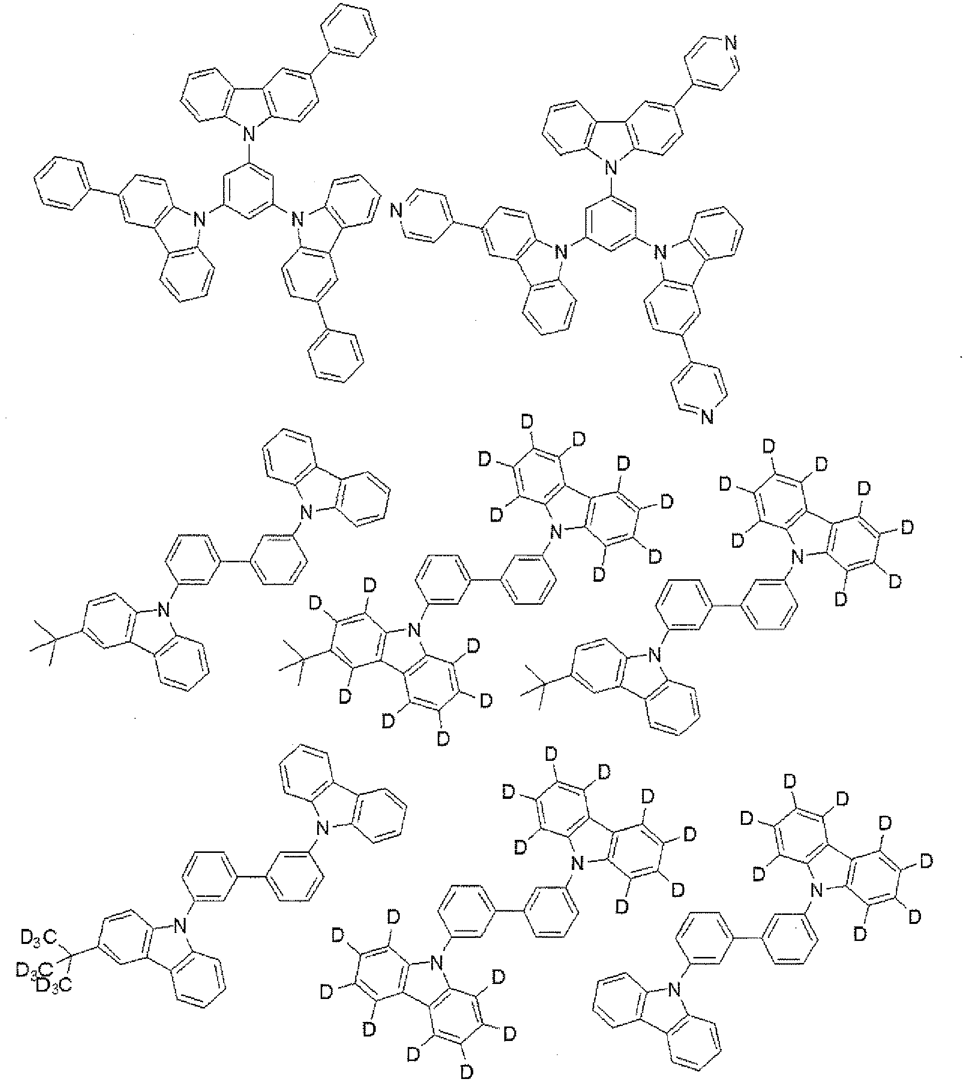

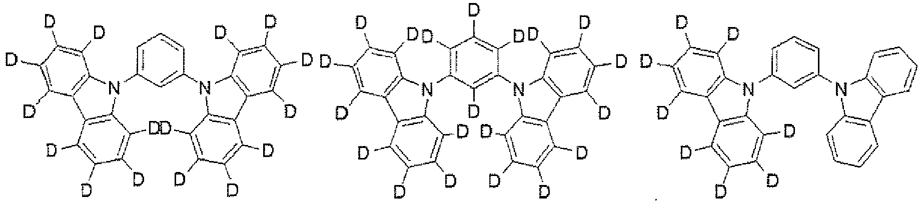

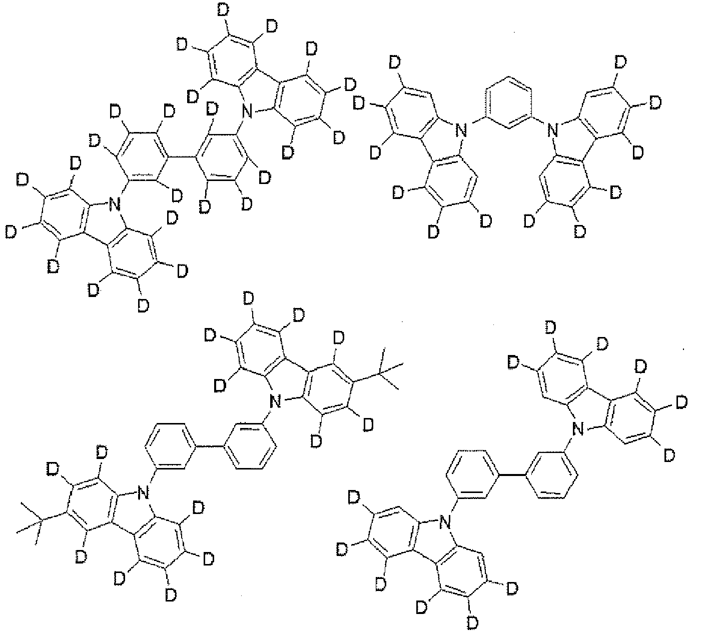

Next, a platinum complex represented by Formula (C-1) of a phosphorescence emitting material will be described.

First, in the present invention, the groups A and B of substituents will be defined as follows.

### (Group A of Substituents)

Examples may include an alkyl group (preferably the number of carbon atoms is 1 to 30, more preferably the number of carbon atoms is 1 to 20, and particularly preferably the number of carbon atoms is 1 to 10, and examples may include, for example, methyl, ethyl, isopropyl, tert-butyl, n-octyl, n-decyl, n-hexadecyl, cyclopropyl, cyclopentyl and cyclohexyl), an alkenyl group (preferably the number of carbon atoms is 2 to 30, more preferably the number of carbon atoms is 2 to 20, and particularly preferably the number of carbon atoms is 2 to 10, and examples may include, for example, vinyl, allyl, 2-buthenyl and 3-pentenyl), an alkynyl group (preferably the number of carbon atoms is 2 to 30, more preferably the number of carbon atoms is 2 to 20, and particularly preferably the number of carbon atoms is 2 to 10, and examples may include, for example, propargyl and 3-pentynyl), an aryl group(preferably the number of carbon atoms is 6 to 30, more preferably the number of carbon atoms is 6 to 20, and particularly preferably the number of carbon atoms is 6 to 12, and examples may include, for example, phenyl, p-methylphenyl, naphthyl and anthryl), an amino group (preferably the number of carbon atoms is 0 to 30, more preferably the number of carbon atoms is 0 to 20, and particularly preferably the number of carbon atoms is 0 to 10, and examples may include, for example, amino, methylamino, dimethylamino, diethylamino, dibenzylamino, diphenylamino and ditolylamino), an alkoxy group (preferably the number of carbon atoms is 1 to 30, more preferably the number of carbon atoms is 1 to 20, and particularly preferably the number of carbon atoms is 1 to 10, and examples may include, for example, methoxy, ethoxy, butoxy and 2-ethylhexyloxy), an aryloxy group (preferably the number of carbon atoms is 6 to 30, more preferably the number of carbon atoms is 6 to 20, and particularly preferably the number of carbon atoms is 6 to 12, and examples may include, for example, phenyloxy, 1-naphthyloxy and 2-naphthyloxy), a heterocyclic oxy group (preferably the number of carbon atoms is 1 to 30, more preferably the number of carbon atoms is 1 to 20, and particularly preferably the number of carbon atoms is 1 to 12, and examples may include, for example, pyridyloxy, pyrazyloxy, pyrimidyloxy and quinolyloxy), an acyl group (preferably the number of carbon atoms is 2 to 30, more preferably the number of carbon atoms is 2 to 20, and particularly preferably the number of carbon atoms is 2 to 12, and examples may include, for example, acetyl, benzoyl, formyl and pyvaloyl), an alkoxycarbonyl group (preferably the number of carbon atoms is 2 to 30, more preferably the number of carbon atoms is 2 to 20, and particularly preferably the number of carbon atoms is 2 to 12, and examples may include, for example, methoxycarbonyl and ethoxycarbonyl), an aryloxycarbonyl group (preferably the number of carbon atoms is 7 to 30, more preferably the number of carbon atoms is 7 to 20, and particularly preferably the number of carbon atoms is 7 to 12, and examples may include, for example, phenyloxycarbonyl), an acyloxy group (preferably the number of carbon atoms is 2 to 30, more preferably the number of carbon atoms is 2 to 20, and particularly preferably the number of carbon atoms is 2 to 10, and examples may include, for example, acetoxy and benzoyloxy), an acylamino group (preferably the number of carbon atoms is 2 to 30, more preferably the number of carbon atoms is 2 to 20, and particularly preferably the number of carbon atoms is 2 to 10, and examples may include, for example, acetylamino and benzoylamino), an alkoxycarbonylamino group (preferably the number of carbon atoms is 2 to 30, more preferably the number of carbon atoms is 2 to 20, and particularly preferably the number of carbon atoms is 2 to 12, and examples may include, for example, methoxycarbonylamino), an aryloxycarbonylamino group (preferably the number of carbon atoms is 7 to 30, more preferably the number of carbon atoms is 7 to 20, and particularly preferably the number of carbon atoms is 7 to 12, and examples may include, for example, phenyloxycarbonylamino), a sulfonylamino group (preferably the number of carbon atoms is 1 to 30, more preferably the number of carbon atoms is 1 to 20, and particularly preferably the number of carbon atoms is 1 to 12, and examples may include, for example, methanesulfonylamino and benzenesulfonylamino), a sulfamoyl group (preferably the number of carbon atoms is 0 to 30, more preferably the number of carbon atoms is 0 to 20, and particularly preferably the number of carbon atoms is 0 to 12, and examples may include, for example, sulfamoyl, methylsulfamoyl, dimethylsulfamoyl and phenylsulfamoyl), a carbamoyl group (preferably the number of carbon atoms is 1 to 30, more preferably the number of carbon atoms is 1 to 20, and particularly preferably the number of carbon atoms is 1 to 12, and examples may include, for example, carbamoyl, methylcarbamoyl, diethylcarbamoyl and phenylcarbamoyl), an alkylthio group (preferably the number of carbon atoms is 1 to 30, more preferably the number of carbon atoms is 1 to 20, and particularly preferably the number of carbon atoms is 1 to 12, and examples may include, for example, methylthio and ethylthio), an arylthio group (preferably the number of carbon atoms is 6 to 30, more preferably the number of carbon atoms is 6 to 20, and particularly preferably the number of carbon atoms is 6 to 12, and examples may include, for example phenylthio), a heterocyclic thio group (preferably the number of carbon atoms is 1 to 30, more preferably the number of carbon atoms is 1 to 20, and particularly preferably the number of carbon atoms is 1 to 12, and exmaples may include, for example, pyridylthio, 2-benzimidazolylthio, 2-benzoxazolylthio and 2-benzthiazoleylthio), sulfonyl group (preferably the number of carbon atoms is 1 to 30, more preferably the number of carbon atoms is 1 to 20, and particularly preferably the number of carbon atoms is 1 to 12, and exmaples may include, for example, mesyl and tosyl), a sulfinyl group (preferably the number of carbon atoms is 1 to 30, more preferably the number of carbon atoms is 1 to 20, and particularly preferably the number of carbon atoms is 1 to 12, and exmaples may include, for example, methanesulfinyl and benzenesulfinyl), a ureido group (preferably the number of carbon atoms is 1 to 30, more preferably the number of carbon atoms is 1 to 20, and particularly preferably the number of carbon atoms is 1 to 12, and exmaples may include, for example, ureido, methylureido and phenylureido), a phosphoric acid amide group (preferably the number of carbon atoms is 1 to 30, more preferably the number of carbon atoms is 1 to 20, and particularly preferably the number of carbon atoms is 1 to 12, and exmaples may include, for example, diethylphosphoric acid amide and phenylphosphoric acid amide), a hydroxy group, a mercapto group, a halogen atom (exmaples may include, for example, a fluorine atom, a chlorine atom, a bromine atom and an iodine atom), a cyano group, a sulfo group, a carboxyl group, a nitro group, a hydroxamic acid group, a sulfino group, a hydrazino group, an imino group, a heterocyclic group (aromatic heterocyclic group is included, preferably the number of carbon atoms is 1 to 30, and more preferably the number of carbon atoms is 1 to 12, and examples of the heteroatom may include, for example, a nitrogen atom, an oxygen atom, a sulfur atom, a phosphorus atom, a silicon atom, a selenium atom and a tellurium atom, and specifically, exmaples may include pyridyl, pyrazynyl, pyrimidyl, pyridazynyl, pyrrolyl, pyrazolyl, triazolyl, imidazolyl, oxazolyl, thiazoleyl, isoxazolyl, isothiazoleyl, quinolyl, furyl, thienyl, selenophenyl, tellurophenyl, piperidyl, piperidino, morpholino, pyridyl, pyrrolidino, benzoxazoleyl, benzoimidazolyl, benzothiazoleyl, carbazolyl group, azepinyl group and silolyl group), a silyl group (preferably the number of carbon atoms is 3 to 40, more preferably the number of carbon atoms is 3 to 30, and particularly preferably the number of carbon atoms is 3 to 24, and exmaples may include, for example, trimethylsilyl and triphenylsilyl), a silyloxy group (preferably the number of carbon atoms is 3 to 40, more preferably the number of carbon atoms is 3 to 30, and particularly preferably the number of carbon atoms is 3 to 24, and exmaples may include, for example, trimethylsilyloxy and triphenylsilyloxy), and a phosphoryl group (exmaples may include, for example, diphenylphosphoryl group and dimethylphosphoryl group). These substituents may be further substituted, and as the further substituents, a group selected from the aforementioned group A of substituents can be exemplified.

### (Group B of Substituents)

Examples may include an alkyl group (preferably the number of carbon atoms is 1 to 30, more preferably the number of carbon atoms is 1 to 20, and particularly preferably the number of carbon atoms is 1 to 10, and exmaples may include, for example, methyl, ethyl, isopropyl, tert-butyl, n-octyl, n-decyl, n-hexadecyl, cyclopropyl, cyclopentyl and cyclohexyl), an alkenyl group (preferably the number of carbon atoms is 2 to 30, more preferably the number of carbon atoms is 2 to 20, and particularly preferably the number of carbon atoms is 2 to 10, and exmaples may include, for example, vinyl, allyl, 2-buthenyl and 3-pentenyl), an alkynyl group (preferably the number of carbon atoms is 2 to 30, more preferably the number of carbon atoms is 2 to 20, and particularly preferably the number of carbon atoms is 2 to 10, and exmaples may include, for example propargyl and 3-pentynyl), an aryl group (preferably the number of carbon atoms is 6 to 30, more preferably the number of carbon atoms is 6 to 20, and particularly preferably the number of carbon atoms is 6 to 12, and exmaples may include, for example, phenyl, p-methylphenyl, naphthyl, and anthryl), a cyano group, and a heterocyclic group (aromatic heterocyclic group is included, preferably the number of carbon atoms is 1 to 30, and more preferably the number of carbon atoms is 1 to 12, and examples of the heteroatom may include, for example, a nitrogen atom, an oxygen atom, a sulfur atom, a phosphorus atom, a silicon atom, a selenium atom and a tellurium atom, and specifically, exmaples may include pyridyl, pyrazynyl, pyrimidyl, pyridazynyl, pyrrolyl, pyrazolyl, triazolyl, imidazolyl, oxazolyl, thiazoleyl, isoxazolyl, isothiazoleyl, quinolyl, furyl, thienyl, selenophenyl, tellurophenyl, piperidyl, piperidino, morpholino, pyrrolidyl, pyrrolidino, benzoxazoleyl, benzoimidazolyl, benzothiazoleyl, carbazolyl group, azepinyl group and silolyl group). These substituents may be further substituted, and as the further substituents, a group selected from the aforementioned groups A and B of substituents can be exemplified.

In the present invention, "the number of carbon atoms" of a substituent, such as the alkyl group, is used as a meaning to include the case where the substituent such as the alkyl group may be substituted with an additional substituent, and to include the number of carbon atoms of the additional substituent as well.

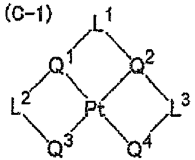

(In the formula, each of Q¹, Q², Q³ and Q⁴ independently represents a ligand which is coordinated to Pt and whereby Q3 and Q4 are not linked. Each of L¹, L² and L³ independently represents a single bond or a divalent linking group).

Formula (C-1) will be described. Each of Q¹, Q², Q³ and Q⁴ independently represents a ligand which is coordinated to Pt. Here, the bond of Q¹, Q², Q³ and Q⁴ to Pt may be any of a covalent bond, an ionic bond and a coordination bond. As an atom bound to Pt in each of Q¹, Q², Q³ and Q⁴, a carbon atom, a nitrogen atom, an oxygen atom, a sulfur atom and a phosphorus atom are preferable. Among the atoms bound to Pt in Q¹, Q², Q³ and Q⁴, it is preferable that at least one of the atoms is a carbon atom; it is more preferable that two of the atoms are a carbon atom; and it is particularly preferable that two of the atoms are a carbon atom and the other two are a nitrogen atom.

Q¹, Q², Q³ and Q⁴ bound to Pt with its carbon atom, may be an anionic ligand or a neutral ligand. Examples of the anionic ligand include a vinyl ligand, an aromatic hydrocarbon ring ligand (for example, a benzene ligand, a naphthalene ligand, an anthracene ligand, a phenanthrene ligand and the like), a heterocyclic ligand (for example, a furan ligand, a thiophene ligand, a pyridine ligand, a pyrazine ligand, a pyrimidine ligand, a pyridazine ligand, a triazine ligand, a thiazole ligand, an oxazole ligand, a pyrrole ligand, an imidazole ligand, a pyrazole ligand, a triazole ligand, and a condensed ring including the same (for example, a quinoline ligand, a benzothiazole ligand and the like)). Examples of the neutral ligand include a carbene ligand.

Q¹, Q², Q³ and Q⁴ bound to Pt with its nitrogen atom, may be a neutral ligand or an anionic ligand. Examples of the neutral ligand include a nitrogen-containing aromatic heterocyclic ligand (a pyridine ligand, a pyrazine ligand, a pyrimidine ligand, a pyridazine ligand, a triazine ligand, an imidazole ligand, a pyrazole ligand, a triazole ligand, an oxazole ligand, a thiazole ligand, and a condensed ring including the same (for example, a quinoline ligand, a benzoimidazole ligand and the like)), an amine ligand, a nitrile ligand and an imine ligand. Examples of the anionic ligand include an amino ligand, an imino ligand, a nitrogen-containing aromatic heterocyclic ligand (a pyrrole ligand, an imidazole ligand, a triazole ligand, and a condensed ring including the same (for example, an indole ligand, a benzoimidazole ligand and the like)).

Q¹, Q², Q³ and Q⁴ bound to Pt with its oxygen atom, may be a neutral ligand or an anionic ligand. Examples of the neutral ligand include an ether ligand, a ketone ligand, an ester ligand, an amide ligand and an oxygen-containing heterocyclic ligand (a furan ligand, an oxazole ligand, and a condensed ring including the same (a benzoxazole ligand and the like)). Examples of the anionic ligand include an alkoxy ligand, an aryloxy ligand, a heteroaryloxy ligand, an acyloxy ligand, a silyloxy ligand and the like.

Q¹, Q², Q³ and Q⁴ bound to Pt with its sulfur atom, may be a neutral ligand or an anionic ligand. Examples of the neutral ligand include a thioether ligand, a thioketone ligand, a thioester ligand, a thioamide ligand, a sulfur-containing heterocyclic ligand (a thiophene ligand, a thiazole ligand, and a condensed ring bodies (a benzothiazole ligand and the like)). Examples of the anionic ligand include an alkyl mercapto ligand, an aryl mercapto ligand, a heteroaryl mercapto ligand and the like.

Q¹, Q², Q³ and Q⁴ bound to Pt with its phosphorus atom, may be a neutral ligand or an anionic ligand. Examples of the neutral ligand include a phosphine ligand, a phosphoric ester ligand, a phosphorous ester ligand and a phosphorus-containing heterocyclic ligand (a phosphinine ligand and the like). Examples of the anionic ligand include a phosphino ligand, a phosphinyl ligand, a phosphoryl ligand and the like.

The group represented by Q¹, Q², Q³ and Q⁴ may be substituted. As a substituent, those exemplified above for the group A of substituents may be appropriately applied. Also, the substituents may be linked to each other (with the condition that Q³ and Q⁴ are not linked to each other).

The group represented by Q¹, Q², Q³ and Q⁴ is preferably an aromatic hydrocarbon ring ligand bound to Pt with its carbon atom, an aromatic heterocyclic ligand bound to Pt with its carbon atom, a nitrogen-containing aromatic heterocyclic ligand bound to Pt with its nitrogen atom, an acyloxy ligand, an alkyloxy ligand, an aryloxy ligand, a heteroaryloxy ligand and a silyloxy ligand, more preferably an aromatic hydrocarbon ring ligand bound to Pt with its carbon atom, an aromatic heterocyclic ligand bound to Pt with its carbon atom, a nitrogen-containing aromatic heterocyclic ligand bound to Pt with its nitrogen atom, an acyloxy ligand and an aryloxy ligand, and even more preferably an aromatic hydrocarbon ring ligand bound to Pt with its carbon atom, an aromatic heterocyclic ligand bound to Pt with its carbon atom, a nitrogen-containing aromatic heterocyclic ligand bound to Pt with its nitrogen atom and an acyloxy ligand.

L¹, L² and L³ represent a single bond or a divalent linking group. Examples of the divalent linking group represented by L¹, L² and L³ include an alkylene group (methylene, ethylene, propylene and the like), an arylene group (phenylene and naphthalenediyl), a heteroarylene group (pyridinediyl, thiophenediyl and the like), an imino group (-NR_{L}-) (a phenylimino group and the like), an oxy group (-O-), a thio group (-S-), a phosphinidene group (-PR_{L}-) (a phenylphosphinidene group and the like), a silylene group (-SiR_{L}R_{L}'-) (a dimethylsilylene group, a diphenylsilylene group and the like), or a combination thereof. Here, each of R_{L} and R_{L}' independently represents an alkyl group or an aryl group. These linking groups may further have a substituent.

From the viewpoints of stability and light emission quantum yield of the complex, each of L¹, L² and L³ is preferably a single bond, an alkylene group, an arylene group, a heteroarylene group, an imino group, an oxy group, a thio group and a silylene group, more preferably a single bond, an alkylene group, an arylene group and an imino group, further preferably a single bond, an alkylene group and an arylene group, even further preferably a single bond, a methylene group and a phenylene group, even more preferably a single bond, and a di-substituted methylene group, even still further preferably a single bond, a dimethylmethylene group, a diethylmethylene group, a diisobutylmethylene group, a dibenzylmethylene group, an ethylmethylmethylene group, a methylpropylmethylene group, an isobutylmethylmethylene group, a diphenylmethylene group, a methylphenylmethylene group, a cyclohexanediyl group, a cyclopentanediyl group, a fluorenediyl group and a fluoromethylmethylene group.

L¹ is particularly preferably a single bond, a dimethylmethylene group, a diphenylmethylene group, a cyclohexanediyl group and most preferably a dimethylmethylene group. L² and L³ are most preferably a single bond.

The platinum complex represented by Formula (C-1) is more preferably a platinum complex represented by the following Formula (C-2).

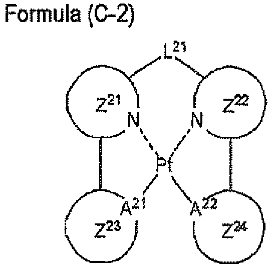

(In the formula, L²¹ represents a single bond or a divalent linking group. Each of A²¹ and A²² independently represents a carbon atom or a nitrogen atom. Each of Z²¹ and Z²² independently represents a nitrogen-containing aromatic heterocyclic ring. Each of Z²³ and Z²⁴ independently represents a benzene ring or an aromatic heterocyclic ring)

Formula (C-2) will be described. L²¹ has the same meaning as L¹ in Formula (C-1), and preferred ranges thereof are also the same.

Each of A²¹ and A²² independently represents a carbon atom or a nitrogen atom. It is preferable that at least one of A²¹ and A²² is a carbon atom, and it is preferable that both of A²¹ and A²² are a carbon atom from the viewpoint of stability of the complex and light emission quantum yield of the complex.

Each of Z²¹ and Z²² independently represents a nitrogen-containing aromatic heterocyclic ring. Examples of the nitrogen-containing aromatic heterocyclic ring represented by Z²¹ and Z²² include a pyridine ring, a pyrimidine ring, a pyrazine ring, a triazine ring, an imidazole ring, a pyrazole ring, an oxazole ring, a thiazole ring, a triazole ring, an oxadiazole ring, a thiadiazole ring and the like. From the viewpoints of stability, control of light emission wavelength, and light emission quantum yield of the complex, the ring represented by Z²¹ and Z²² is preferably a pyridine ring, a pyrazine ring, an imidazole ring and a pyrazole ring, more preferably a pyridine ring, an imidazole ring and a pyrazole ring, even more preferably a pyridine ring and a pyrazole ring, and particularly preferably a pyridine ring.

The nitrogen-containing aromatic heterocyclic ring represented by Z²¹ and Z²² may be substituted. As a substituent on the carbon atom, the group A of substituents may be applied, and as a substituent on the nitrogen atom, the group B of substituents may be applied. The substituent on the carbon atom is preferably an alkyl group, a perfluoroalkyl group, an aryl group, an aromatic heterocyclic group, a dialkylamino group, a diarylamino group, an alkoxy group, a cyano group and a halogen atom. Although the substituent is appropriately selected for the purpose of controlling the light emission wavelength or potential, in the case of shortening the wavelength, the substituent is preferably an electron donating group, a fluorine atom and an aromatic ring group, and for example, an alkyl group, a dialkylamino group, an alkoxy group, a fluorine atom, an aryl group, an aromatic heterocyclic group and the like are selected. Also, in the case of lengthening the wavelength, the substituent is preferably an electron-withdrawing group, and for example, a cyano group, a perfluoroalkyl group and the like are selected. The substituent on the nitrogen atom is preferably an alkyl group, an aryl group and an aromatic heterocyclic group, and from the viewpoint of stability of the complex, an alkyl group and an aryl group are preferable. The substituents may be linked to each other to form a condensed ring, and examples of the ring to be formed include a benzene ring, a pyridine ring, a pyrazine ring, a pyridazine ring, a pyrimidine ring, an imidazole ring, an oxazole ring, a thiazole ring, a pyrazole ring, a thiophene ring, a furan ring and the like.

Each of Z²³ and Z²⁴ independently represents a benzene ring or an aromatic heterocyclic ring. Examples of the nitrogen-containing aromatic heterocyclic ring represented by Z²³ and Z²⁴ include a pyridine ring, a pyrimidine ring, a pyrazine ring, a pyridazine ring, a triazine ring, an imidazole ring, a pyrazole ring, an oxazole ring, a thiazole ring, a triazole ring, an oxadiazole ring, a thiadiazole ring, a thiophene ring, a furan ring and the like. From the viewpoints of stability, control of light emission wavelength, and light emission quantum yield of the complex, the ring represented by Z²³ and Z²⁴ is preferably a benzene ring, a pyridine ring, a pyrazine ring, an imidazole ring, a pyrazole ring and a thiophene ring, more preferably a benzne ring, a pyridine ring and a pyrazole ring, and even more preferably a benzene ring and a pyridine ring.

The benzene ring and nitrogen-containing aromatic heterocyclic ring represented by Z²³ and Z²⁴ may be substituted. As a substituent on the carbon atom, the group A of substituents may be applied, and as a substituent on the nitrogen atom, the group B of substituents may be applied. The substituent on the carbon atom is preferably an alkyl group, a perfluoroalkyl group, an aryl group, an aromatic heterocyclic group, a dialkylamino group, a diarylamino group, an alkoxy group, a cyano group and a halogen atom. Although the substituent is appropriately selected for the purpose of controlling the light emission wavelength or potential, in the case of lengthen the wavelength, the substituent is preferably an electron donating group and an aromatic ring group, and for example, an alkyl group, a dialkylamino group, an alkoxy group, an aryl group, an aromatic heterocyclic group and the like are selected. Also, in the case of shortening the wavelength, the substituent is preferably an electron-withdrawing group, and for example, a fluorine group, a cyano group, a perfluoroalkyl group and the like are selected. The substituent on the nitrogen atom is preferably an alkyl group, an aryl group and an aromatic heterocyclic group, and from the viewpoint of stability of the complex, an alkyl group and an aryl group are preferable. The substituents may be linked to each other to form a condensed ring, and examples of the ring to be formed include a benzene ring, a pyridine ring, a pyrazine ring, a pyridazine ring, a pyrimidine ring, an imidazole ring, an oxazole ring, a thiazole ring, a pyrazole ring, a thiophene ring, a furan ring and the like.

Among platinum complexes represented by Formula (C-2), a more preferable aspect is a platinum complex represented by the following Formula (C-3).

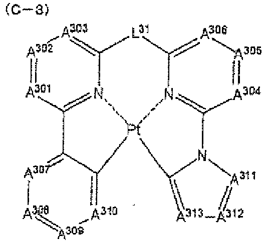

(In the formula, each of A³⁰¹ to A³¹³ independently represents C-R or a nitrogen atom. R represents a hydrogen atom or a substituent. L³¹ represents a single bond or a divalent linking group)

Formula (C-3) will be described. L³¹ has the same meaning as L²¹ in Formula (C-2), and preferred ranges thereof are also the same. Each of A³⁰¹ to A³⁰⁶ independently represents C-R or a nitrogen atom. R represents a hydrogen atom or a substituent. As a substituent represented by R, those exemplified above for the group A of substituents may be applied.

A³⁰¹ to A³⁰⁶ are preferably C-R, and R groups may be linked to each other to form a ring. When A³⁰¹ to A³⁰⁶ are C-R, the R groups of A³⁰² and A³⁰⁵ are preferably a hydrogen atom, an alkyl group, an aryl group, an amino group, an alkoxy group, an aryloxy group, a fluorine group, and a cyano group, more preferably a hydrogen atom, an amino group, an alkoxy group, an aryloxy group and a fluorine group, and particularly preferably a hydrogen atom and a fluorine group. The R groups of A³⁰¹, A³⁰³, A³⁰⁴ and A³⁰⁶ are preferably a hydrogen atom, an alkyl group, an aryl group, an amino group, an alkoxy group, an aryloxy group, a fluorine group and a cyano group, more preferably a hydrogen atom, an amino group, an alkoxy group, an aryloxy group and a fluorine group, and particularly preferably a hydrogen atom. Each of A³⁰⁷, A³⁰⁸, A³⁰⁹ and A³¹⁰ independently represents C-R or a nitrogen atom. R represents a hydrogen atom or a substituent. As a substituent represented by R, those exemplified above for the group A of substituents may be applied. When A³⁰⁷, A³⁰⁸, A³⁰⁹ and A³¹⁰ are C-R, R is preferably a hydrogen atom, an alkyl group, a perfluoroalkyl group, an aryl group, an aromatic heterocyclic group, a dialkylamino group, a diarylamino group, an alkyloxy group, a cyano group and a halogen atom, more preferably a hydrogen atom, an alkyl group, a perfluoroalkyl group, an aryl group, a dialkylamino group, a cyano group and a fluorine group, and even more preferably a hydrogen atom, an alkyl group, a trifluoromethyl group and a fluorine atom. Also, if possible, the substituents may be linked to each other to form a condensed ring structure. When the light emission wavelength is shifted to the short wavelength side, A³⁰⁸ is preferably a nitrogen atom.

When A³⁰⁷ to A³¹⁰ are selected as described above, examples of a six-membered ring to be formed by two carbon atoms and A³⁰⁷, A³⁰⁸, A³⁰⁹ and A³¹⁰ include a benzene ring, a pyridine ring, a pyrazine ring, a pyrimidine ring, a pyridazine ring and a triazine ring, more preferably a benzene ring, a pyridine ring, a pyrazine ring, a pyrimidine ring and a pyridazine ring, and particularly preferably a benzene ring and a pyridine ring. Since the six-membered ring is a pyridine ring, a pyrazine ring, a pyrimidine ring and a pyridazine ring (particularly preferably a pyridine ring), it is advantageous in that the acidity of a hydrogen atom existing at a position where a metal-carbon bond is formed is enhanced as compared to the case of a benzene ring, and thus, a metal complex is more easily formed.

Each of A³¹¹, A³¹² and A³¹³ independently represents C-R or a nitrogen atom. R represents a hydrogen atom or a substituent. As a substituent represented by R, those exemplified above for the group A of substituents may be applied. When A³¹¹, A³¹² and A³¹³ are C-R, R is preferably a hydrogen atom, an alkyl group, a perfluoroalkyl group, an aryl group, an aromatic heterocyclic group, a dialkylamino group, a diarylamino group, an alkyloxy group, a cyano group and a halogen atom, more preferably a hydrogen atom, an alkyl group, a perfluoroalkyl group, an aryl group, a dialkylamino group, a cyano group and a fluorine group, and even more preferably a hydrogen atom, an alkyl group, a trifluoromethyl group and a fluorine atom. Also, if possible, the substituents may be linked to each other to form a condensed ring structure. At least one of A³¹¹, A³¹² and A³¹³ is preferably a nitrogen atom, and A311 is particularly preferably a nitrogen atom.

Among platinum complexes represented by Formula (C-2), a more preferable aspect is a platinum complex represented by the following Formula (C-4).

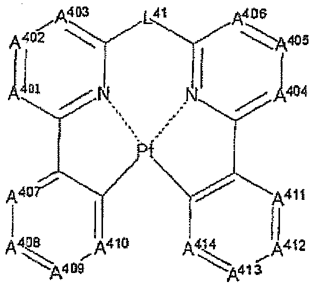

(In Formula (C-4), each of A⁴⁰¹ to A⁴¹⁴ independently represents C-R or a nitrogen atom. R represents a hydrogen atom or a substituent. L⁴¹ represents a single bond or a divalent linking group)

Formula (C-4) will be described.

Each of A⁴⁰¹ to A⁴¹⁴ independently represents C-R or a nitrogen atom. R represents a hydrogen atom or a substituent. A⁴⁰¹ to A⁴⁰⁶ and L⁴¹ have the same meaning as A³⁰¹ to A³⁰⁶ and L³¹ in Formula (C-3), and preferred ranges thereof are also the same.

For A⁴⁰⁷ to A⁴¹⁴, the number of nitrogen atoms in each of A⁴⁰⁷ to A⁴¹⁰ and A⁴¹¹ to A⁴¹⁴ is preferably 0 to 2, and more preferably 0 or 1. When the light emission wavelength is shifted to the short wavelength side, A⁴⁰⁸ or A⁴¹² is preferably a nitrogen atom, and both of A⁴⁰⁸ and A⁴¹² are more preferably a nitrogen atom.

When A⁴⁰⁷ to A⁴¹⁴ represent C-R, the Rs of A⁴⁰⁸ and A⁴¹² are preferably a hydrogen atom, an alkyl group, a perfluoroalkyl group, an aryl group, an amino group, an alkoxy group, an aryloxy group, a fluorine group and a cyano group, more preferably a hydrogen atom, a perfluoroalkyl group, an alkyl group, an aryl group, a fluorine group and a cyano group, and particularly preferably a hydrogen atom, a phenyl group, a perfluoroalkyl group and a cyano group. The R groups of A⁴⁰⁷, A⁴⁰⁹, A⁴¹¹ and A⁴¹³ are preferably a hydrogen atom, an alkyl group, a perfluoroalkyl group, an aryl group, an amino group, an alkoxy group, an aryloxy group, a fluorine group and a cyano group, more preferably a hydrogen atom, a perfluoroalkyl group, a fluorine group and a cyano group, and particularly preferably a hydrogen atom, a phenyl group and a fluorine group. The R groups of A⁴¹⁰ and A⁴¹⁴ are preferably a hydrogen atom and a fluorine group, and more preferably a hydrogen atom. When any one of A⁴⁰⁷ to A⁴⁰⁹ and A⁴¹¹ to A⁴¹³ represents C-R, R groups may be linked to each other to form a ring.

Among platinum complexes represented by Formula (C-2), a more preferable aspect is a platinum complex represented by the following Formula (C-5).

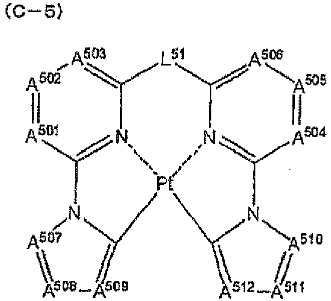

(In Formula (C-5), each of A⁵⁰¹ to A⁵¹² independently represents C-R or a nitrogen atom. R represents a hydrogen atom or a substituent. L⁵¹ represents a single bond or a divalent linking group)

Formula (C-5) will be described. A⁵⁰¹ to A⁵⁰⁶ and L⁵¹ have the same meaning as A³⁰¹ to A³⁰⁶ and L³¹ in Formula (C-3), and preferred ranges thereof are also the same.

Each of A⁵⁰⁷, A⁵⁰⁸ and A⁵⁰⁹, and A⁵¹⁰, A⁵¹¹ and A⁵¹² independently has the same meaning as A³¹¹, A³¹² and A³¹³ in Formula (C-3), and preferred ranges thereof are also the same.

Among platinum complexes represented by Formula (C-1), another more preferable aspect is a platinum complex represented by the following Formula (C-6).

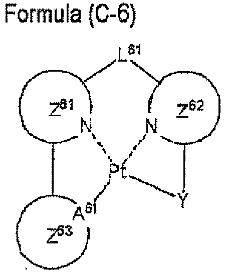

(In the formula, L⁶¹ represents a single bond or a divalent linking group. Each of A⁶¹ independently represents a carbon atom or a nitrogen atom. Each of Z⁶¹ and Z⁶² independently represents a nitrogen-containing aromatic heterocyclic ring. Each of Z⁶³ independently represents a benzene ring or an aromatic heterocyclic ring. Y is an anionic acyclic ligand bound to Pt)

Formula (C-6) will be described. L⁶¹ has the same meaning as L¹ in Formula (C-1), and preferred ranges thereof are also the same.

A⁶¹ represents a carbon atom or a nitrogen atom. From the viewpoint of stability of the complex and the viewpoint of light emission quantum yield of the complex, A⁶¹ is preferably a carbon atom.

Each of Z⁶¹ and Z⁶² has the same meaning as Z²¹ and Z²² in Formula (C-2), respectively, and preferred ranges thereof are also the same. L⁶³ has the same meaning as in Z²³ in Formula (C-2), and preferred ranges thereof are also the same.

Y is an anionic acyclic ligand bound to Pt. The acyclic ligand is one in which an atom bound to Pt does not form a ring in a ligand state. The atom bound to Pt in Y is preferably a carbon atom, a nitrogen atom, an oxygen atom and a sulfur atom, more preferably a nitrogen atom and an oxygen atom, and most preferably an oxygen atom. Examples of Y bound to Pt with a carbon atom include a vinyl ligand. Examples of Y bound to Pt with a nitrogen atom include an amino ligand and an imino ligand. Examples of Y bound to Pt with an oxygen atom include an alkoxy ligand, an aryloxy ligand, a heteroaryloxy ligand, an acyloxy ligand, a silyloxy ligand, a carboxyl ligands, a phosphate ligand, a sulfonate ligand and the like. Examples of Y bound to Pt with a sulfur atom include an alkyl mercapto ligand, an aryl mercapto ligand, a heteroaryl mercapto ligand, a thiocarboxylate ligand and the like.

The ligand represented by Y may be substituted. As a substituent, those exemplified above for the group A of substituents may be appropriately applied. Also, the substituents may be linked to each other.

The ligand represented by Y is preferably a ligand bound to Pt with an oxygen atom, more preferably an acyloxy ligand, an alkyloxy ligand, an aryloxy ligand, a heteroaryloxy ligand and a silyloxy ligand, and even more preferably an acyloxy ligand.

Among platinum complexes represented by Formula (C-6), a more preferable aspect is a platinum complex represented by the following Formula (C-7).

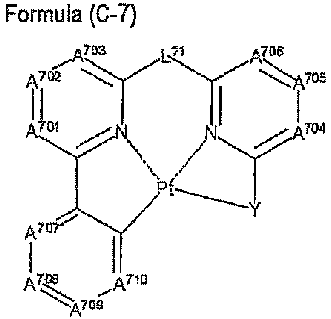

(In the formula, each of A⁷⁰¹ to A⁷¹⁰ independently represents C-R or a nitrogen atom. R represents a hydrogen atom or a substituent. L⁷¹ represents a single bond or a divalent linking group. Y is an anionic acyclic ligand bound to Pt)

Formula (C-7) will be described. L⁷¹ has the same meaning as L⁶¹ in Formula (C-6), and preferred ranges thereof are also the same. A⁷⁰¹ to A⁷¹⁰ have the same meaning as in A³⁰¹ to A³¹⁰ in Formula (C-3), and preferred ranges thereof are also the same. Y has the same meaning as that in Formula (C-6), and preferred ranges thereof are also the same.

Specific examples of the platinum complex represented by Formula (C-1) include compounds disclosed in [0143] to [0152], [0157] to [0158], and [0162] to [0168] of Japanese Patent Application Laid-Open No. 2005-310733, compounds disclosed in [0065] to [0083] of Japanese Patent Application Laid-Open No. 2006-256999, compounds disclosed in [0065] to [0090] of Japanese Patent Application Laid-Open No. 2006-93542, compounds disclosed in [0063] to [0071] of Japanese Patent Application Laid-Open No. 2007-73891, compounds disclosed in [0079] to [0083] of Japanese Patent Application Laid-Open No. 2007-324309, compounds disclosed in [0055] to [0071] of Japanese Patent Application Laid-Open No. 2007-96255, and [0043] to [0046] of Japanese Patent Application Laid-Open No. 2006-313796, and other platinum complexes exemplified below. The complexes 6-2, 6-3, 7-1, 7-2, 7-3, 7-4 and 7-5 do not form part of the invention.

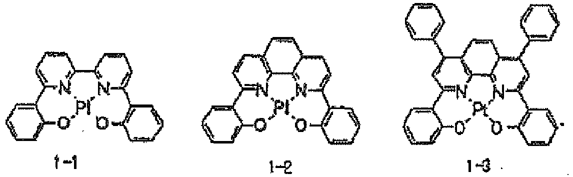

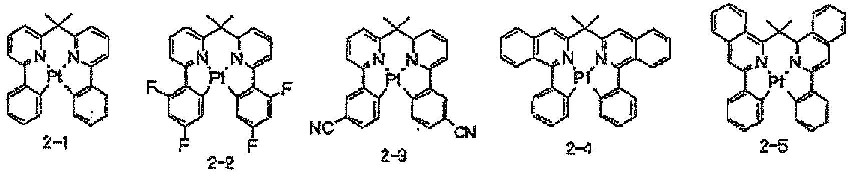

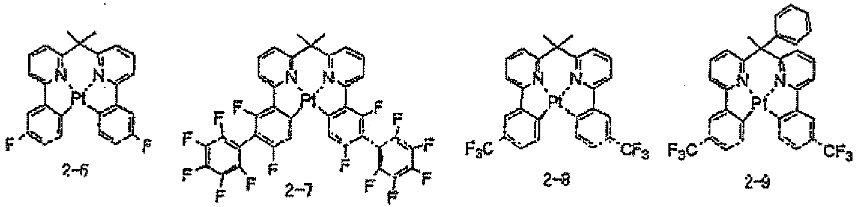

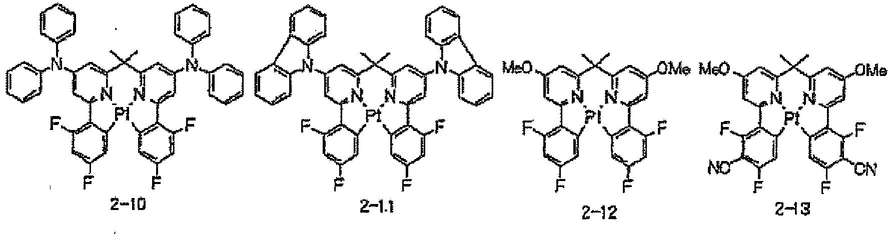

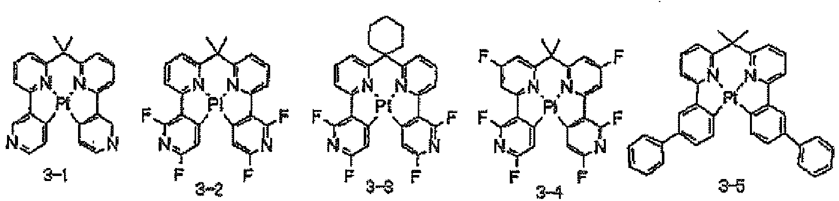

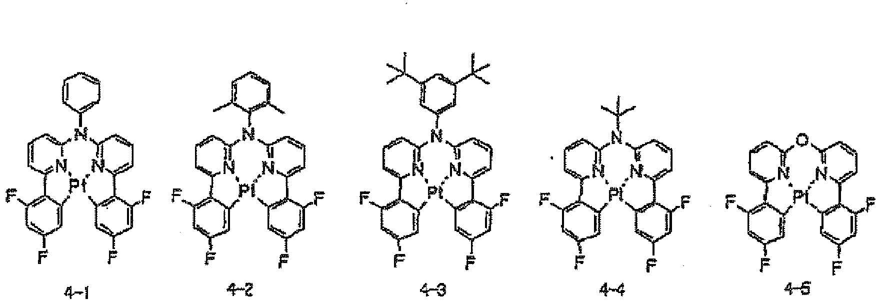

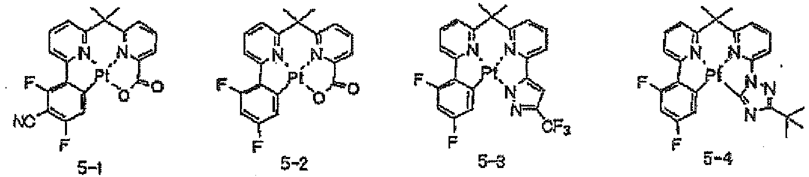

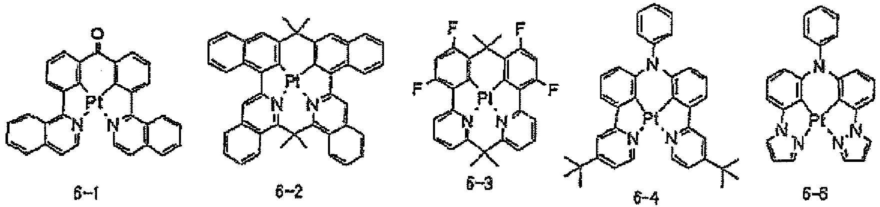

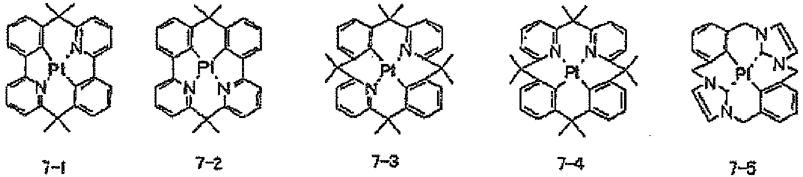

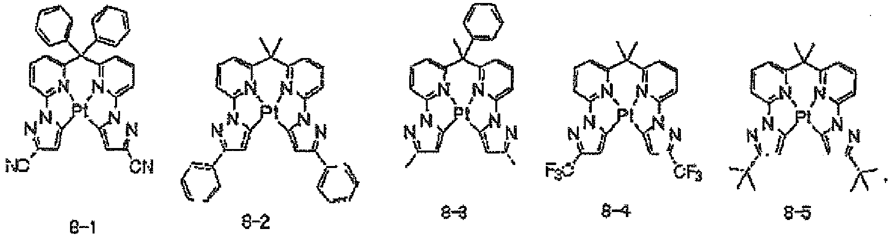

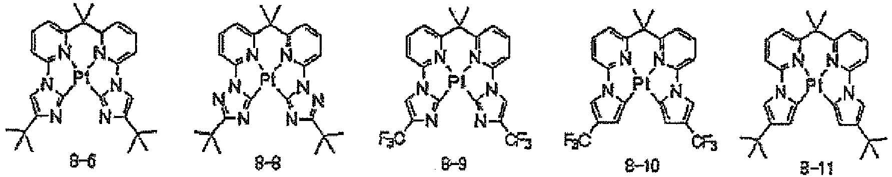

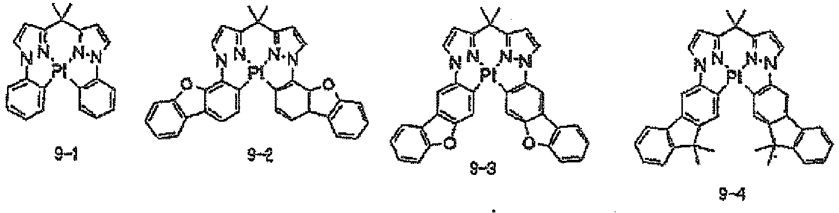

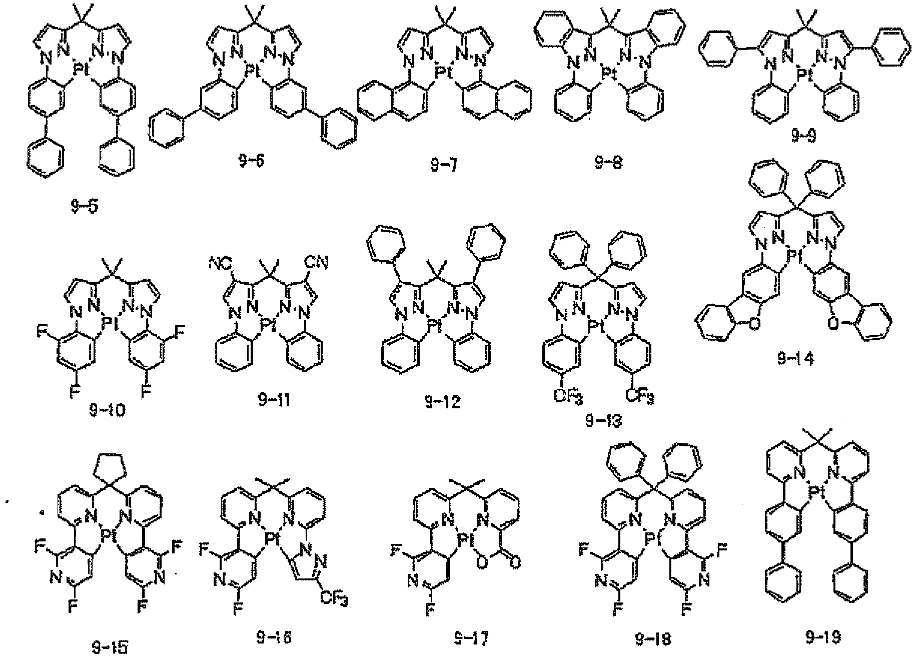

The platinum complex compound represented by Formula (C-1) may be synthesized by various techniques, for example, a method described on page 789, line 53 of the left-hand column to line 7 of the left-hand column, a method described on page 790, lines 18 to 3 of the left-hand column, a method described on page 790, lines 19 to 30 of the right-hand column in Journal of Organic Chemistry 53, 786, (1988), G. R. Newkome et al. and a combination thereof, a method described on page 2752, lines 26 to 35 in Chemische Berichte 113, 2749 (1980), H. Lexy et al., and the like.

For example, the platinum complex compound may be obtained by treating a ligand or a dissociate thereof and a metal compound in the presence or absence of a solvent (for example, a halogen-based solvent, an alcohol-based solvent, an ether-based solvent, an ester-based solvent, a ketone-based solvent, a nitrile-based solvent, an amide-based solvent, a sulfone-based solvent, a sulfoxide-based solvent, water and the like) and in the presence or absence of a base (various inorganic or organic bases, for example, sodium methoxide, t-butoxy potassium, triethylamine, potassium carbonate and the like) at room temperature or a lower temperature, or by heating (in addition to typical heating, a technique of heating by microwaves is also effective).

In the present invention, when the compound represented by Formula (C-1) is contained in the light emitting layer, the content thereof is preferably 1 to 30 mass%, more preferably 3 to 25 mass%, and even more preferably 5 to 20 mass%.

In the present invention, an iridium (Ir) complex may be used in combination as a light emitting material in addition to the platinum complex compounds. As the iridium (Ir) complex which is used in combination, a compound represented by the following formula (PQ-1) is preferably used.

The compound represented by Formula (PQ-1) will be described.

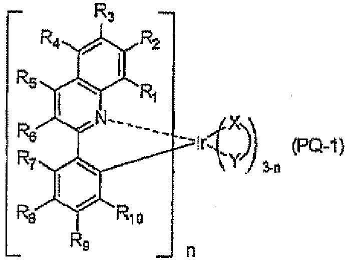

(In Formula PQ-1, R₁ to R₁₀ represent a hydrogen atom or a substituent. If possible, substituents may be linked to each other to form a ring. X-Y represents a mono-anionic bidentate ligand. n represents an integer of 1 to 3)

Examples of the substituent represented by R₁ to R₁₀ include the group A of substituents. R₁ to R₁₀ are preferably a hydrogen atom, an alkyl group (the alkyl group may have a fluorine atom, and preferably 1 to 8 carbon atoms, and more preferably 1 to 6 carbon atoms), a cycloalkyl group (preferably 3 to 20 carbon atoms, more preferably 3 to 10 carbon atoms, and even more preferably 5 to 10 carbon atoms), an aryl group (preferably 6 to 12 carbon atoms, and more preferably 6 to 10 carbon atoms), an amino group, an alkoxy group (preferably 1 to 8 carbon atoms, and more preferably 1 to 6 carbon atoms), an aryloxy group (preferably 6 to 12 carbon atoms, and more preferably 6 to 10 carbon atoms), an heterocyclic oxy group, a cyano group, a heterocyclic group (preferably 2 to 12 carbon atoms, and more preferably 3 to 10 carbon atoms), a silyl group, a silyloxy group and fluorine atoms, and more preferably a hydrogen atom, an alkyl group, a cycloalkyl group, an aryl group, an amino group, a heterocyclic group, an alkoxy group, a cyano group, a silyl group and fluorine atoms, and further preferably a hydrogen atom, a methyl group, an ethyl group, an isopropyl group, a t-butyl group, a neopentyl group, an isobutyl group, a phenyl group, a naphthyl group, a phenanthryl group and a tolyl group, and even further preferably a hydrogen atom, a methyl group and a phenyl group. When a ring is formed, two adjacent in the R₁ to R₁₀ are preferably linked to each other to form a ring, and R₇ and R₈, R₈ and R₉, or R₈ and R₁₀ are more preferably linked to each other to form a ring. When R₇ and R₈, R₈ and R₉, or R₉ and R₁₀ are linked to each other to form a ring, examples of the ring to be formed with a benzene ring substituted with R₇ to R₁₀ include an aryl ring and the like, which may be substituted with an alkyl group, an alkoxy group and the like.

The aryl ring to be formed is preferably an aryl ring having 6 to 30 carbon atoms, and more preferably an aryl ring having 6 to 15 carbon atoms. Examples of the aryl ring to be formed include a naphthalene ring, a phenanthrene ring, a fluorene ring and the like, preferably a naphthalene ring or a fluorene ring, and more preferably a naphthalene ring. These rings may be substituted with, for example, an alkyl group, an alkoxy group and the like, and are preferably a naphthalene ring or a fluorene ring which may be substituted with an alkyl group or an alkoxy group.

In Formula (PQ-1), it is preferable that each of 0 to 3 of R₁ to R₆ independently represents an alkyl group, a cycloalkyl group, an aryl group, a cyano group or a fluorine atom, and all of the remaining R₁ to R₆ are a hydrogen atom, it is more preferable that 0 or 1 of R₁ to R₆ represents an alkyl group, a cycloalkyl group, an aryl group, a cyano group, or a fluorine atom, and all of the remaining R₁ to R₆ are a hydrogen atom, and it is even more preferable that all of R₁ to R₆ are a hydrogen atom for enhancement of durability. In Formula (PQ-1), it is preferable that each of 0 to 2 of R₇ to R₁₀ independently represents an alkyl group, an aryl group, a cyano group, a heterocyclic group or a fluorine atom, and all of the remaining R₇ to R₁₀ are a hydrogen atom, it is more preferable that each of 0 to 2 of R₇ to R₁₀ independently represents an alkyl group, an aryl group, a cyano group or a fluorine atom, and all of the remaining R₇ to R₁₀ are a hydrogen atom, and it is even more preferable that R₉ represents an aryl group, and all of R₇, R₈, and R₁₀ are a hydrogen atom. Also, R₇ and R₈, R₈ and R₈, or R₈ and R₁₀ may be linked to each other to form the above-described ring, and when a ring is formed, the above-described aryl ring is more preferably formed and the benzene ring is even more preferably formed. If possible, substituents may be linked to each other to form a ring.

n is preferably 2 or 3, and more preferably 2.

(X-Y) represents a mono-anionic bidentate ligand. It is considered that these ligands may not contribute directly to light emission characteristics, and may control light emission characteristics of a molecule. "3-n" may be 0, 1, or 2. The mono-anionic bidentate ligand to be used in a light emitting material may be selected from those known in the art. Examples of the mono-anionic bidentate ligand include a ligand described on pages 89 and 90 of Lamansky, et al., the pamphlet of International Publication No. WO02/15645A, but the present invention is not limited thereto. Preferred mono-anionic bidentate ligands include acetylacetonate (acac) and picolinate (pic), and derivatives thereof. In the present invention, from the viewpoints of stability and high light emission quantum yield of the complex, the mono-anionic bidentate ligand is preferably acetylacetonate. In the following formula, M represents a metal atom.

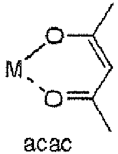

The compound represented by the above Formula (PQ-1) is preferably a compound represented by the following Formula (PQ-2).

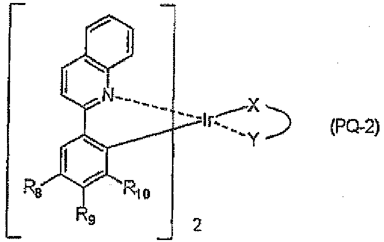

(In Formula (PQ-2), R₈ to R₁₀ represent a hydrogen atom or a substituent. If possible, substituents may be linked to each other to form a ring. X-Y represents a mono-anionic bidentate ligand)

R₈ to R₁₀ and X-Y have the same meaning as R₈ to R₁₀ and X-Y in Formula (PQ-1), and preferred ranges thereof are also the same.

Specific examples of the compound represented by Formula (PQ-1) are listed below, but are not limited to the following examples.

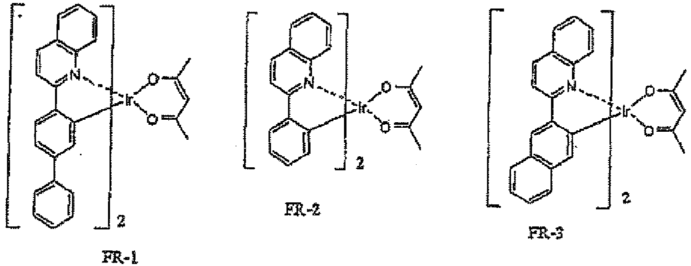

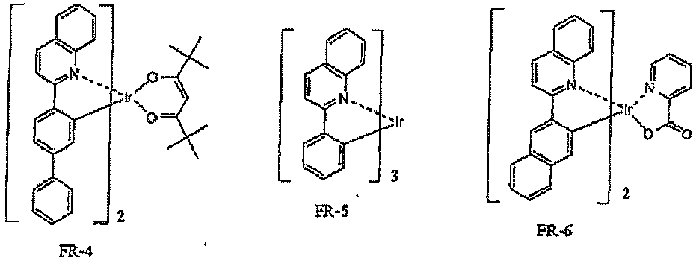

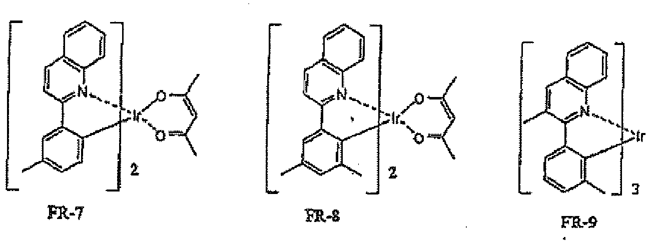

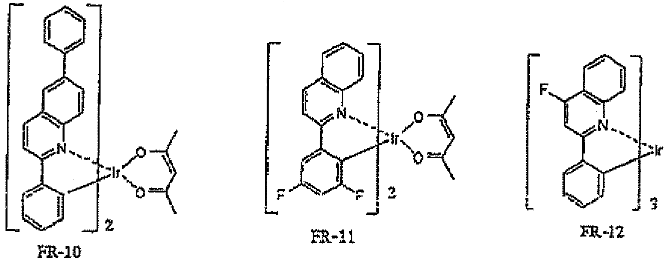

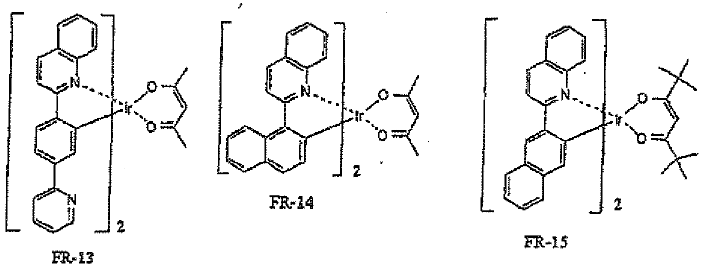

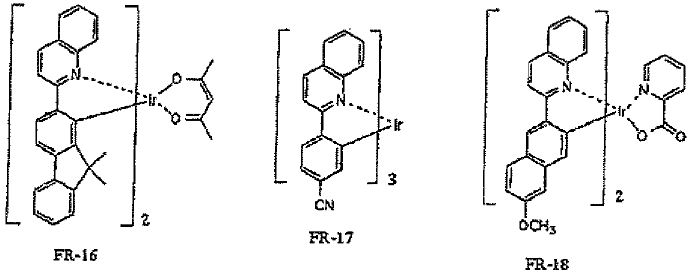

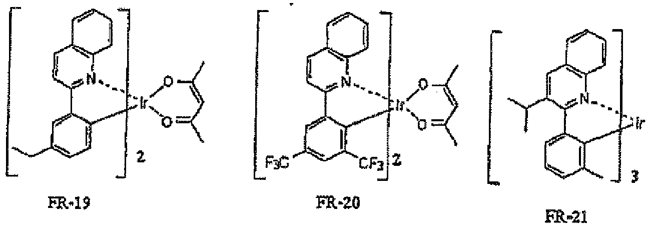

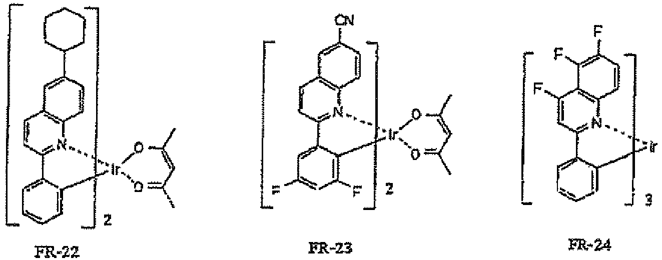

Compounds exemplified as the compounds represented by the above Formula (PQ-1) may be synthesized by various methods, for example, a method described in Japanese Patent No. 3929632, and the like. For example, FR-2 may be synthesized using 2-phenylquinoline as a starting raw material by a method described on page 18, lines 2 to 13 of Japanese Patent No. 3929632. Further, FR-3 may be synthesized using 2-(2-naphthyl)quinoline as a starting raw material by a method described on page 18, line 14 to page 19, line 8 of Japanese Patent No. 3929632.

In the present invention, when the compound represented by Formula (PQ-1) is contained in the light emitting layer, the content thereof is preferably 0.1 to 30 mass%, more preferably 2 to 20 mass%, and even more preferably 5 to 15 mass% in the light emitting layer.

### [Light emitting layer containing the compound represented by Formula (1) and the phosphorescent light emitting material represented by Formula (C-1)]

The present invention relates to a light emitting layer containing the compound represented by Formula (1) and the phosphorescent light emitting material represented by Formula (C-1). The light emitting layer of the present invention may be used in an organic electroluminescence device.

It is preferable that the light emitting layer of the present invention further contains the compound represented by Formula (PQ-1).

By using the compound represented by Formula (PQ-1) in combination with the compound represented by Formula (1) and the phosphorescent light emitting material represented by Formula (C-1), it is possible to provide an organic electroluminescence device having more excellent external quantum efficiency and driving durability. The content of the compound represented by Formula (PQ-1) in the light emitting layer of the present invention is preferably 0.1 to 30 mass%, more preferably 2 to 20 mass%, and even more preferably 3 to 20 mass% in the light emitting layer.

### [Composition containing the compound represented by Formula (1) and the phosphorescent light emitting material represented by Formula (C-1)]

The present invention relates to a composition containing the compound represented by Formula (1) and the phosphorescent light emitting material represented by Formula (C-1).

The content of the compound represented by Formula (1) in the composition of the present invention is preferably 15 to 99 mass%, more preferably 30 to 99 mass%, and even more preferably 50 to 99 mass%.

The content of the compound represented by Formula (C-1) in the composition of the present invention is preferably 1 to 30 mass%, more preferably 5 to 25 mass%, and even more preferably 10 to 20 mass%.

The other components that may be contained in the composition of the present invention may be an organic material or an inorganic material, and as an organic material, the following materials exemplified as a host material, a fluorescent light emitting material, a phosphorescent light emitting material and a hydrocarbon material may be applied, a host material and a hydrocarbon material are preferable, and a compound represented by Formula (VI) is more preferable.

The composition of the present invention may form an organic layer of the organic electroluminescence device by a dry film forming method such as a deposition method or a sputtering method, a transfer method, and a print method.

It is preferable that the composition of the present invention further contains the compound represented by Formula (PQ-1).

The content of the compound represented by Formula (PQ-1) in the composition of the present invention is preferably 1 to 30 mass%, more preferably 2 to 20 mass%, and even more preferably 5 to 15 mass% in the composition.

### [Organic Electroluminescence Device]

The device of the present invention will be described in detail.

The organic electroluminescence device of the present invention has an organic layer including a light emitting layer between a pair of electrodes. Due to properties of the luminescence device, at least one electrode of the anode and the cathode is preferably transparent or semi-transparent. The organic layer may include a hole injection layer, a hole transporting layer, a hole blocking layer, an electron transporting layer and the like, in addition to the light emitting layer.

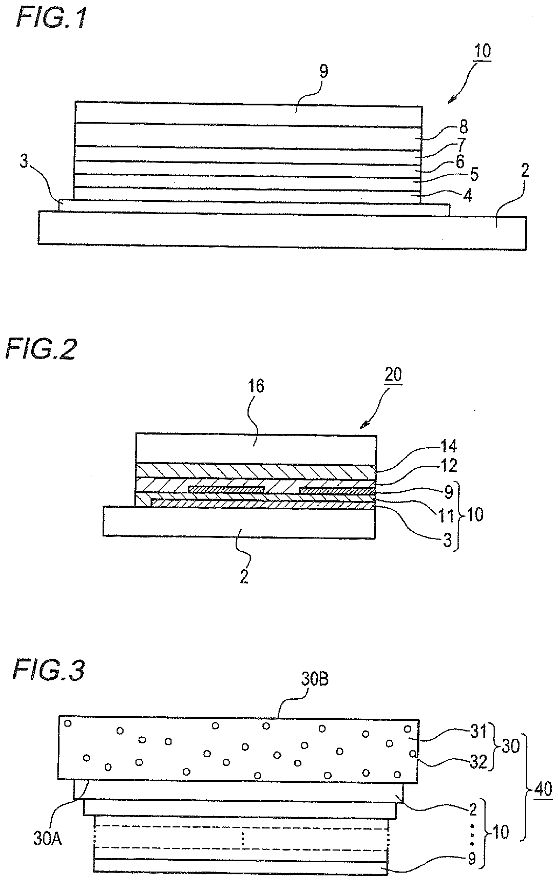

FIG. 1 shows an example of the configuration of an organic electroluminescence device according to the present invention. In FIG. 1, an organic electroluminescence device 10 has an organic layer including a light emitting layer 6 between a pair of electrodes (an anode 3 and a cathode 9) on a substrate 2. As the organic layer, a hole injection layer 4, a hole transporting layer 5, the light emitting layer 6, a hole blocking layer 7, and an electron transporting layer 8 from the anode side 3 are laminated in this order.

The device configuration, substrate, cathode, and anode of the organic electroluminescence device are described in detail in, for example, Japanese Patent Application Laid-Open No. 2008-270736, and the subject matters described in the publication may be applied to the present invention.

### (Light Emitting Layer)

The light emitting layer is a layer having functions, when applying an electric field, of accepting a hole from the anode, the hole injection layer or the hole transporting layer, and accepting an electron from the cathode, the electron injection layer or the electron transporting layer to provide a site of recombination of the hole and the electron, thereby achieving light emission.

### <Light Emitting Material>

As the light emitting material in the invention, a phosphorescence emitting material represented by Formula (C-1) may be used. However, in addition to the material, a fluorescent material or a phosphorescence emitting material may be used in combination.

The fluorescence emitting material and the phosphorescence emitting material which may be used in combination are described in detail, in, for example, paragraph Nos. [0100] to [0164] of Japanese Patent Application Laid-Open No. 2008-270736 and paragraph Nos. [0088] to [0090] of Japanese Patent Application Laid-Open No. 2007-266458, and the subject matters described in these publications may be applied to the present invention.

The light emitting material in the light emitting layer is generally contained in an amount of 0.1 mass% to 50 mass% based on the total mass of the compounds forming the light emitting layer. However, from the viewpoints of durability, external quantum efficiency, low driving voltage and change in chromaticity while driving at a high temperature, the material is contained in an amount of preferably 1 mass% to 30 mass%, more preferably 5 mass% to 25 mass%, and even more preferably 7 mass% to 20 mass%.

A thickness of the light emitting layer is not particularly limited, but typically, is preferably 2 nm to 500 nm, and among them, from the viewpoint of external quantum efficiency, the thickness of the light emitting layer is more preferably 3 nm to 200 nm, and even more preferably 5 nm to 100 nm.

### <Host Material>

In the light emitting layer, a host material is preferably used with the light emitting material. The host material refers to a compound which is usually responsible for injecting and transporting electric charges in the light emitting layer, and which does not substantially emit light in itself. As used herein, "not substantially emit light" means that the amount of light emission from the compound that does not substantially emit light is preferably 5% or less based on the total amount of light emission in the whole device, more preferably 3% or less, and even more preferably 1% or less.

As the host material used in the present invention, for example, the following compounds may be used.

Examples may include, for example, pyrrole, indole, carbazole, azaindole, azacarbazole, triazole, oxazole, oxadiazole, pyrazole, imidazole, thiophene, polyarylalkane, pyrazoline, pyrazolone, phenylenediamine, arylamine, amino substituted carcone, styrylanthracene, fluorenone, hydrazone, stilbene, silazane, aromatic tertiary amine compound, a styrylamine compound, a porphyrin-based compound, a conductive polymer oligomers such as a polysilane-based compound, poly(N-vinylcarbazole), an aniline-based copolymer, thiophene oligomer and polythiophene, organic silane, a carbon film, pyridine, pyrimidine, triazine, imidazole, pyrazole, triazole, oxazole, oxadiazole, fluorenone, anthraquinodimethane, anthrone, diphenylquinone, thiopyrandioxide, carbodiimide, fluorenylidene methane, distyrylepyrazine, fluorine substituted aromatic compound, heterocyclic tetracarboxylic acid anhydrides such as naphthalene perylene, various metal complexes represented by a metal complex of phthalocyanine or a 8-quinolinol derivative, metal phthalocyanine, and a metal complex using benzoxazole or benzothiazole as a ligand, and derivatives thereof (may have a substituent or a condensed ring).

In the light emitting layer of the present invention, the lowest triplet excited state energy (T₁ energy) of the host material is preferably higher than the T₁ energy of the phosphorescent light emitting material from the viewpoint of chromaticity, emission efficiency, and driving durability.

Further, the content of the host compound in the present invention is not particularly limited, but from the viewpoint of emission efficiency and driving voltage, the content is preferably 15 mass% or more and 95 mass% or less on the basis of the total mass of the compound forming the light emitting layer.

It is more preferable that the organic electroluminescence device contains a hydrocarbon compound, and it is even more preferable that the light emitting layer contains a hydrocarbon compound.

Further, the hydrocarbon compound is preferably a compound represented by the following Formula (VI).

By appropriately using the compound represented by Formula (VI) in conjunction with the light emitting material, it is possible to appropriately control an interaction between material molecules, and make an energy gap interaction between the adjacent molecules uniform, thereby further decreasing driving voltage.

In addition, the compound represented by Formula (VI) used in the organic electroluminescence device may prevent a decrease in efficiency of the organic electroluminescence device or a decrease in the lifespan of the device by decomposed products of the material because the chemical stability is excellent, and denaturing such as decomposition of the material when driving the device is hardly occurred.

The compound represented by Formula (VI) will be described.

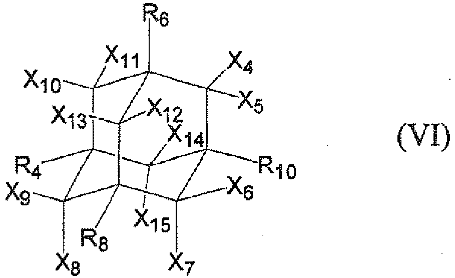

In Formula (VI), each of R₄, R₈, R₈, R₁₀, and X₄ to X₁₅ independently represents a hydrogen atom, an alkyl group or an aryl group.

The alkyl group represented by R₄, R₆, R₈, R₁₀ and X₄ to X₁₅ in Formula (VI) may be substituted with an adamantane structure or an aryl structure, and has preferably 1 to 70 carbon atoms, more preferably 1 to 50 carbon atoms, even more preferably 1 to 30 carbon atoms, much more preferably 1 to 10 carbon atoms, particularly preferably 1 to 6 carbon atoms, and most preferably a straight-chained alkyl group having 2 to 6 carbon atoms.

The alkyl group represented by R₄, R₆, R₈, R₁₀ and X₄ to X₁₅ in Formula (VI) may include, for example, a n-C₅₀H₁₀₁ group, a n-C₃₀H₆₁ group, a 3-(3,5,7-triphenyladamantane-1-yl)propyl group (31 carbon atoms), a trityl group (19 carbon atoms), a 3-(adamantane-1-yl)propyl group (13 carbon atoms), a 9-decanyl group (10 carbon atoms), a benzyl group (7 carbon atoms), a cyclohexyl group (6 carbon atoms), a n-hexyl group (6 carbon atoms), a n-pentyl group (5 carbon atoms), a n-butyl group (4 carbon atoms), a n-propyl group (3 carbon atoms), a cyclopropyl group(3 carbon atoms), an ethyl group (2 carbon atoms) and a methyl group (1 carbon atom).

The aryl group represented by R₄, R₆, R₈, R₁₀ and D to X₁₅ in Formula (VI) may be substituted with an adamantane structure or an alkyl structure, and has preferably 6 to 30 carbon atoms, more preferably 6 to 20 carbon atoms, even more preferably 6 to 15 carbon atoms, particularly preferably 6 to 10 carbon atoms, and most preferably 6 carbon atoms.

The aryl group represented by R₄, R₆, R₈, R₁₀ and X₄ to X₁₅ in Formula (VI) may include, for example, a 1-pyrenyl group (16 carbon atoms), a 9-anthracenyl group (14 carbon atoms), a 1-naphthyl group (10 carbon atoms), a 2-naphthyl group (10 carbon atoms), a p-t-butylphenyl group (10 carbon atoms), a 2-m-xylyl group (8 carbon atoms), a 5-m-xylyl group (8 carbon atoms), an o-tolyl group (7 carbon atoms), an m-tolyl group (7 carbon atoms), a p-tolyl group (7 carbon atoms), and a phenyl group (6 carbon atoms).

R₄, R₆, R₈ and R₁₀ in Formula (VI) may be a hydrogen atom, an alkyl group or an aryl group, but from the viewpoint of the above description that the high glass transition temperature is preferable, it is preferable that at least one is an aryl group, it is more preferable that at least two are aryl groups, and it is particularly preferable that three to four are aryl groups.

X₄ to X₁₅ in Formula (VI) may be a hydrogen atom, an alkyl group or an aryl group, preferably a hydrogen atom or aryl group, and particularly preferably a hydrogen atom.

The molecular weight of the compound represented by Formula (VI) in the present invention is preferably 2000 or less, more preferably 1200 or less, and particularly preferably 1000 or less from the viewpoint of deposition application or solubility, because the organic electroluminescence device is manufactured by using a vacuum deposition process or a solution coating process. Further, from the viewpoint of deposition application, if the molecular weight is too small, a vapor pressure is decreased and a change from a gas phase to a solid phase does not occur, and as a result, it is difficult to form the organic layer. Therefore, the molecular weight is preferably 250 or more, more preferably 350 or more, and particularly preferably 400 or more.

The compound represented by Formula (VI) is preferably a solid at room temperature (25°C), more preferably a solid from room temperature (25°C) to 40°C, and particularly preferably a solid from room temperature (25°C) to 60°C.

In the case where the compound represented by Formula (VI), which does not form a solid at room temperature (25°C), is used, a solid phase may be formed at ambient temperature by combining with other materials.

The purpose of the compound represented by Formula (VI) is not particularly limited, and the compound may be contained in any layer of the organic layers. A layer introducing the compound represented by Formula (VI) in the present invention is preferably any one or a plurality of layers of a light emitting layer, a hole injection layer, a hole transporting layer, an electron transporting layer, an electron injection layer, an exciton blocking layer and a charge blocking layer as described below, more preferably any one or a plurality of layers of a light emitting layer, a hole injection layer, a hole transporting layer, an electron transporting layer and an electron injection layer, particularly preferably any one or a plurality of layers of a light emitting layer, a hole injection layer and a hole transporting layer, and most preferably a light emitting layer.

In the case where the compound represented by Formula (VI) is used in the organic layer, it is required that the content of the compound represented by Formula (VI) used is limited to an amount that does not suppress charge transportability, and the compound represented by Formula (VI) is contained in an amount of preferably 0.1 to 70 mass%, more preferably 0.1 to 30 mass%, and particularly preferably 0.1 to 25 mass%.

Further, in the case where the compound represented by Formula (VI) is used in a plurality of organic layers, it is preferable that the compound is contained in the above range in each layer.

Only one kind of compound represented by Formula (VI) may be contained in any one of organic layers, or a plurality of compounds represented by Formula (VI) may be contained in combination at a predetermined ratio.

Particular examples of the hydrocarbon compound will be described below, but are not limited thereto.

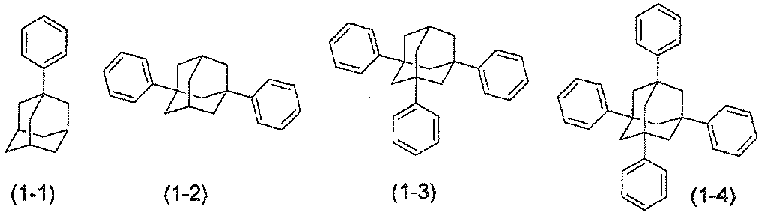

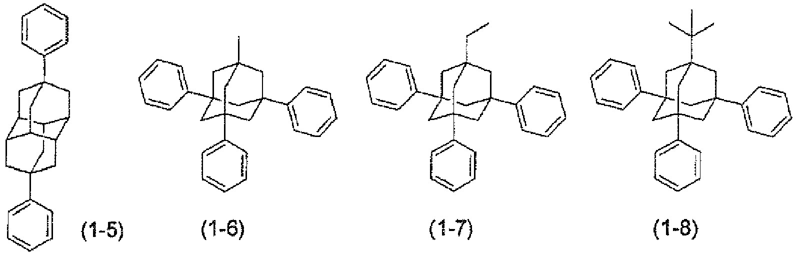

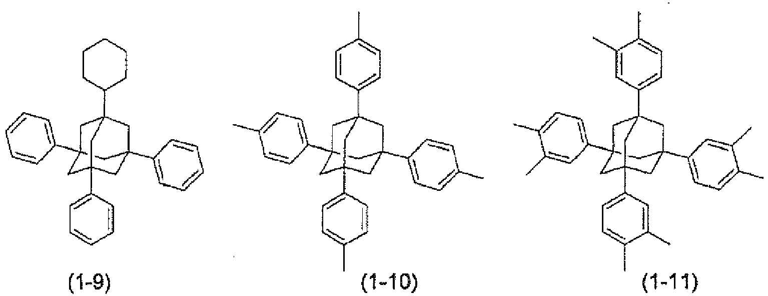

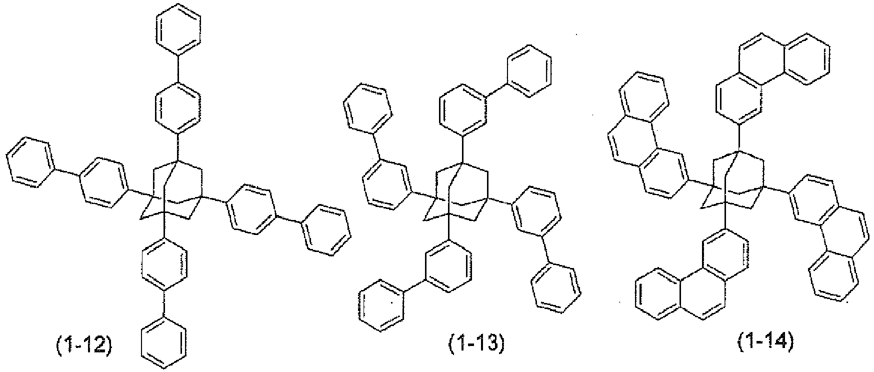

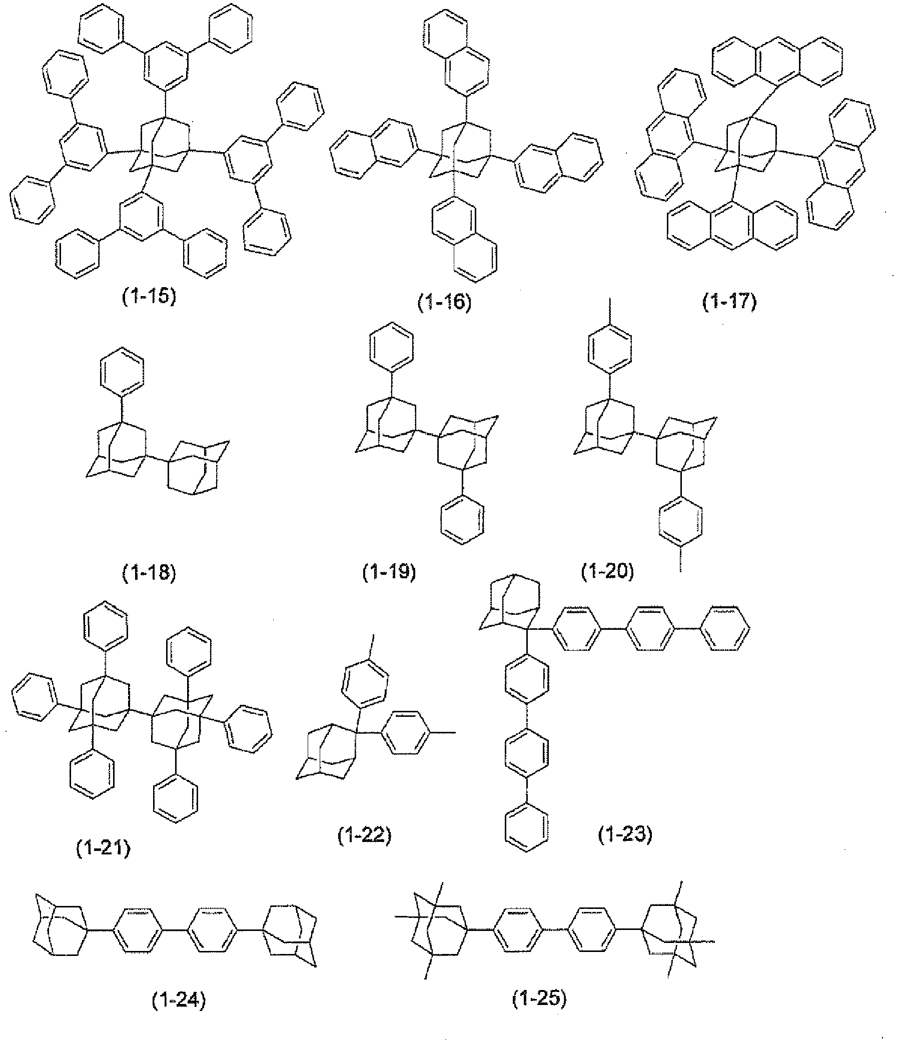

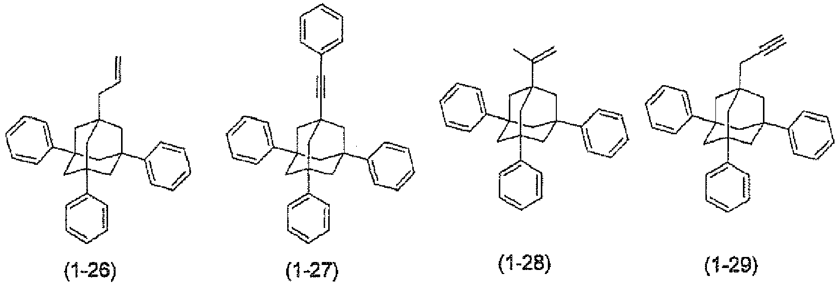

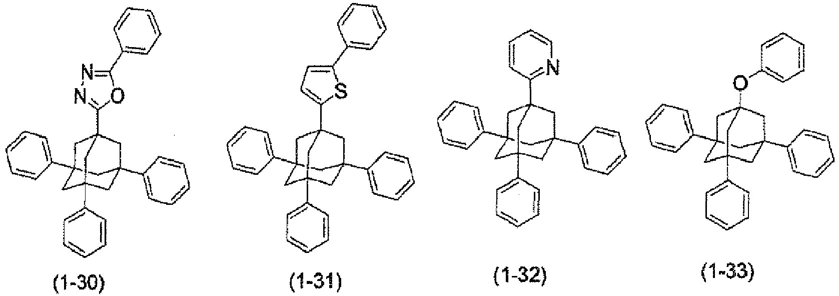

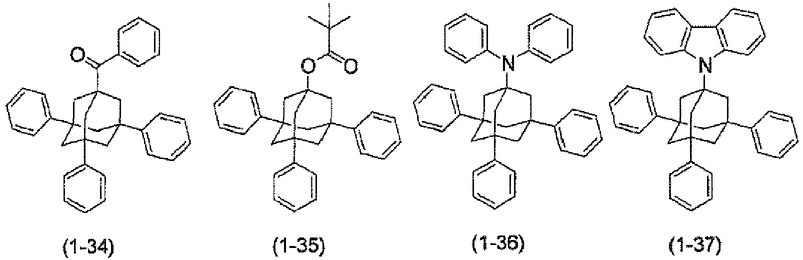

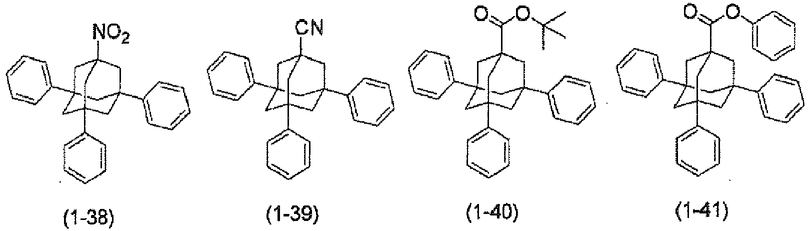

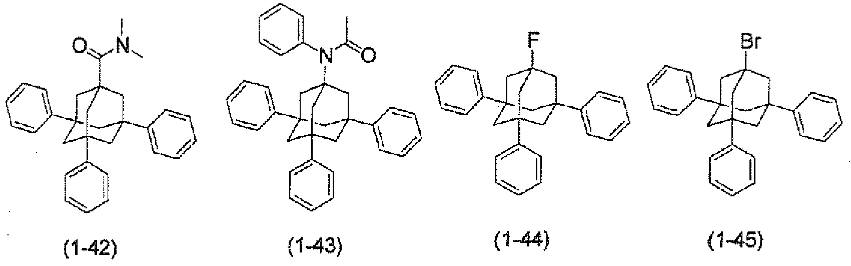

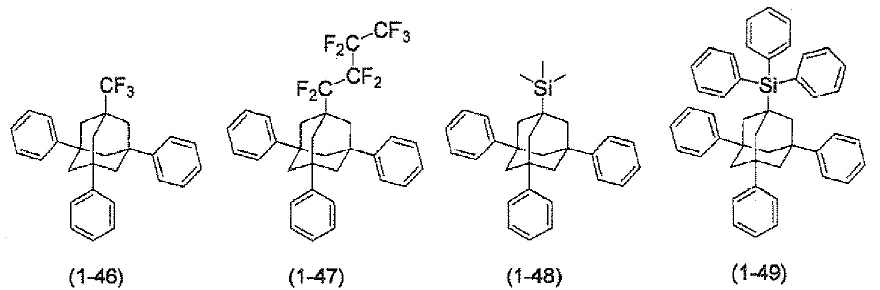

The compound represented by Formula (VI) may be synthesized by appropriately combining adamantane, halogenated adamantine, halogenated alkyl or alkylmagnesium halide (Grignard reagent). For example, halogenated adamantane and halogenated alkyl may be coupled by using indium (Document 1). Further, the aromatic compound may be coupled with the Grignard reagent by converting halogenated alkyl with an alkylcopper reagent (Document 2). In addition, halogenated alkyl may be coupled by using an appropriate arylboric acid and a palladium catalyst (Document 3).

Document 1: Tetrahedron Lett. 39,1998, 9557-9558.

Document 2: Tetrahedron Lett. 39, 1998, 2095-2096.

Document 3: J. Am. Chem. Soc. 124, 2002, 13662-13663.

The adamantane structure having an aryl group may be synthesized by appropriately combining adamantane or halogenated adamantane with corresponding arene or aryl halide.

Further, in the manufacturing method as described above, in the case where the defined substituent is changed under conditions of a certain synthesis method or is not suitable to perform the above method, the compound may be easily manufactured by means such as protection or unprotection of the functional group (for example, Protective Groups in Organic Synthesis, written by T. W. Greene, John Wiley & Sons Inc.) (1981)). In addition, if necessary, it is possible to change the order of the reaction process such as appropriate introduction of the substituent.

In the organic electroluminescence device of the present invention, it is preferable that the electrode contains the anode, the charge transporting layer is contained between the light emitting layer and the anode, and the charge transporting layer contains a carbazole compound.

### (Charge transporting layer)

The charge transporting layer refers to a layer which transports charge occurs when voltage is applied to the organic electroluminescence device. Particularly, the layer may be a hole injection layer, a hole transporting layer, an electron blocking layer, a light emitting layer, a hole blocking layer, an electron transporting layer or an electron injection layer. A hole injection layer, a hole transporting layer, an electron blocking layer or a light emitting layer is preferred. If the charge transporting layer formed by a coating method is a hole injection layer, a hole transporting layer, an electron blocking layer or a light emitting layer, it is possible to manufacture the organic electroluminescence device having high efficiency at low cost. Further, the charge transporting layer is more preferably a hole injection layer, a hole transporting layer or an electron blocking layer.

### -Hole injection layer and hole transporting layer-

Each of the hole injection layer and the hole transporting layer are a layer having a function of accepting holes from the anode or the anode side to transport the holes into the cathode side.

A hole injection material or a hole transporting material used in these layers may be a low molecular compound or a polymer compound.

Specifically, the above layer is preferably a layer containing a carbazole derivative, a pyrrole derivative, a triazole derivative, an oxazole derivative, a oxadiazole derivative, an imidazole derivative, a polyarylalkane derivative, a pyrazoline derivative, a pyrazolone derivative, a phenylenediamine derivative, an arylamine derivative, an amino substituted carcone derivative, a styrylanthracene derivative, a fluorenone derivative, a hydrazone derivative, a stilbene derivative, a silazane derivative, an aromatic tertiary amine compound, a styrylamine compound, a phthalocyanine-based compound, a porphyrin-based compound, a thiophene derivative, an organic silane derivative, a carbon, and various metal complexes such as an iridium complex.

It is preferable that the hole injection layer and the hole transporting layer include a carbazole compound.

In the present invention, it is preferable that the carbazole compound is a carbazole compound represented by the following Formula (a).

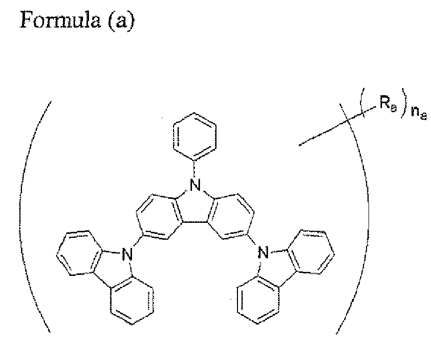

(In Formula (a), Rₐ represents a substituent which may be substituted with a hydrogen atom in the structure, and when a plurality of Rₐ exists, each of Rₐ may be the same as or different from every other Rₐ, and nₐ represents an integer of 0 to 8.)

In the case where the compound represented by Formula (a) is used in the charge transporting layer, the compound represented by Formula (a) is contained in an amount of preferably 50 to 100 mass%, preferably 80 to 100 mass%, and particularly preferably 95 to 100 mass%.

Further, in the case where the compound represented by Formula (a) is used in a plurality of organic layers, the compound is preferably contained in the above range in each layer.

Only one kind of compound represented by Formula (a) may be contained in any one organic layer, or a plurality of compounds represented by Formula (a) may be contained in combination at a predetermined ratio.

In the present invention, in the case where the compound represented by Formula (a) is contained in the hole transporting layer, the thickness of the hole transporting layer containing the compound represented by Formula (a) is preferably 1 nm to 500 nm, more preferably 3 nm to 200 nm, and even more preferably 5 nm to 100 nm. Further, it is preferable that the hole transporting layer is installed while being contacted with the light emitting layer.

The hole transporting layer may have a single layer structure composed of one or two or more kinds of the above-described materials, or may have a multilayer structure composed of a plurality of layers of the same or different compositions.

The substituent represented by Rₐ may include, specifically, a halogen atom, an alkoxy group, a cyano group, a nitro group, an alkyl group, an aryl group and an aromatic heterocyclic group, preferably an alkyl group having 10 or less carbon atoms and a substituted or unsubstituted aryl group having 10 or less carbon atoms, and more preferably an alkyl group having 6 or less carbon atoms. nₐ is preferably 0 to 4, and more preferably 0 to 2.

In the present invention, the hydrogen atoms constituting Formula (a) include isotopes of hydrogen (such as deuterium atom) as well. In this case, all hydrogen atoms of the compound may be replaced by isotopes of hydrogen, and may be a mixture that is a compound in which a portion thereof contains isotopes of hydrogen.

The compounds represented by Formula (a) may be synthesized by combining various known synthesis methods. Most generally, with respects to the carbazole compound, there may be a synthesis by dehydrogenation aromatization after Aza-Cope rearrangement reaction of arylhydrazine and a condensate with cyclohexane derivative (written by L. F. Tieze, and Th. Eicher, translated by Dakano, Ogasawara, Fine Organic Synthesis, p. 339 (published by Nankodo Co., Ltd.)). Further, with respects to the coupling reaction of the obtained carbazole compound and halogenated aryl compound using the palladium catalyst, there may be a method described in Tetrahedron Letters Vol. 39, p. 617 (1998), Vol. 39, p. 2367 (1998) and Vol. 40, p. 6393 (1999). The reaction temperature and the reaction time are not particularly limited, and the condition described in the above document may be applied.

In the present invention, it is preferable that the compounds represented by Formula (a) form a thin layer by a vacuum deposition process, but a wet process such as solution coating may be appropriately used. The molecular weight of the compound is preferably 2,000 or less, more preferably 1,200 or less, and particularly preferably 800 or less from the viewpoint of deposition application or solubility. Further, from the viewpoint of deposition application, if the molecular weight is too small, a vapor pressure is decreased and a change from a gas phase to a solid phase does not occur, and as a result, it is difficult to form the organic layer. Therefore, the molecular weight is preferably 250 or more, and particularly preferably 300 or more.

Hereinafter, particular examples of the compound represented by Formula (a) in the present invention are exemplified, but the present invention is not limited thereto.

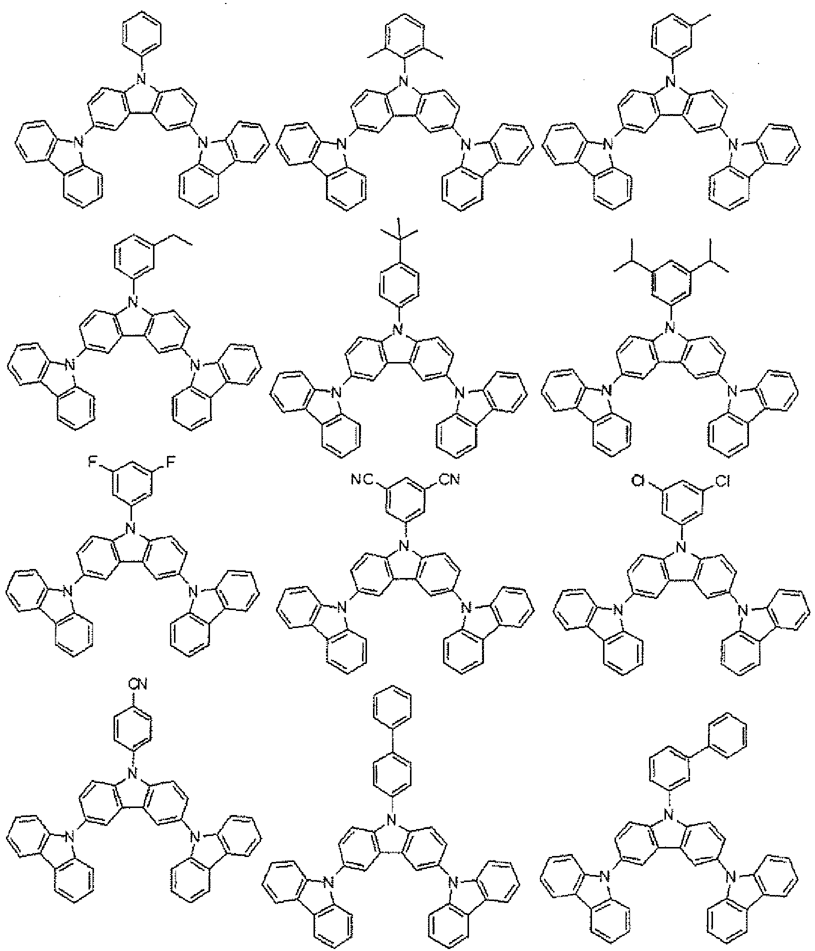

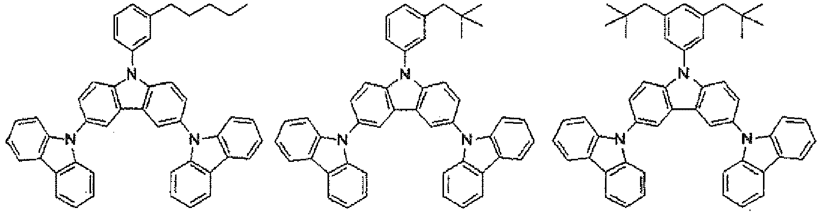

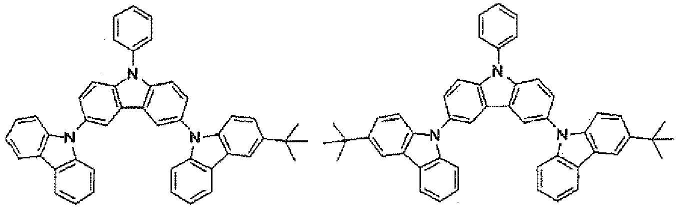

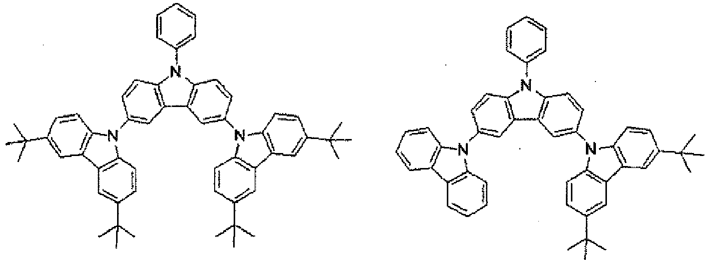

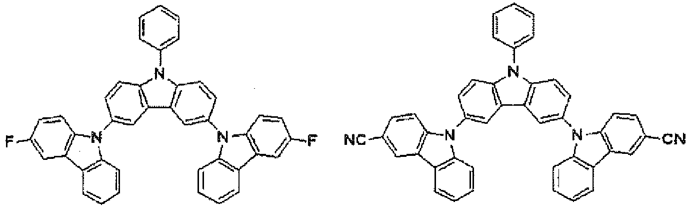

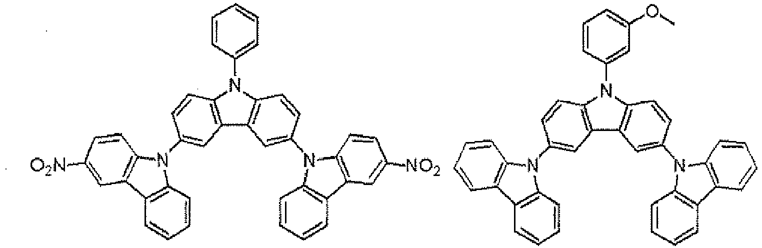

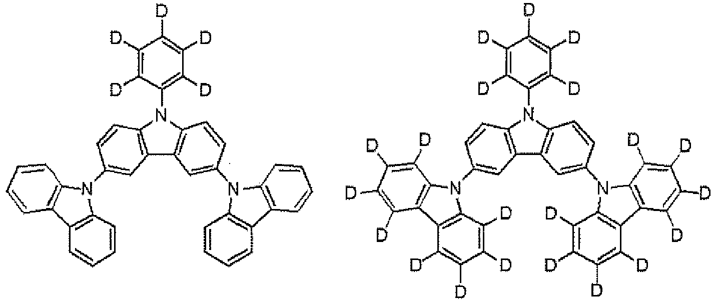

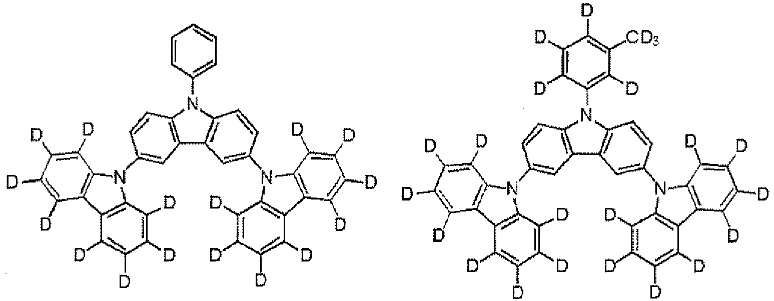

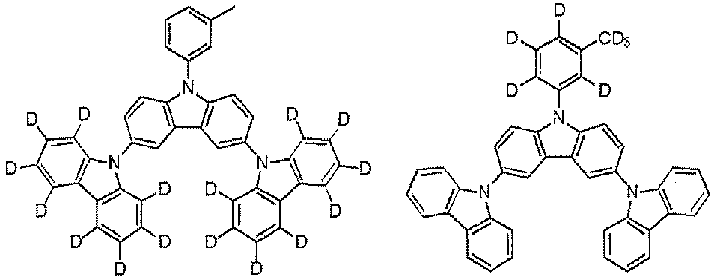

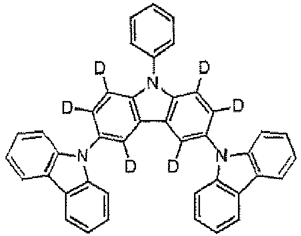

The hole injection layer or hole transporting layer of the organic electroluminescence device of the present invention may contain an electron-accepting dopant. As an electron-accepting dopant introduced into the hole injection layer or hole transporting layer, an inorganic compound or an organic compound may be used so long as the compound is electron-accepting, and has a property to oxidize organic compounds.

Specifically, the inorganic compound may contain halogenated metal such as ferric chloride, aluminum chloride, gallium chloride, indium chloride and antimony pentachloride, and metal oxide such as vanadium pentoxide and molybdenum trioxide.

In the case of the organic compound, a compound having a nitro group, a halogen, a cyano group or a trifluoromethyl group as a substituent, a quinone-based compound, an acid anhydride-based compound and fullerene may be appropriately used.

In addition to these, compounds described in Japanese Patent Application Laid-Open No.6-212153, Japanese Patent Application Laid-Open No. Hei 11-111463, Japanese Patent Application Laid-Open No. Hei 11-251067, Japanese Patent Application Laid-Open No. 2000-196140, Japanese Patent Application Laid-Open No. 2000-286054, Japanese Patent Application Laid-Open No. 2000-315580, Japanese Patent Application Laid-Open No. 2001-102175, Japanese Patent Application Laid-Open No. 2001-160493, Japanese Patent Application Laid-Open No. 2002-252085, Japanese Patent Application Laid-Open No. 2002-56985, Japanese Patent Application Laid-Open No. 2003-157981, Japanese Patent Application Laid-Open No. 2003-217862, Japanese Patent Application Laid-Open No. 2003-229278, Japanese Patent Application Laid-Open No. 2004-342614, Japanese Patent Application Laid-Open No. 2005-72012, Japanese Patent Application Laid-Open No. 2005-166637, and Japanese Patent Application Laid-Open No. 2005-209643 may be appropriately used.

Among them, hexacyanobutadiene, hexacyanobenzene, tetracyanoethylene, tetracyanoquinodimethane, tetrafluorotetracyanoquinodimethane, p-fluoranyl, p-chloranyl, p-bromanyl, p-benzoquinone, 2,6-dichlorobenzoquinone, 2,5-dichlorobenzoquinone, 1,2,4,5-tetracyanobenzene, 1,4-dicyanotetrafluorobenzene, 2,3-dichloro-5,6-dicyanobenzoquinone, p-dinitrobenzene, m-dinitrobenzene, o-dinitrobenzene, 1,4-naphthoquinone, 2,3-dichloronaphthoquinone, 1,3-dinitronaphthalene, 1,5-dinitronaphthalene, 9,10-anthraquinone, 1,3,6,8-tetranitrocarbazole, 2,4,7-trinitro-9-fluorenone, 2,3,5,6-tetracyanopyridine, or fullerine C60 is preferable, hexacyanobutadiene, hexacyanobenzene, tetracyanoethylene, tetracyanoquinodimethane, tetrafluorotetracyanoquinodimethane, p-fluoranil, p-chloranyl, p-bromanyl, 2,6-dichlorobenzoquinone, 2,5-dichlorobenzoquinone, 2,3-dichloronaphthoquinone, 1,2,4,5-tetracyanobenzene, 2,3-dichloro-5,6-dicyanobenzoquinone or 2,3,5,6-tetracyanopyridine is more preferable, and tetrafluorotetracyanoquinodimethane is particularly preferable.

These electron-accepting dopants may be used either alone or in combination of 2 or more kinds thereof. The amount of electron-accepting dopant used varies depending on a kind of material, but preferably 0.01 mass% to 50 mass%, more preferably 0.05 mass% to 20 mass%, and particularly preferably 0.1 mass% to 10 mass% on the basis of the hole transporting layer material.

The thicknesses of each of the hole injection layer and the hole transporting layer preferably are 500 nm or less from the viewpoint of a decrease in driving voltage.

The thickness of the hole transporting layer is preferably 1 nm to 500 nm, more preferably 5 nm to 200 nm, and even more preferably 10 nm to 100 nm. The thickness of the hole injection layer is preferably 0.1 nm to 200 nm, more preferably 0.5 nm to 100 nm, and even more preferably 1 nm to 100 nm.

The hole injection layer and hole transporting layer may have a single layer structure composed of one or two or more kinds of the above-described materials, or may have a multilayer structure composed of a plurality of layers of the same or different compositions.

### -Electron Injection Layer and Electron Transporting Layer-

Each of the electron injection layer and the electron transporting layer is a layer having a function of accepting electrons from the cathode or the cathode side to transport the electron into the anode side. An electron injection material and an electron transporting material, which are used in these layers, may be a low-molecular weight compound or a polymer compound.

Specifically, each of the electron injection layer and the electron transporting layer is preferably a layer containing, in addition to the compound represented by Formula (1) of the present invention, a pyridine derivative, a quinoline derivative, a pyrimidine derivative, a pyrazine derivative, a phthalazine derivative, a phenanthroline derivative, a triazine derivative, a triazole derivative, an oxazole derivative, an oxadiazole derivative, an imidazole derivative, a fluorenone derivative, an anthraquinonedimethane derivative, an anthrone derivative, a diphenylquinone derivative, a thiopyrandioxide derivative, a carbodiimide derivative, a fluorenylidenemethane derivative, a distyrylpyrazine derivative, an aromatic tetracarboxylic acid anhydride of naphthalene, perylene and the like, various metal complexes represented by metal complexes of a phthalocyanine derivative or an 8-quinolinol derivative or metal complexes containing, as a ligand, metal phthalocyanine, benzoxazole or benzothiazole, an organic silane derivative represented by silole, or the like.

An electron-donating dopant may be contained in the electron injection layer or the electron transporting layer of the organic electroluminescence device of the present invention. As the electron-donating dopant which is introduced into the electron injection layer or the electron transporting layer, alkali metals such as Li, alkaline earth metals such as Mg, transition metals including rare earth metals, reducing organic compounds and the like are appropriately used so far as the materials are electron-donating and have properties of reducing an organic compound. In particular, a metal having a work function of 4.2 eV or less may be appropriately used as the metal. Specific examples thereof include Li, Na, K, Be, Mg, Ca, Sr, Ba, Y, Cs, La, Sm, Gd, Yb and the like. Also, examples of the reducing organic compound include nitrogen-containing compounds, sulfur-containing compounds, phosphorus-containing compounds and the like.

Besides, materials described in Japanese Patent Application Laid-Open No. Hei 6-212153, Japanese Patent Application Laid-Open No. 2000-196140, Japanese Patent Application Laid-Open No. 2003-68468, Japanese Patent Application Laid-Open No. 2003-229278, Japanese Patent Application Laid-Open No. 2004-342614, and the like may be used.

These electron-donating dopants may be used either alone or in combinations of two or more species thereof. Though an amount of the electron-donating dopant used varies depending upon the kind of the material, the amount is preferably 0.1 mass% to 99 mass%, more preferably 1.0 mass% to 80 mass%, and particularly preferably 2.0 mass% to 70 mass% based on the electron transporting layer material.

From the viewpoint of lowering the driving voltage, a thickness of each of the electron injection layer and the electron transporting layer is preferably 500 nm or less.

The thickness of the electron transporting layer is preferably 1 nm to 500 nm, more preferably 5 nm to 200 nm, and even more preferably from 10 nm to 100 nm. Also, the thickness of the electron injection layer is preferably 0.1 nm to 200 nm, more preferably 0.2 nm to 100 nm, and even more preferably 0.5 nm to 50 nm.

Each of the electron injection layer and the electron transporting layer may have a single layer structure composed of one or two or more kinds of the above-described materials, or may have a multilayer structure composed of a plurality of layers of the same or different compositions.

### -Hole blocking layer-

The hole blocking layer is a layer having a function of preventing the hole transported from the anode side to the light emitting layer from being discharged to the cathode side. In the present invention, the hole blocking layer may be installed as an organic layer that is adjacent to the light emitting layer and the cathode side.

Examples of the organic compound configuring the hole blocking layer may include an aluminum complex such as aluminum(III)bis(2-methyl-8-quinolinato) 4-phenylphenolate (abbreviated to Balq), a triazole derivative, and a phenanthroline derivative such as 2,9-dimethyl-4,7-diphenyl-1,10-phenanthroline (abbreviated as BCP).

The thickness of the hole blocking layer is preferably 1 nm to 500 nm, more preferably 5 nm to 200 nm, and even more preferably 10 nm to 100 nm.

The hole blocking layer may have a single layer structure composed of one kind or two or more kinds of the above-described materials, or may have a multilayer structure composed of a plurality of layers of the same or different compositions.

### -Electron blocking layer-

The electron blocking layer is a layer having a function of preventing the electron transported from the cathode side to the light emitting layer from being discharged to the anode side. In the present invention, the electron blocking layer may be installed as an organic layer that is adjacent to the light emitting layer in the anode side.

Examples of the organic compound constituting the electron blocking layer may include, for example, those exemplified as the hole transporting material.

The thickness of the electron blocking layer is preferably 1 nm to 500 nm, more preferably 5 nm to 200 nm, and even more preferably 10 nm to 100 nm.

The electron blocking layer may have a single layer structure composed of one kind or two or more kinds of the above-described materials, or may have a multilayer structure composed of a plurality of layers having the same or different compositions.

### <Protective layer>

In the present invention, the entire organic EL device may be protected by a protective layer.

With respect to the protective layer, the subject matters described in [0169] to [0170] of Japanese Patent Application Laid-Open No. 2008-270736 may be applied to the present invention.

### <Substrate>

It is preferred that the substrate which is used in the present invention is a substrate which does not scatter or decay light generated from the organic layer.

### <Anode>

Typically, the anode may have a function as an electrode for supplying a hole into the organic layer. The anode is not particularly limited with respect to a shape, a structure, a size and the like, and may be appropriately selected among known electrode materials depending upon a use or purpose of the luminescence device. As described above, the anode is usually provided as a transparent anode.

### <Cathode>

Typically, the cathode may have a function as an electrode for injecting an electron into the organic layer. The cathode is not particularly limited with respect to a shape, a structure, a size and the like, and may be appropriately selected among known electrode materials depending upon a use or purpose of the luminescence device.

With respect to the substrate, the anode, and the cathode, the subject matters described in paragraph Nos. [0070] to [0089] of Japanese Patent Application Laid-Open No. 2008-270736 may be applied to the present invention.

### <Sealing Container>

In the element of the present invention, the whole device may be sealed using a sealing container.

With respect to the sealing container, the subject matters described in paragraph No. [0171] of Japanese Patent Application Laid-Open No. 2008-270736 may be applied to the present invention.

### (Driving)

In the organic electroluminescence device of the present invention, light emission may be obtained by applying a DC voltage (typically 2 volts to 15 volts) (may include an alternating current component if necessary) or a direct current between the anode and the cathode.

With respect to the driving method of the organic electroluminescence device of the present invention, driving methods described in Japanese Patent Application Laid-Open No. Hei 2-148687, Japanese Patent Application Laid-Open No. Hei 6-301355, Japanese Patent Application Laid-Open No. Hei 5-29080, Japanese Patent Application Laid-Open No. Hei 7-134558, Japanese Patent Application Laid-Open No. Hei 8-234685, and Japanese Patent Application Laid-Open No. Hei 8-241047, and Japanese Patent No. 2784615, US Patent No. 5,828,429, and US Patent No. 6,023,308, and the like may be applied.

The external quantum efficiency of the organic electroluminescence device of the present invention is preferably 5% or more, and more preferably 7% or more. As values of external quantum efficiency, a maximum value of external quantum efficiency, or a value of external quantum efficiency near 100 to 300 cd/m² when driving the device at 20°C may be used when driving the device at 20°C.

The internal quantum efficiency of the organic electroluminescence device of the present invention is preferably 30% or more, more preferably 50% or more, and even more preferably 70% or more. The internal quantum efficiency of the device is calculated by dividing the external quantum efficiency by the light-extraction efficiency. Although typical organic EL devices have a light-extraction efficiency of about 20%, it is possible to achieve a light-extraction efficiency of 20% or more by studying the shape of the substrate, the shape of the electrode, the film thickness of the organic layer, the film thickness of the inorganic layer, the refractive index of the organic layer, the refractive index of the inorganic layer, and the like.

The organic electroluminescence device of the present invention has a ultra-high power light emission wavelength (maximum intensity wavelength of the emission spectrum) of preferably 350 nm or more and 700 nm or less, more preferably 350 nm or more and 600 nm or less, even more preferably 400 nm or more and 520 nm or less, and particularly preferably 400 nm or more and 465 nm or less.

### (Use of the luminescence device of the present invention)

The luminescence device of the present invention may be appropriately used for a display device, a display, a backlight, an electronic picture, an illumination light source, a recording light source, an exposure light source, a reading light source, a signal, a signboard, an interior or an optical communication. Particularly, the luminescence device is preferably used in a device that is driven in a field where luminance intensity is high, such as an illumination apparatus and a display apparatus.

Next, with reference to FIG. 2, a light emission apparatus of the present invention will be described.

FIG. 2 is a cross-sectional view schematically illustrating an example of the light emission apparatus of the present invention.

A light emission apparatus 20 of FIG. 2 is composed of a transparent substrate (substrate) 2, an organic electroluminescence device 10, and a sealing vessel 16.

The organic electroluminescence device 10 is configured by sequentially laminating an anode (first electrode) 3, an organic layer 11, a cathode (second electrode) 9 on the substrate 2. Further, on the cathode 9, a protective layer 12 is laminated, and on the protective layer 12, the sealing vessel 16 is further installed through an adhesive layer 14. In addition, a portion of each of the electrodes 3 and 9, a partition and an insulating layer are omitted.

Herein, as the adhesive layer 14, a photocurable adhesive or a thermosetting adhesive such as an epoxy resin may be used, and for example, a thermosetting adhesive sheet may be used.

The use of the light emission apparatus of the present invention is not particularly limited, and for example, in addition to the illumination apparatus, the apparatus may be used as a display apparatus such as a television, a personal computer, a mobile phone and an electronic paper.

### (Illumination apparatus)

Next, with reference to FIG. 3, an illumination apparatus according to the exemplary embodiment of the present invention will be described.

FIG. 3 is a cross-sectional view schematically illustrating an example of the illumination apparatus according to the exemplary embodiment of the present invention.

The illumination apparatus 40 according to the exemplary embodiment of the present invention, as shown in FIG. 3, is provided with the organic EL device 10 and a light scattering member 30. More specifically, the illumination apparatus 40 is configured so that the substrate 2 of the organic EL device 10 comes in contact with the light scattering member 30.

The light scattering member 30 is not particularly limited so long as the member can scatter light, but in FIG. 3, the member is formed of a member in which microparticles 32 are dispersed in a transparent substrate 31. The transparent substrate 31 may appropriately be, for example, a glass substrate. The microparticles 32 may appropriately be transparent resin microparticles. Publicly known glass substrate and transparent resin microparticles may be used. If light emitted from the organic electroluminescence device 10 is incident on a light incident surface 30A of the scattering member 30, the illumination apparatus 40 scatters the incident light by the light scattering member 30 and reflect the scattered light from a light reflecting surface 30B as illumination light.

### Example

Hereinafter, the present invention will be described in detail with reference to Examples, but the present invention is not limited thereto.

### <Example 1>

### [Preparation of Device 1-1]

A glass substrate having an ITO film having a thickness of 0.5 mm and each side of 2.5 cm in square (manufactured by Geomatec Co., Ltd., and surface resistance 10Ω/□) was put into a washing container, ultrasonically washed in 2-propanol, followed by UV-ozone treatment for 30 min. The following organic layers were sequentially vapor deposited on this transparent anode (ITO film) by means of vacuum deposition.
First layer: 2-TNATA and F₄-TCNQ (mass ratio 99.7:0.3): film thickness 120 nm
Second layer: αNPD(N,N'-di-α-naphthyl-N,N'-diphenyl)-benzidine): film thickness 7 nm
Third layer: C-1: film thickness 3 nm
Fourth layer: H-1 and 9-16 (mass ratio 85:15): film thickness 30 nm
Fifth layer: Exemplary compound 4: film thickness 29 nm
Sixth layer: BCP: film thickness 1 nm

0.1 nm-thick lithium fluoride and a 100 nm-thick metal aluminum were vapor deposited in this order thereon, thereby forming a cathode.

This laminate was placed in a glove box substituted with a nitrogen gas without being in contact with the atmosphere, and sealed using a glass-made sealing tube and a UV-curable adhesive (XNR5516HV, manufactured by Nagase-CHIBA Ltd.) to obtain an organic electroluminescence device 1-1 of the present invention.

### [Preparation of Other Devices]

Devices 1-2 to 1-38 in Working Examples and comparative devices 1-1 to 1-22 in Comparative Example of the present invention were obtained in the same manner as in Preparation of Device 1-1, except that constituting materials of the third, fourth, and fifth layers were changed into compositions shown in the following Table 1-1 and Table 1-2.

As a result of emitting light from these devices, light emission derived from each of the light emitting materials was obtained. Maximum light emission wavelengths were also shown in Table 1-1 and Table 1-2.

### [Table 1]

**Table 1-1**

| Device No. | Fourth layer | Fifth layer | Ultra-high power light emission wavelength |
|---|---|---|---|
| Device 1-1 of example | | Exemplary compound 4 | |
| Device 1-2 of example | | Exemplary compound 112 | |
| Device 1-3 of example | | Exemplary compound 45 | |
| Device 1-4 of example | | Exemplary compound 77 | |
| Device 1-5 of example | | Exemplary compound 75 | |
| Device 1-6 of example | | Exemplary compound 108 | |
| Device 1-7 of example | | Exemplary compound 109 | |
| Device 1-8 of example | H-1 / 9-16 | Exemplary compound 110 | 466nm |
| Device 1-9 of example | Mass ratio (85:15) | Exemplary compound 113 | |
| Device 1-10 of example | | Exemplary compound 117 | |
| Device 1-11 of example | | Exemplary compound 120 | |
| Device 1-12 of example | | Exemplary compound 122 | |
| Comparative Device 1-1 | | F-1 | |
| Comparative Device 1-2 | | F-2 | |
| Comparative Device 1-3 | | F-3 | |
| Device 1-13 of example | H-1 / 3-2 Mass ratio (85:15) | Exemplary compound 4 | |
| Device 1-14 of example | | Exemplary compound 112 | 468nm |
| Device 1-15 of example | | Exemplary compound 77 | |
| Comparative Device 1-4 | | F-1 | |
| Device 1-16 of example | H-1 / 8-4 Mass ratio (85:15) | Exemplary compound 4 | 456nm |
| Device 1-17 of example | | Exemplary compound 45 | |
| Comparative Device 1-5 | | F-2 | |
| Device 1-18 of example | H-1 / 9-17 Mass ratio (85:15) | Exemplary compound 4 | 467nm |
| Device 1-19 of example | | Exemplary compound 75 | |
| Comparative Device 1-6 | | F-3 | |
| Device 1-20 of example | H-1 / 2-3 Mass ratio (85:15) | Exemplary compound 4 | 505nm |
| Device 1-21 of example | | Exemplary compound 112 | |
| Device 1-22 of example | | Exemplary compound 108 | |
| Comparative Device 1-7 | | F-1 | |
| Comparative Device 1-8 | H-1 / B-1 Mass ratio (85:15) | Exemplary compound 4 | 470nm |
| Comparative Device 1-9 | | Exemplary compound 112 | |
| Comparative Device 1-10 | | Exemplary compound 45 | |
| Comparative Device 1-11 | | Exemplary compound 77 | |
| Comparative Device 1-12 | | Exemplary compound 75 | |
| Comparative Device 1-13 | | Exemplary compound 108 | |
| Comparative Device 1-14 | | F-1 | |
| Comparative Device 1-15 | | F-2 | |
| Comparative Device 1-16 | | F-3 | |
| Comparative Device 1-17 | H-1 / B-2 Mass ratio (85:15) | Exemplary compound 4 | 513nm |
| Comparative Device 1-18 | | Exemplary compound 45 | |
| Comparative Device 1-19 | | F-2 | |
| Comparative Device 1-20 | H-1 / B-3 Mass ratio (85:15) | Exemplary compound 4 | 511nm |
| Comparative Device 1-21 | | Exemplary compound 75 | |
| Comparative Device 1-22 | | F-3 | |
| Comparative Device 1-23 | H-1 / 9-16/ (1-4) Mass ratio (75 : 10 : 15) | Exemplary compound 4 | 466nm |

### [Table 2]

**Table 1-2**

| Device No. | Third layer | Fourth layer | Fifth layer | Ultra-high power light emission wavelength |
|---|---|---|---|---|
| Device 1-24 of example | C-4 | H-7 / 2-1 Mass ratio (85:15) | Exemplary compound 4 | 511nm |
| Device 1-25 of example | C-2 | H-7 / 2-2 Mass ratio (85:15) | Exemplary compound 112 | 492nm |
| Device 1-26 of example | C-2 | H-4 / 2-6 Mass ratio (85:15) | Exemplary compound 45 | 528nm |
| Device 1-27 of example | C-2 | H-6 / 2-8 Mass ratio (85:15) | Exemplary compound 4 | 501nm |
| Device 1-28 of example | C-3 | H-2 / 3-3 Mass ratio (80:20) | Exemplary compound 45 | 466nm |
| Device 1-29 of example | C-2 | H-4 / 3-3 Mass ratio (85:15) | Exemplary compound 4 | 466nm |
| Device 1-30 of example | C-5 | H-9 / 3-4 Mass ratio (93:7) | Exemplary compound 112 | 464nm |
| Device 1-31 of example | C-2 | H-7 / 3-4 Mass ratio (90:10) | Exemplary compound 45 | 464nm |
| Device 1-32 of example | C-2 | H-8 / 3-5 Mass ratio (85:15) | Exemplary compound 4 | 531nm |
| Device 1-33 of example | C-5 | H-3 / 3-5 Mass ratio (85:15) | Exemplary compound 112 | 531nm |
| Device 1-34 of example Device 1-34 | C-6 | H-3 / 9-6 Mass ratio (85:15) | Exemplary compound 4 | 483nm |
| Device 1-35 of example | C-2 | H-5 / 9-19 Mass ratio (85:15) | Exemplary compound 45 | 538nm |
| Device 1-36 of example | C-2 | H-1 / 2-3 / FR-1 Mass ratio (90:9:1) | Exemplary compound 4 | 608nm |
| Device 1-37 of example | C-3 | H-7 / 9-19 / FR-2 Mass ratio (85:14:1) | Exemplary compound 4 | 603nm |
| Device 1-38 of example | C-2 | H-2 / 3-5 / FR-3 Mass ratio (90 : 9 : 1) | Exemplary compound 45 | 631nm |

Structures of the compounds used in the Working Examples and the Comparative Example are shown bellow.

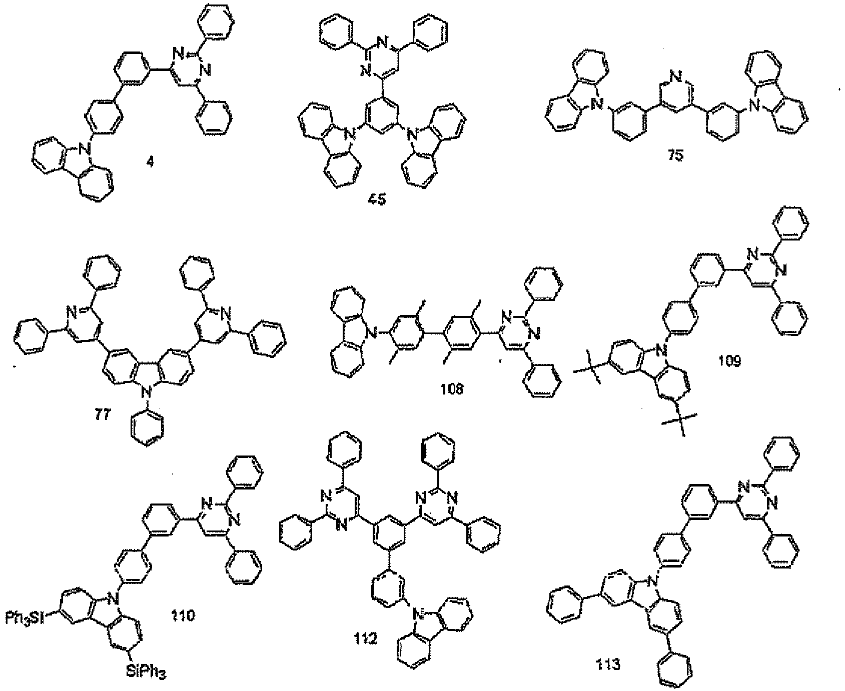

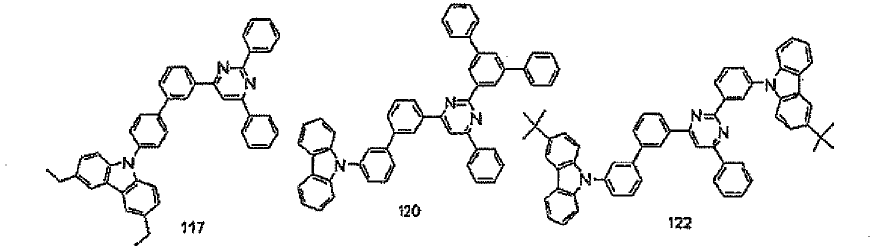

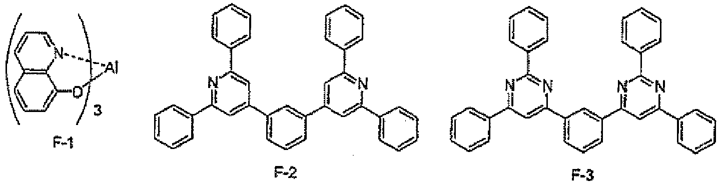

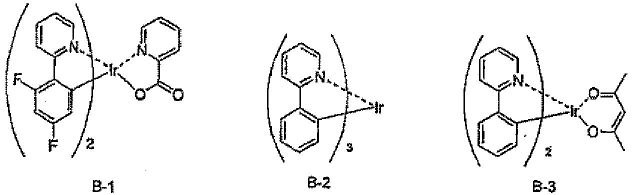

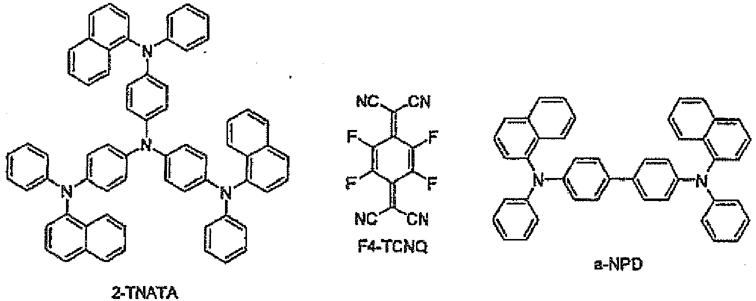

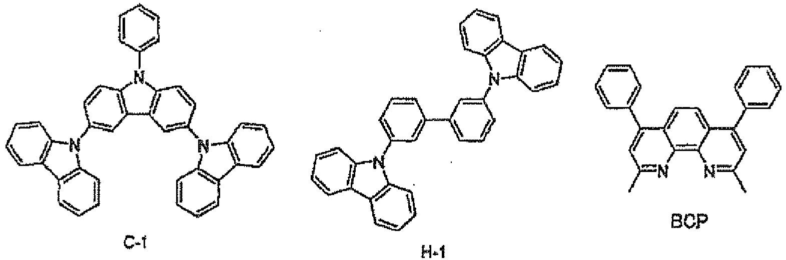

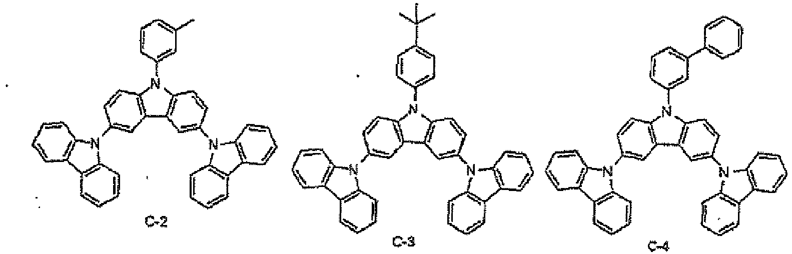

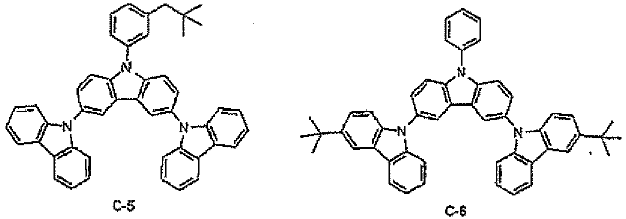

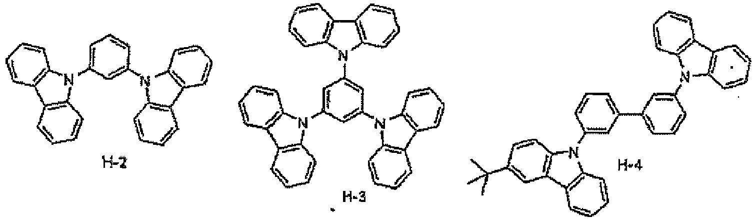

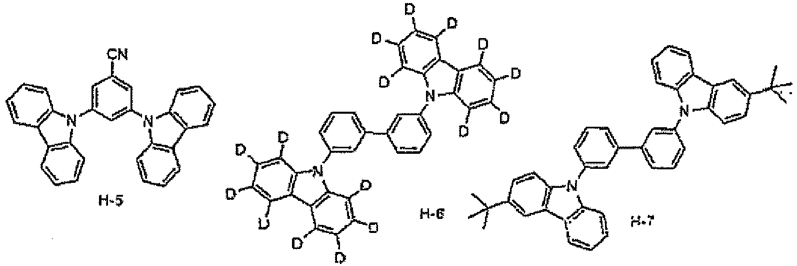

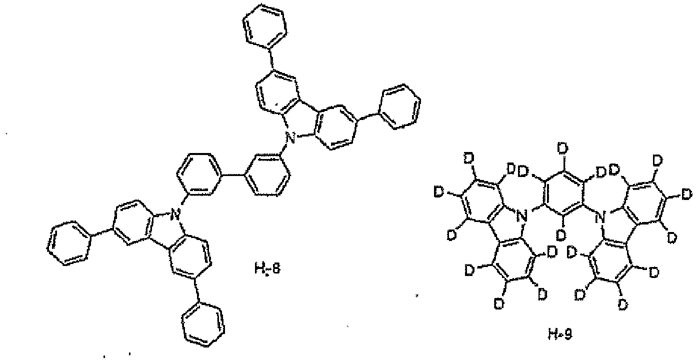

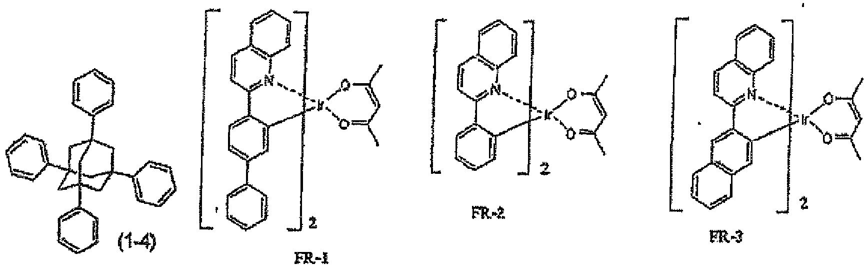

The compound represented by Formula (1), which was used in the Examples, was synthesized with reference to the pamphlet of International Publication No. WO03/080760, the pamphlet of International Publication No. WO03/078541, the pamphlet of International Publication No. WO05/085387, the pamphlet of International Publication No. WO05/022962, and the like. For example, exemplary compound 4 may be synthesized using m-bromobenzoaldehyde as a starting raw material by a method described in [0074] to [0075] of the pamphlet of International Publication No. WO05/085387 (page 45, line 11 to page 46, line 18). Further, exemplary compound 45 may be synthesized using 3,5-dibromobenzoaldehyde as a starting raw material by a method described on page 46, line 9 to page 46, line 12 of the pamphlet of International Publication No. WO03/080760. Further, exemplary compound 77 may be synthesized using N-phenylcarbazole as a starting raw material by a method described on page 137, line 10 to page 139, line 9 of the pamphlet of International Publication No. WO05/022962.

The compound represented by Formula (C-1), which was used in the Examples, was synthesized by methods described in Japanese Patent Application Laid-Open No. 2005-310733, Japanese Patent Application Laid-Open No. 2006-93542, Japanese Patent Application Laid-Open No. 2007-19462, Journal of Organic Chemistry 53, 786 (1988), and the like. For example, compound 8-4 may be synthesized by a method described on page 52, line 34 to page 53, line 23 of Japanese Patent Application Laid-Open No. 2007-19462 using the following compound BBPy as a starting raw material which may be obtained by a method described on page 789, line 53 of the left-hand column to line 7 of the right-hand column, a method described on page 790, lines 18 to 38 of the left-hand column, a method described on page 790, lines 19 to 30 of the right-hand column in Journal of Organic Chemistry 53, 786 (1988), G. R. Newkome et al., and a combination thereof.

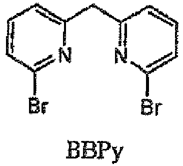

In addition, all of the organic materials used in the Examples in the present specification were subjected to sublimation purification and used.

### (Evaluation of Performance of Organic Electroluminescence Devices)

The performance of each of the obtained devices was evaluated by measuring the external quantum efficiency, driving voltage, driving durability and change in chromaticity while driving at a high temperature. Also, various measurements were performed as follows.

### (a) External Quantum Efficiency

DC was applied to the respective devices by using a Source Measure Unit 2400 manufactured by Toyo Technica Corporation to enable the devices to emit light. The luminance intensity was measured by using a luminance meter BM-8 manufactured by TOPCON CORPORATION. Emission spectrum and emission wavelengths were measured by using a spectrum analyzer PMA-11 manufactured by Hamamatsu Photonics K.K. On the basis of the obtained numerical values, the external quantum efficiency in the vicinity of the luminance intensity of about 1000 cd/m² was calculated by a luminance intensity conversion method.

### (b) Driving Voltage

Direct current voltage was applied to the respective devices such that the luminance intensity was 360 cd/m², thereby enabling the devices to emit light. The voltage applied at that time was defined as an index for evaluating driving voltage.

### (c) Driving Durability

DC voltage was applied to the respective devices such that luminance intensity was 1000 cd/m², and then the time until the luminance intensity decreased to 500 cd/m² was measured. The half decay time of luminance intensity of each device was defined as an index for evaluating driving durability.

### (d) Change in chromaticity when driving at a high temperature

The differences between x values and between y values (Δx and Δy) of chromaticity obtained between when DC voltage was applied to the respective devices such that the luminance intensity was 1000 cd/m² to enable the devices to emit light, and when DC voltage was applied to respective devices such that the luminance intensity was 1000 cd/m² in a thermostatic bath at 80°C to enable the devices to emit light continuously and the luminance intensity became 500 cd/m², were used as indices for a change in chromaticity when driving at a high temperature.

Evaluation results of the respective devices are shown in Table 2.

### [Table 3]

**Table 2**

| Device No. | External quantum efficiency (%) | Driving voltage (V) | Durability | Change in chromaticity |
|---|---|---|---|---|
| Device 1-1 of Example | 11 | 59 | 1 | (<0.005, <0.005) |
| Device 1-2 of Example | 12 | 6.1 | 0.8 | (<0.005, <0.005) |
| Device 1-3 of Example | 9 | 6.4 | 0.9 | (<0.005, <0.005) |
| Device 1-4 of Example | 9 | 6.7 | 0.8 | (<0.005, <0.005) |
| Device 1-5 of Example | 9 | 7.1 | 0.7 | (<0.005, <0.005) |
| Device 1-6 of Example | 10 | 6.0 | 0.5 | (<0.005, <0.005) |
| Device 1-7 of Example | 9 | 6.2 | 0.5 | (<0.005, <0.005) |
| Device 1-8 of Example | 9 | 6.0 | 0.7 | (<0.005, <0.005) |
| Device 1-9 of Example | 10 | 6.0 | 0.6 | (<0.005, <0.005) |
| Device 1-10 of Example | 9 | 5.9 | 0.6 | (<0.005, <0.005) |
| Device 1-11 of Example | 11 | 6.0 | 0.7 | (<0.005, <0.005) |
| Device 1-12 of Example | 9 | 6.2 | 0.7 | (<0.005, <0.005) |
| Comparative Device 1-1 | 7 | 8.0 | 0.7 | (0.01, 0.02) |
| Comparative Device 1-2 | 8 | 8.3 | <0.1 | (0.02, >0.03) |
| Comparative Device 1-3 | 6 | 7.7 | <0.1 | (0.02, >0.03) |
| Device 1-13 of Example | 12 | 5.5 | 0.9 | (<0.005, <0.005) |
| Device 1-14 of Example | 12 | 5.6 | 0.8 | (<0.005, <0.005) |
| Device 1-15 of Example | 8 | 6.1 | 0.7 | (<0.005, <0.005) |
| Comparative Device 1-4 | 7 | 7.6 | 0.6 | (0.01, 0.02) |
| Device 1-16 of Example | 9 | 6.6 | 0.7 | (<0.005, <0.005) |
| Device 1-17 of Example | 7 | 7.2 | 0.5 | (<0.005, <0.005) |
| Comparative Device 1-5 | 5 | 8.1 | <0.1 | (0.02, 0.03) |
| Device 1-18 of Example | 7 | 6.4 | 0.8 | (<0.005, <0.005) |
| Device 1-19 of Example | 5 | 7.0 | 0.4 | (<0.005, <0.005) |
| Comparative Device 1-6 | 4 | 6.7 | <0.1 | (0.02, 0.03) |
| Device 1-20 of Example | 17 | 4.4 | 57 | (<0.005, <0.005) |
| Device 1-21 of Example | 17 | 4.6 | 50 | (<0.005, <0.005) |
| Device 1-22 of Example | 16 | 4.4 | 45 | (<0.005, <0.005) |
| Comparative Device 1-7 | 13 | 5.7 | 39 | (0.01, 0.02) |
| Comparative Device 1-8 | 5 | 6.5 | 0.3 | (0.01, 0.02) |
| Comparative Device 1-9 | 5 | 6.4 | 0.2 | (0.01, 0.02) |
| Comparative Device 1-10 | 4 | 6.8 | 0.3 | (0.02, 0.03) |
| Comparative Device 1-11 | 4 | 7.0 | <0.1 | (0.02, >0.03) |
| Comparative Device 1-12 | 4 | 7.2 | <0.1 | (0.02, 0.03) |
| Comparative Device 1-13 | 5 | 6.6 | <0.1 | (0.01, >0.03) |
| Comparative Device 1-14 | 4 | 7.8 | 0.1 | (>0.03, >0.03) |
| Comparative Device 1-15 | 3 | 7.6 | <0.1 | (>0.03, >0.03) |
| Comparative Device 1-16 | 3 | 7.3 | <0.1 | (>0.03, >0.03) |
| Comparative Device 1-17 | 12 | 5.1 | 11 | (0.02, >0.03) |
| Comparative Device 1-18 | 10 | 5.4 | 9 | (0.02, >0.03) |
| Comparative Device 1-19 | 8 | 6.3 | 2 | (>0.03, >0.03) |
| Comparative Device 1-20 | 12 | 5.2 | 10 | (0.02, >0.03) |
| Comparative Device 1-21 | 9 | 5.5 | 8 | (0.02, >0.03) |
| Comparative Device 1-22 | 7 | 5.7 | 0.4 | (>0.03, >0.03) |
| Device 1-23 of Example | 11 | 5.9 | 1.2 | (<0.005, <0.005) |
| Device 1-24 of Example | 14 | 5.2 | 22 | (<0.005, <0.005) |
| Device 1-25 of Example | 12 | 5.7 | 1.5 | (<0.005, <0.005) |
| Device 1-26 of Example | 15 | 5.0 | 35 | (<0.005, <0.005) |
| Device 1-27 of Example | 12 | 5.5 | 17 | (<0.005, <0.005) |
| Device 1-28 of Example | 12 | 5.1 | 0.8 | (<0.005, <0.005) |
| Device 1-29 of Example | 12 | 5.3 | 0.9 | (<0.005, <0.005) |
| Device 1-30 of Example | 8 | 6.0 | 0.6 | (<0.005, <0.005) |
| Device 1-31 of Example | 7 | 6.0 | 0.5 | (<0.005, <0.005) |
| Device 1-32 of Example | 16 | 4.7 | 45 | (<0.005, <0.005) |
| Device 1-33 of Example | 16 | 4.6 | 40 | (<0.005, <0.005) |
| Device 1-34 of Example | 10 | 5.6 | 0.6 | (<0.005, <0.005) |
| Device 1-35 of Example | 17 | 4.5 | 37 | (<0.005, <0.005) |
| Device 1-36 of Example | 16 | 5.2 | 120 | (<0.005, <0.005) |
| Device 1-37 of Example | 16 | 5.4 | 107 | (<0.005, <0.005) |
| Device 1-38 of Example | 16 | 5.2 | 135 | (<0.005, <0.005) |

From the results in Table 2, it is determined that the device of the present invention has excellent light emission efficiency and durability due to low driving voltage, and shows a small change in chromaticity when driving at a high temperature, compared to devices having the same light emission wavelength in the Comparative Example.

Further, it is determined by comparison of the devices in Comparative Devices 1-8 to 1-22 with the devices in the Examples that effects of suppressing the change in chromaticity when driving at a high temperature are significant when the compound represented by Formula (1) and the phosphorescence emitting material represented by Formula (C-1) as a light emitting material are used. In addition, as shown from the results of devices 1-24 to 1-38 in the Examples, it is determined that devices having high device characteristics and a small change in chromaticity at a high temperature may be obtained by a combination of various materials described in the specification.

### <Example 2>

### [Preparation of Device 2-1]

Devices 2-1 to 2-12 in the Working Examples and comparative devices 2-1 to 2-4 in the Comparative Example of the present invention were obtained in the same manner as in Preparation of Device -1-1, except that components of the third, fourth, and fifth layers were changed into compositions shown in the following Table 3.

As a result of emitting light from these devices, light emission derived from each of the light emitting materials was obtained. Maximum light emission wavelengths are also shown in Table 3.

### [Table 4]

**Table 3**

| Device No. | Third layer | Fourth layer | Fifth layer | Ultra-high power light emission wavelength |
|---|---|---|---|---|
| Device 2-1 of Example | C-1 | Exemplary compound 4/9-16 mass ratio (90:14) | F-1 | 466nm |
| Device 2-2 of Example | C-1 | Exemplary compound 4/9-16 mass ratio (93:7) | F-1 | 466nm |
| Comparative Device 2-1 | C-1 | F-1 / 9-16 mass ratio (90:10) | F-1 | - |
| Device 2-3 of Example | C-1 | Exemplary compound 45 / 2-3 mass ratio (85:15) | F-1 | 505nm |
| Device 2-4 of Example | C-1 | Exemplary compound 45/2-3 mass ratio (93:7) | F-1 | 505nm |
| Comparative Device 2-2 | C-1 | F-3 / 2-3 mass ratio (85:15) | F-1 | - |
| Device 2-5 of Example | C-1 | Exemplary compound 109 /9-16 mass ratio (90:10) | F-1 | 466nm |
| Device 2-6 of Example | C-1 | Exemplary compound 109/9-16 mass ratio (93:7) | F-1 | 466nm |
| Comparative Device 2-3 | C-1 | F-1 / 9-16 mass ratio (90:10) | F-1 | - |
| Device 2-7 of Example | C-1 | Exemplary compound 120/9-2 mass ratio (90:10) | F-1 | 479nm |
| Device 2-8 of Example | C-1 | Exemplary compound 120/9-2 mass ratio (93:7) | F-1 | 479nm |
| Comparative Device 2-4 | C-1 | F-1 / 9-2 mass ratio (90:10) | F-1 | - |
| Device 2-9 of Example | C-2 | Exemplary compound 45/3-3 mass ratio (85:15) | F-1 | 466nm |
| Device 2-10 of Example | C-2 | H-2 / Exemplary compound 4 / 3-5 mass ratio (70:15:15) | F-1 | 531nm |
| Device 2-11 of Example | C-5 | H-8 / Exemplary compound 45 / 9-19 mass ratio (50:35:15) | F-1 | 538nm |
| Device 2-12 of Example | C-6 | H-4 / Exemplary compound 112 / 2-6 mass ratio (50:35:15) | F-1 | 528nm |

The evaluation of performance of these devices was performed in the same manner as in Example 1. The evaluation results are shown in Table 4.

### [Table 5]

**Table 4**

| Device No. | External quantum efficiency (%) | Driving voltage (V) | Durability | Change in chromaticity |
|---|---|---|---|---|
| Device 2-1 of Example | 12 | 5.5 | 0.8 | (<0.005, <0.005) |
| Device 2-2 of Example | 10 | 6.0 | 0.6 | (<0.005, <0.005) |
| Comparative Device 2-1 | No light emission | - | - | (-, -) |
| Device 2-3 of Example | 18 | 4.0 | 50 | (<0.005, <0.005) |
| Device 2-4 of Example | 15 | 4.9 | 44 | (<0.005, <0.005) |
| Comparative Device 2-2 | No light emission | - | - | (-, -) |
| Device 2-5 of Example | 10 | 5.9 | 0.7 | (<0.005, <0.005) |
| Device 2-6 of Example | 9 | 6.2 | 0.8 | (<0.005, <0.005) |
| Comparative Device 2-3 | No light emission | - | - | (-, -) |
| Device 2-7 of Example | 8 | 6.0 | 0.4 | (<0.005, <0.005) |
| Device 2-8 of Example | 8 | 6.1 | 0.3 | (<0.005, <0.005) |
| Comparative Device 2-4 | No light emission | - | - | (-, -) |
| Device 2-9 of Example | 12 | 5.5 | 0.5 | (<0.005, <0.005) |
| Device 2-10 of Example | 18 | 4.5 | 45 | (<0.005, <0.005) |
| Device 2-11 of Example | 17 | 4.7 | 38 | (<0,005, <0.005) |
| Device 2-12 of Example | 17 | 4.7 | 42 | (0.005, <0.005) |

From the results in Table 4, it is understood that the device of the present invention, in which the compound represented by Formula (1) and the phosphorescence emitting material represented by Formula (C-1) are used in the light emitting layer, has excellent light emission efficiency and durability due to low driving voltage, and a very small change in chromaticity when driving at a high temperature, compared to the devices having the same light emission wavelength in the Comparative Example. Further, as shown from the results of devices 2-9 to 2-12 in the Examples, it is understood that the device of the present invention, in which the compound represented by Formula (1) and the phosphorescence emitting material represented by Formula (C-1) are used in the light emitting layer, may have high device characteristics and a small change in chromaticity at high temperature when obtained by a combination of various materials described in the specification.

The device of the present invention has a small change in chromaticity and has high light emission efficiency or excellent durability even when the device is used in a high temperature environment, such as in-vehicle use, and may be advantageously used in light emission apparatuses, display apparatuses and illumination apparatuses.

### Industrial Applicability

The organic electroluminescence device of the present invention may suppress electric power consumption by driving at low voltage, and has high luminous efficiency and excellent durability. Moreover, the organic electroluminescence device of the present invention has a small change in chromaticity when driving at a high temperature, and is also appropriate for the use of which the driving durability is required in a high temperature environment, such as in-vehicle use.

### Explanation of Reference Numerals and Symbols

- 2:: Substrate
- 3:: Anode
- 4:: Hole injection layer
- 5:: Hole transporting tayer
- 6:: Light emitting layer
- 7:: Hole blocking layer
- 8:: Electron transporting layer
- 9:: Cathode
- 10:: Organic electroluminescence device (organic EL device)
- 11:: Organic layer
- 12:: Protective layer
- 14:: Adhesive layer
- 16:: Sealing vessel
- 20:: Light emission apparatus
- 30:: Light scattering member
- 30A:: Light incident surface
- 30B:: Light reflecting surface
- 31:: Transparent substrate
- 32:: Microparticule
- 40:: Illumination apparatus

## Claims

1. A device, which is an organic electroluminescence device (10) comprising,
- a substrate (2), and provided thereon
- a pair of electrodes (3, 9), and
- disposed between the electrodes, organic layers (11) including at least
(i) a light emitting layer (6) containing a phosphorescent emitting material of formula (C-1): 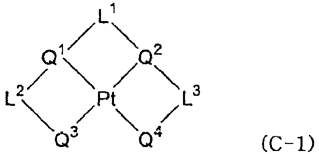 wherein
Q¹-Q⁴ each independently are a ligand which is coordinated to Pt, and
L¹-L³ each independently are a single bond or a divalent linking group, and whereby Q₃ and Q₄ are not linked; and
(ii) contained in a layer directly adjacent to the light emitting layer, a compound of formula (1)
(Cz)p-L- (A)q (1)
wherein
Cz is an optionally substituted arylcarbazolyl group or a carbazolylaryl group,
L is a single bond or an optionally substituted arylene, cycloalkylene or heteroaryl group,
A is an optionally substituted N-containing six-membered heteroaromatic ring, and
p, q each independently are an integer of 1-6.

2. The device of claim 1, wherein the compound of formula (1) is a compound of formula (2): 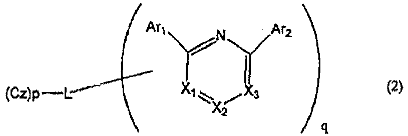 wherein
Cz is an optionally substituted arylcarbazolyl or carbazolylaryl group,
L is a single bond or an optionally substituted arylene, cycloalkylene or heteroaryl group, and is linked to a C-atom of Ar₁, Ar₂, X₁, X₂ or X₃,
Ar₁, Ar₂ each independently are an optionally substituted aryl or heteroaryl group,
X₁-X₃ each independently are N, or C to which a hydrogen or a substituent is bound, and
p, q each independently are an integer of 1-6.

3. The device of claim 2, wherein in formula (2) at least one of X₁ and X₃ is N.

4. The device of claim 2 or 3, wherein in formula (2), Ar₁ and Ar₂ each independently are phenyl or biphenyl.

5. The device of any of claims 1 to 4, wherein the aryl group in Cz is substituted at the 9-position of the carbazole moiety.

6. The device of any of claims 1 to 5, wherein in formula (1) Cz is a carbazolylaryl group.

7. The device of any of claims 1 to 6, wherein the compound of formula (1) is a compound of formula (3): 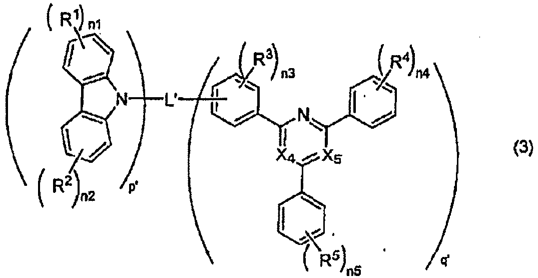 wherein
one of X₄ and X₅ is N and the other one is C to which a hydrogen or a substituent is bound,
L' is a single bond or an optionally substituted arylene, cycloalkylene or heteroaryl group,
R¹-R⁵ each independently are a substituent,
n1-n5 each independently are an integer of 0-5, and
p', q' each independently are an integer of 1-4.

8. The device of any of claims 1 to 7, wherein the compound of formula (1) is composed only of C, H and N.

9. The device of any of claims 1 to 8, wherein the compound of formula (1) has a molecular weight of 450-800.

10. The device of any of claims 1 to 9, wherein the compound of formula (1) has a lowest triplet excited state (T₁) energy of 2.61-3.51 eV in a film state.

11. The device of any of claims 1 to 10, wherein the compound of formula (1) has a glass transition temperature Tg of 80-400°C.

12. The device of claim 1, wherein the phosphorescence emitting material of formula (C-1) is represented by formula (C-2): 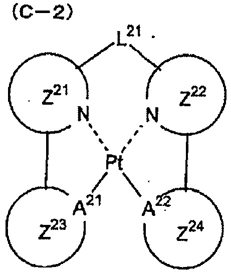 wherein
L²¹ is a single bond or a divalent linking group,
A²¹, A²² each independently are C or N,
Z²¹, Z²² each independently are a N-containing heteroaromatic ring, and
Z²³, Z²⁴ each independently are a benzene ring or an heteroaromatic ring.

13. The device of any of claims 1 to 12, wherein the maximum light emission wavelength is 400-465 nm.

14. The device of any of claims 1 to 13, wherein the light emitting material is contained in the light emitting layer in an amount of 1-30 wt.-%.

15. The device of any of claims 1 to 14, comprising, as an organic layer, a hole injection layer or a hole transporting layer containing an electron-accepting dopant.

16. A composition, comprising the compound of formula (1) and the phosphorescence emitting material of formula (C-1) as defined in claim 1.

17. The composition of claim 16, further comprising a compound of formula (PQ-1): 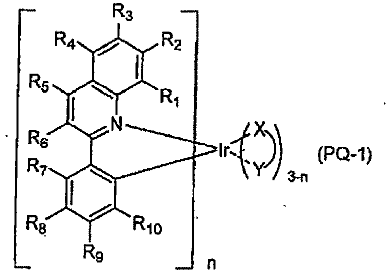 wherein
R₁-R₁₀ each are H or a substituent, and the substituents may be bonded to each other to form a ring,
X-Y is a mono-anionic bidentate ligand, and
n is an integer of 1-3.

18. An apparatus selected from a light emission apparatus, a display apparatus and an illumination apparatus, which comprises the device of any of claims 1 to 15.

## Patentansprüche

1. Eine Vorrichtung, welche eine organische elektroluminezente Vorrichtung (10) ist, umfassend:
- ein Substrat (2), und darauf angeordnet
- ein Paar von Elektroden (3, 9), und
- angeordnet zwischen den Elektroden, organische Schichten (11) umfassend mindestens
(i) eine lichtemittierende Schicht (6) enthaltend ein phosphoreszentes emittierendes Material gemäß
Formel (C-1): 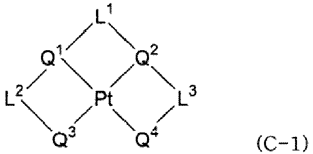 wobei
Q¹-Q⁴ jeweils unabhängig einen Liganden darstellen der an Pt koordiniert ist, und
L¹-L³ jeweils unabhängig für eine Einfachbindung oder eine divalente Verknüpfungsgruppe stehen, und wobei Q₃ und Q₄ nicht verknüpft sind; und
(ii) enthaltend in einer Schicht direkt benachbart zur lichtemittierenden Schicht, eine Verbindung gemäß
Formel (1)
(Cz)ₚ-L-(A)_{q} (1)
wobei
Cz eine gegebenenfalls subsituierte Arylcarbazolylgruppe oder eine Carbazolylarylgruppe ist,
L eine Einfachbindung oder eine gegebenenfalls subsituierte Arylen-, Cycloalkylen- oder Heteroarylgruppe ist,
A ein gegebenenfalls substituierter N-enthaltender sechsgliedriger aromatischer Ring ist, und
p, q jeweils unabhängig für eine ganze Zahl von 1-6 stehen.

2. Die Vorrichtung nach Anspruch 1, wobei die Verbindung gemäß Formel (1) eine Verbindung gemäß Formel (2) ist: 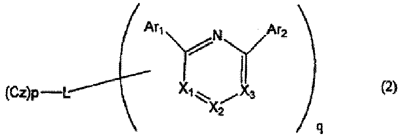 wobei
Cz eine gegebenenfalls subsituierte Arylcarbazolyl- oder Carbazolylarylgruppe ist,
L eine Einfachbindung oder eine gegebenenfalls substituierte Arylen-, Cycloalkylen- oder Heteroarylgruppe ist, und an ein C-Atom von Ar₁, Ar₂, X₁, X₂ oder X₃ gebunden ist,
Ar₁, Ar₂ jeweils unabhängig eine gegebenenfalls subsituierte Aryl- oder Heteroarylgruppe sind,
X₁-X₃ unabhängig N oder C an welches ein Wasserstoff oder ein Substituent gebunden ist sind, und
p, q jeweils unabhängig für eine ganze Zahl von 1-6 stehen.

3. Die Vorrichtung nach Anspruch 2, wobei in Formel (2) mindestens einer von X₁ und X₃ N ist.

4. Die Vorrichtung nach Anspruch 2 oder 3, wobei in Formel (2) Ar₁ und Ar₂ jeweils unabhängig für Phenyl oder Biphenyl stehen.

5. Die Vorrichtung nach einem der Ansprüche 1 bis 4, wobei die Arylgruppe in Cz an der neuen Position der Carbazolgruppe substituiert ist.

6. Die Vorrichtung nach einem der Ansprüche 1 bis 5, wobei in Formel (1) Cz eine Carbazolylarylgruppe ist.

7. Die Vorrichtung nach einem der Ansprüche 1 bis 6, wobei die Verbindung gemäß Formel (1) eine Verbindung gemäß Formel (3) ist: 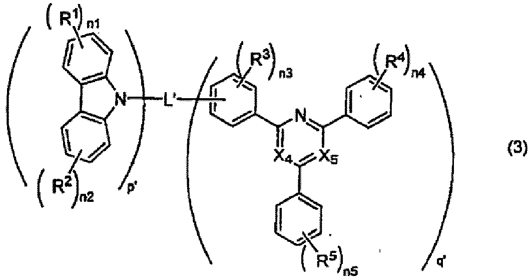 wobei
einer von X₄ und X₅ für N steht und der andere C ist, an welchen ein Wasserstoff oder ein Substituent gebunden ist,
L' ist eine Einfachbindung oder ein gegebenenfalls substituierte Arylen-, Cycloalkylen- oder Heteroarylgruppe,
R¹-R₅ stehen jeweils unabhängig für einen Substituenten,
n1-n5 sind jeweils unabhängig eine ganze Zahl von 0 bis 5, und
p', q' sind jeweils unabhängig eine ganze Zahl von 1 bis 4.

8. Die Vorrichtung nach einem der Ansprüche 1 bis 7, wobei die Verbindung gemäß Formel (1) nur aus C, H und N zusammengesetzt ist.

9. Die Vorrichtung nach einem der Ansprüche 1 bis 8, wobei die Verbindung gemäß Formel (1) ein Molekulargewicht von 450-800 aufweist.

10. Die Vorrichtung nach einem der Ansprüche 1 bis 9, wobei die Verbindung gemäß Formel (1) einen niedrigste angeregte Triplett Zustands (T₁) Energie von 2,61-3,51 eV in einem Filmzustand aufweist.

11. Die Vorrichtung nach einem der Ansprüche 1 bis 10, wobei die Verbindung gemäß Formel (1) eine Glasübergangstemperatur Tg von 80-400°C aufweist.

12. Die Vorrichtung nach Anspruch 1, wobei das Phosphoreszenz emittierende Material gemäß Formel (C-1) wiedergegeben wird durch Formel (C-2): 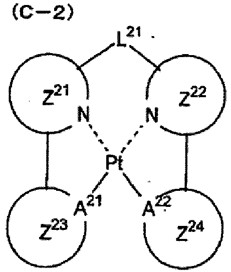 wobei
L²¹ eine Einfachbindung oder eine divalente Verbindungsgruppe ist,
A²¹, A²² sind jeweils unabhängig C oder N,
Z²¹, Z²² sind jeweils unabhängig ein Stickstoffenthaltender heteroaromatischer Ring, und
Z²³, Z²⁴ sind jeweils unabhängig ein Benzolring oder ein heteroaromatischer Ring.

13. Die Vorrichtung nach einem der Ansprüche 1 bis 12, wobei die maximale Lichtemissionswellenlänge 400-465 nm beträgt.

14. Die Vorrichtung nach einem der Ansprüche 1 bis 13, wobei das lichtemittierende Material in der lichtemittierenden Schicht in einer Menge von 1-30 Gew.-% enthalten ist.

15. Die Vorrichtung nach einem der Ansprüche 1 bis 14, umfassend, als eine organische Schicht, eine Lochinjektionsschicht oder eine Lochtransportschicht enthaltend einen Elektronenakzeptordotierstoff.

16. Eine Zusammensetzung umfassend die Verbindung gemäß Formel (1) und das Phosphoreszenz emittierende Material gemäß Formel (C-1) wie in Anspruch 1 definiert.

17. Die Zusammensetzung nach Anspruch 16, ferner umfassend eine Verbindung gemäß Formel (PQ-1): 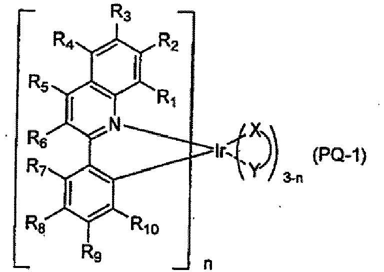 wobei
R₁-R₁₀ jeweils H oder ein Substituent sind, und die Substituenten aneinander gebunden sein können um einen Ring zu bilden,
X-Y ist ein monoanionischer bidentater Ligand, und
N ist eine ganze Zahl 1-3.

18. Ein Gerät ausgewählt aus einem Lichtemissionsgerät, einem Anzeigegerät und einem Beleuchtungsgerät, welches die Vorrichtung nach einem der Ansprüche 1 bis 15 umfasst.

## Revendications

1. Dispositif, qui est un dispositif d'électroluminescence organique (10) comprenant,
- un substrat (2), et disposées sur celui-ci
- une paire d'électrodes (3, 9), et
- disposées entre les électrodes, des couches organiques (11) comprenant au moins
(i) une couche électroluminescente (6) contenant une matière d'émission de phosphorescence de formule (C-1) : 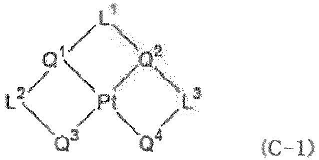 dans laquelle
Q¹-Q⁴ sont chacun indépendamment un ligand qui est coordonné à Pt, et
L¹-L³ sont chacun indépendamment une liaison simple ou un groupe de liaison divalent, et
Q³ et Q⁴ n'étant pas liés ; et
(ii) contenu dans une couche directement adjacente à la couche électroluminescente, un composé de formule (1)
(Cz)ₚ-L-(A)_{q} (1)
dans laquelle
Cz est un groupe arylcarbazolyle facultativement substitué ou un groupe carbazolylaryle,
L est une liaison simple ou un groupe arylène, cycloalkylène ou hétéroaryle facultativement substitué,
A est un cycle hétéroaromatique à six chaînons contenant N facultativement substitué, et
p, q sont chacun indépendamment un entier de 1 à 6.

2. Dispositif de la revendication 1, dans lequel le composé de formule (1) est un composé de formule (2) : 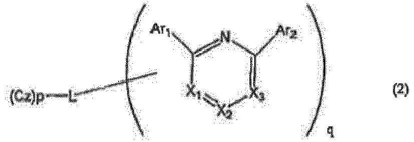 dans laquelle
Cz est un groupe arylcarbazolyle ou carbazolylaryle facultativement substitué,
L est une liaison simple ou un groupe arylène, cycloalkylène ou hétéroaryle facultativement substitué, et est lié à un atome C de Ar₁, Ar₂, X₁, X₂ ou X₃,
Ar₁, Ar₂ sont chacun indépendamment un groupe aryle ou hétéroaryle facultativement substitué,
X₁-X₃ sont chacun indépendamment N ou C auquel un hydrogène ou un substituant est lié, et
p, q sont chacun indépendamment un entier de 1 à 6.

3. Dispositif de la revendication 2, dans lequel, dans la formule (2), au moins l'un parmi X₁ et X₃ est N.

4. Dispositif de la revendication 2 ou 3, dans lequel, dans la formule (2), Ar₁ et Ar₂ sont chacun indépendamment phényle ou biphényle.

5. Dispositif de l'une quelconque des revendications 1 à 4, dans lequel le groupe aryle dans Cz est substitué à la position 9 de la fraction carbazole.

6. Dispositif de l'une quelconque des revendications 1 à 5, dans lequel, dans la formule (1), Cz est un groupe carbazolylaryle.

7. Dispositif de l'une quelconque des revendications 1 à 6, dans lequel le composé de formule (1) est un composé de formule (3) : 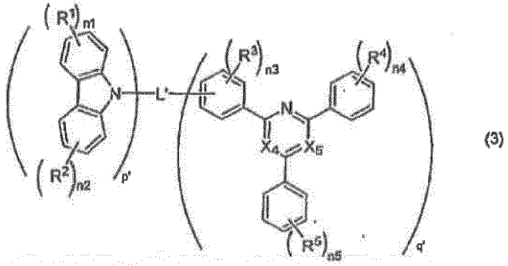 dans laquelle
l'un parmi X₄ et X₅ est N et l'autre est C auquel un hydrogène ou un substituant est lié,
L' est une liaison simple ou un groupe arylène, cycloalkylène ou hétéroaryle facultativement substitué,
R₁-R₅ sont chacun indépendamment un substituant,
n1-n5 sont chacun indépendamment un entier de 0 à 5, et
p', q' sont chacun indépendamment un entier de 1 à 4.

8. Dispositif de l'une quelconque des revendications 1 à 7, dans lequel le composé de formule (1) est uniquement composé de C, H et N.

9. Dispositif de l'une quelconque des revendications 1 à 8, dans lequel le composé de formule (1) a une masse moléculaire de 450 à 800.

10. Dispositif de l'une quelconque des revendications 1 à 9, dans lequel le composé de formule (1) a une énergie d'état triplet excité le plus bas (T1) de 2,61 à 3,51 eV dans un état de film.

11. Dispositif de l'une quelconque des revendications 1 à 10, dans lequel le composé de formule (1) a une température de transition vitreuse Tg de 80 à 400 °C.

12. Dispositif de la revendication 1, dans lequel la matière d'émission de phosphorescence de formule (C-1) est représentée par la formule (C-2) : 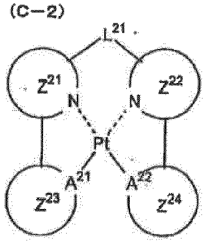 dans laquelle
L²¹ est une liaison simple ou un groupe de liaison divalent, et
A²¹, A²² sont chacun indépendamment C ou N,
Z²¹, Z²² sont chacun indépendamment un cycle hétéroaromatique contenant N, et
Z²³, Z²⁴ sont chacun indépendamment un cycle benzène ou un cycle hétéroaromatique.

13. Dispositif de l'une quelconque des revendications 1 à 12, dans lequel la longueur d'onde d'émission de lumière maximale est de 400 à 465 nm.

14. Dispositif de l'une quelconque des revendications 1 à 13, dans lequel la matière électroluminescente est contenue dans la couche électroluminescente dans une quantité de 1 à 30 % en poids.

15. Dispositif de l'une quelconque des revendications 1 à 14, comprenant, en tant que couche organique, une couche d'injection de trou ou une couche de transport de trou contenant un dopant accepteur d'électron.

16. Composition, comprenant le composé de formule (1) et la matière d'émission de phosphorescence de formule (C-1) tels que définis dans la revendication 1.

17. Composition de la revendication 16, comprenant en outre un composé de formule (PQ-1) : 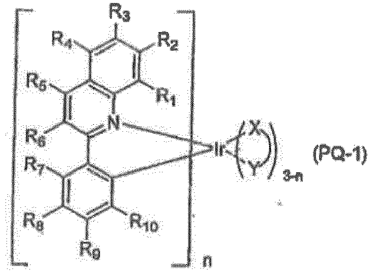 dans laquelle
R₁-R₁₀ sont chacun H ou un substituant, et les substituants peuvent être liés les uns aux autres pour former un cycle,
X-Y est un ligand bidentate monoanionique, et
n est un entier de 1 à 3.

18. Appareil choisi parmi un appareil d'émission de la lumière, un appareil d'affichage et un appareil d'éclairage, qui comprend le dispositif de l'une quelconque des revendications 1 à 15.
